(19) Europäisches Patentamt
European Patent Office
Office européen des brevets

(11) EP 0 662 972 B1

(12) **EUROPÄISCHE PATENTSCHRIFT**

(45) Veröffentlichungstag und Bekanntmachung des Hinweises auf die Patenterteilung:
**30.10.2002 Patentblatt 2002/44**

(21) Anmeldenummer: **93921875.6**

(22) Anmeldetag: **28.09.1993**

(51) Int Cl.[7]: **C07D 487/22**, C07B 59/00, A61K 49/00, A61K 31/40 // (C07D487/22, 257:00, 209:00, 209:00, 209:00, 209:00)

(86) Internationale Anmeldenummer:
**PCT/EP93/02645**

(87) Internationale Veröffentlichungsnummer:
**WO 94/007894 (14.04.1994 Gazette 1994/09)**

(54) **3-,8-SUBSTITUIERTE DEUTEROPORPHYRINDERIVATE, DIESE ENTHALTENDE PHARMAZEUTISCHE MITTEL UND VERFAHREN ZU IHRER HERSTELLUNG**

3-,8-SUBSTITUTED DEUTEROPORPHYRINE DERIVATIVES, PHARMACEUTICALS CONTAINING THEM AND METHODS OF PRODUCING THEM

DERIVES DE DEUTEROPORPHYRINE SUBSTITUES EN POSITIONS 3,8, PRODUITS PHARMACEUTIQUES LES CONTENANT ET PROCEDE PERMETTANT DE LES PREPARER

(84) Benannte Vertragsstaaten:
**AT BE CH DE DK ES FR GB GR IE IT LI LU MC NL PT SE**

(30) Priorität: **28.09.1992 DE 4232925**

(43) Veröffentlichungstag der Anmeldung:
**19.07.1995 Patentblatt 1995/29**

(73) Patentinhaber: **INSTITUT FÜR DIAGNOSTIKFORSCHUNG GmbH AN DER FREIEN UNIVERSITÄT BERLIN**
**14050 Berlin (DE)**

(72) Erfinder:
- **HILGER, Christoph-Stephan**
  **D-13353 Berlin (DE)**
- **MAIER, Franz-Karl**
  **D-13629 Berlin (DE)**
- **GRIES, Heinz**
  **D-10717 Berlin (DE)**
- **NIEDBALLA, Ulrich**
  **D-12161 Berlin (DE)**
- **PLATZEK, Johannes**
  **D-14195 Berlin (DE)**
- **LEE-VAUPEL, Mary**
  **D-10711 Berlin (DE)**
- **EBERT, Wolfgang**
  **D-12203 Berlin (DE)**
- **CONRAD, Jürgen**
  **D-12163 Berlin (DE)**
- **GAIDA, Josef**
  **D-10551 Berlin (DE)**

(56) Entgegenhaltungen:
**EP-A- 0 355 041**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 662 972 B1

Printed by Jouve, 75001 PARIS (FR)

## Beschreibung

Die Erfindung betrifft den in den Patentansprüchen gekennzeichneten Gegenstand, das heißt neue Porphyrin-Komplexverbindungen mit verschiedenen Substituenten in den Positionen 3, 8, 13 und 17 des Porphyringerüstes, diese Verbindungen enthaltende pharmazeutische Mittel, ihre Verwendung in der Diagnostik sowie Verfahren zur Herstellung dieser Verbindungen und Mittel.

Die Anwendung von Komplexbildnern oder Komplexen bzw. deren Salzen in der Medizin ist seit langem bekannt. Als Beispiele seien genannt:

Komplexbildner als Stabilisatoren pharmazeutischer Präparate, Komplexe und deren Salze als Hilfsmittel zur Verabreichung schlecht löslicher Ionen (z.B. Eisen), Komplexbildner und Komplexe (bevorzugt Calcium- oder Zink-), gegebenenfalls als Salze mit anorganischen und/oder organischen Basen, als Antidots zur Entgiftung bei versehentlicher Inkorporation von Schwermetallen oder deren radioaktiven Isotopen und Komplexbildner als Hilfsmittel in der Nuklearmedizin unter Verwendung radioaktiver Isotope wie $^{99m}Tc$ fiir die Szintigraphie.

In den Patentschriften EP 0 071 564, EP 0 139 934 und DE 34 01 052 sind Komplexe und Komplexsalze als Diagnostika, vorwiegend als NMR-Diagnostika, vorgestellt worden. Sie erfüllen jedoch noch nicht optimal alle Anforderungen, die die relative Wirksamkeit eines NMR-Kontrastmittels bestimmen, von denen beispielsweise zu nennen sind:

Eine günstige Relaxivität, so daß das Kontrastmittel in möglichst geringen Konzentrationen die Relaxationszeiten der Protonen im Gewebewasser und anderer fiir die NMR relevanter Kerne (wie Phosphor, Fluor, Natrium) in vivo erniedrigt und beispielsweise die Lokalisation von Tumoren durch Erhöhung der Signalintensität des mit Hilfe des Kernspintomographen erhaltenen Bildes ermöglicht; eine möglichst selektive Anreicherung und/oder Retention des Kontrastmittels im Zielorgan; ausreichende Wasserlöslichkeit; hohe Wirksamkeit; gute Verträglichkeit; gute chemische und biochemische Stabilität.

Für die Bildgebung relevant sind dabei vor allem die beiden erstgenannten Punkte. Da die Relaxationszeiten zwischen den Geweben sich meistens nur um den Faktor 2-3 unterscheiden (T.E. Budinger und P.C. Lauterbur, Science 226, pp 288-298, 1984; J.M.S. Hutchinson und F.W. Smith in Nuclear Magnetic Resonance Imaging Edit. C.L. Partain et. al., pp 231-249, Saunders, New York 1983) und die Komplexe und Komplexsalze der genannten Patentschriften im allgemeinen mit dem Nachteil behaftet sind, daß sie sich nur relativ unspezifisch im Extrazellulärraum verteilen und daher nicht immer eine Erkennung pathologisch veränderter Gewebe ermöglichen, besteht ein Bedarf nach vor allem selektiv-bindenden, tumorspezifischen Verbindungen, die sich in der Diagnostik und Strahlentherapie verwenden lassen.

Es ist nun seit einigen Jahren bekannt, daß sich Porphyrinderivate selektiv in menschlichen und tierischen Tumoren anreichern (D. Kessel und T.-H. Chou, Cancer Res. 43, pp 1994-1999, 1983, P. Hambright, Bioinorg. Chem. 5, pp 87-92, 1975; R. Lipson et al., Cancer 20, pp. 2250-2257, 1967; D.Sanderson et al., Cancer 30, pp. 1368-1372, 1972). Erste Ansätze, diese Verbindungsklasse auch als Diagnostika einzusetzen sind ebenfalls beschrieben worden (J. Winkelmann et al., Cancer Research 27, pp. 2060-2064, 1967; N.J. Patronas et al., Cancer Treatment Reports 70, pp. 391-395, 1986).

Die bisher beschriebenen Verbindungen sind jedoch weit davon entfernt die oben genannten Kriterien zufriedenstellend zu erfüllen; besondere Aufmerksamkeit verlangt immer noch ihre mangelnde Anreicherung in den Zielorganen. Eine Verbesserung dieser Eigenschaft sollte gleichzeitig die bestehenden Probleme mit der Toxizität und Verträglichkeit der vorbekannten Verbindungen reduzieren helfen.

In der Patentanmeldung EP 0 355 041 werden substituierte Hämatoporphyrin-Komplexverbindungen beschrieben, die Anwendung in der Diagnostik und Therapie finden.

Zwar zeigen diese Verbindungen ein gutes Anreicherungsverhalten in verschiedenen Zielorganen, jedoch weisen die beschriebenen Verbindungen bei der Verwendung als NMR-Diagnostika ein noch nicht völlig befriedigendes Verhältnis zwischen der fiir eine optimale Bildgebung erforderlichen Dosis und der letalen Dosis auf. Auch haben HämatoporphyrinDerivate den Nachteil, daß die beiden pseudobenzylischen OH-Gruppen in den Hydroxyethylseitenketten eliminieren können.

In der Patentschrift EP 0 071 569 werden NMR-Diagnostika auf der Basis von DTPA-Komplexen beschrieben, die zwar einen günstigen Sicherheitsabstand aufweisen, jedoch relativ schnell ausgeschieden werden, wodurch der verbleibende Untersuchungszeitraum mit optimalem Enhancement nur kurz ist.

Für das Tumor Imaging mit Radioisotopen sind Derivate des Deuteroporphyrins vorgeschlagen worden, die als zusätzliche komplexbildende Gruppen Polyaminopolycarbonsäuren über Ethylenglycolbrücken an das Porphyringerüst gebunden enthalten (Photochemistry and Photobiology Vol. 46, pp 783-788 (1987)). Solche Porphyrinester sind

jedoch für eine parenterale Anwendung am Patienteninsbesondere für die NMR-Diagnostik - wenig geeignet, da die daraus erhältlichen Injektionslösungen weder hitzesterilisierbar noch über eine ausreichenden Zeitraum lagerfähig sind.

Es besteht daher weiterhin für vielfältige Zwecke ein Bedarf an stabilen, gut löslichen, aber auch besser verträglichen, selektiver bindenden, über eine größere chemische Variationsbreite der Substiuenten (die z.B. den Einbau anderer Metalle als Mangan bzw. mehrerer, auch unterschiedlicher, Metalle ermöglicht und damit gleichzeitig auch zu einer Steuerung der Eigenschaften und Anwendungen der Verbindungen führt) verfügenden Komplexverbindungen, die z.B. für die Diagnostik und Therapie von Tumoren geeignet sind.

Der Erfindung liegt somit die Aufgabe zugrunde, derartige Verbindungen und diese Verbindungen enthaltende pharmazeutischen Mittel zur Verfügung zu stellen, sowie Verfahren zu ihrer Herstellung zu schaffen.

Diese Aufgabe wird durch die Erfindung gelöst.

Es wurde gefunden, daß sich Porphyrin-Komplexverbindungen, bestehend aus einem Porphyrinliganden, mindestens einem Ion eines Elements der Ordnungszahlen 21-32, 37-39, 42-51 oder 57-83 sowie gegebenenfalls Kationen anorganischer und/oder organischer Basen, überraschenderweise hervorragend zur Herstellung von NMR- und Radio-Diagnostika sowie Radio-Therapeutika eignen.

Die erfindunggemäßen Porphyrin-Komplexverbindungen bestehen aus einem Liganden der allgemeinen Formel I

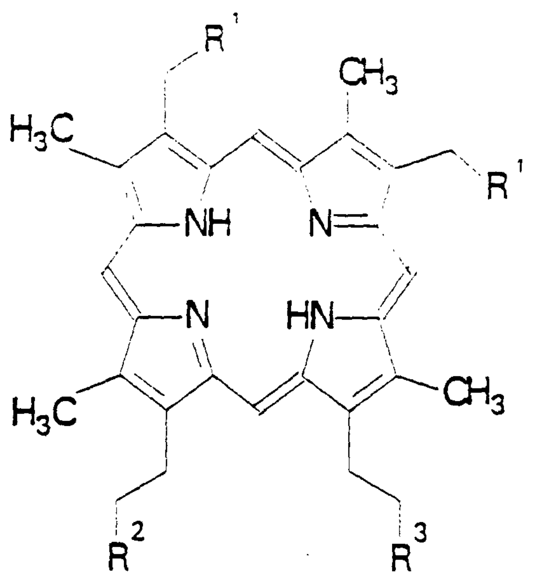

(I)

sowie mindestens einem Ion eines Elementes der Ordnungszahl 21-32, 37-39, 42-51 oder 57-83, worin

$R^1$ für ein Wasserstoffatom, für einen geradkettigen $C_1$-$C_6$-Alkylrest, einen $C_7$-$C_{12}$-Aralkylrest oder für eine Gruppe OR' worin
R' ein Wasserstoffatom oder ein $C_1$-$C_3$-Alkylrest ist, steht,

$R^2$ für $R^3$, eine Gruppe -CO-Z oder eine Gruppe $-(NH)_o-(A)_q-NH-D$ steht, worin
Z eine Gruppe -OL ist, mit L in der Bedeutung eines anorganischen oder organischen Kations oder eines $C_1$-$C_4$-Alkylrestes ist,
A eine $(CH_2)_{\overline{2-4}}$, Phenylenoxy- oder eine im Alkylteil durch ein Sauerstoffatom unterbrochene $C_1$-$C_{12}$-Alkylen- oder $C_7$-$C_{12}$ Aralkylengruppe bedeutet,
o und q unabhängig voneinander die Ziffern 0 oder 1 bedeuten und
D ein Wasserstoffatom oder eine Gruppe $CO-A-(COOL)_o-(H)_m$ bedeutet, mit m gleich 0 oder 1 und unter der Maßgabe, daß die Summe aus m und o gleich 1 ist,

$R^3$ für eine Gruppe $-(C{=}M)(NR^4)_o-(A)_q(NR^5)-K$ steht,
worin M für ein Sauerstoffatom oder für zwei Wasserstoffatome steht,
$R^4$ eine Gruppe $-(A)_q-H$ bedeutet und
K einen Komplexbildner der allgemeinen Formel (IIa) oder (IIb) bedeutet und wobei $R^5$, für den Fall, daß K ein Komplexbildner der Formel (IIa) ist die gleiche Bedeutung wie $R^4$ hat und $R^5$ für den Fall, daß K ein Komplexbildner der Formel (IIb) ist, die gleiche Bedeutung wie D hat,

mit der Maßgabe, daß eine direkte Sauerstoff-Stickstoff Bindung nicht zugelassen ist, und K für einen Komplexbildner der allgemeinen Formel (IIa) oder (IIb)

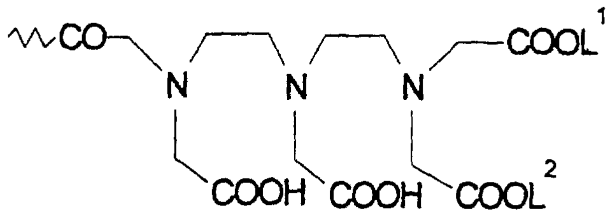

(IIa)

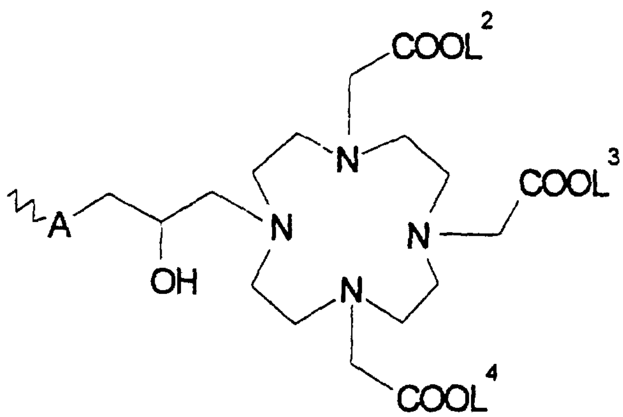

(IIb)

steht, mit $L^1$ in der Bedeutung eines $C_1$-$C_6$-Alkyl-Restes oder eines anorganischen oder organischen Kations und worin $L^2$, $L^3$ und $L^4$ unabhängig voneinander die Bedeutung von $L^1$ oder eines Wasserstoffatoms haben, unter der Maßgabe, daß im Komplexbildner mindestens zwei freie Carbonsäuregruppen vorliegen,

sowie gegebenenfalls weitere Anionen zum Ausgleich der Ladungen im Metalloporphyrin.

Die erfindungsgemäßen Komplexverbindungen umfassen insgesamt drei Gruppen von Verbindungen.

a) Verbindungen, die ein Metallion im Porphyrin enthalten,
b) Verbindungen, die mindestens ein Metallion im Komplexbildner-Rest K enthalten und
c) Verbindungen, die sowohl Metallionen im Porphyrin als auch im Komplex bildnerrest K gebunden enthalten, wobei die Metallionen unterschiedlich sein können.

Für die Verwendung der erfindungsgemäßen Mittel in der NMR-Diagnostik müssen paramagnetische Metallionen im Komplex vorhanden sein. Dies sind insbesondere zwei und dreiwertige Ionen der Elemente der Ordnungszahlen 21-29, 42, 44 und 57-70. Geeignete Ionen sind beispielsweise Chrom(III)-, Mangan(II)-, Mangan(III)-, Eisen (III)-, Cobalt(II)-, Cobalt (III), Nickel(II)-, Kupfer(II)-, Praseodym(II)-, Neodym(III)-, Samarium(III)- und Ytterbium(III)-ion. Wegen ihres hohen magnetischen Moments sind besonders bevorzugt das Gadolinium(III)-, Terbium(III)-, Dysprosium(III)-, Holmium(III)-, Erbium(III)- und Eisen(III)-ion.

Für die Radiodiagnostik und Radiotherapie sind Komplexe geeignet, die als Zentralatom ein Radioisotop der Elemente 27, 29-32, 37-39, 42-51, 62, 64, 70, 75, 77, 82 oder 83 enthalten.

Soll die Anreicherung z.B. eines Yttrium-90 markierten Komplexes bei Verwendung in der Radio-Therapie, NMR-diagnostisch kontrolliert werden, so eignen sich Komplexe die neben dem Radioisotop als weiteres Metallion ein paramagnetisches Metallion, bevorzugt ein Gadolinium-Ion, enthalten.
Auf diese Weise gelingt es, Diagnostik und Therapie mit Hilfe der erfmdungsgemäßen Komplexkonjugate zu kombinieren.

Ein wesentlicher Vorteil der erfindungsgemäßen, den Komplexbildnerbildnerrest K enthaltenden Metallkomplexe ist, daß bei diesen der von den Metallionen bewirkte diagnostische Effekt durch den Einbau weiterer Metallionen verstärkt werden kann.

Überraschenderweise zeigen die erfindungsgemäßen Komplexe gegenüber den bislang bekannten, strukturell ähnlichen Verbindungen eine deutlich höhere Relaxivität. Da die Relaxivität als ein Maß für die Kontrastmittelwirksamkeit einer Verbindung angesehen werden kann, gelingt bei Verwendung der erfindunggemäßen Komplexe im Bereich der NMR-Diagnostik eine vergleichbare, positive Signalbeeinflussung schon bei einer niedrigen Dosis. Dadurch vergrößert sich der Sicherheitsabstand signifikant, für den als Richtwert das Produkt aus Relaxivität und Verträglichkeit angesehen werden kann.

Auch die übrigen Anforderungen, wie z.B. eine hohe Selektivität und Anreicherung erfüllen die erfindungsgemäßen Komplexverbindungen in hervorragenden Maße. Mit Hilfe der erfindungsgemäßer Komplexverbindungen können überraschenderweise nicht nur Tumorgewebe und einzelne Organe, wie zum Beispiel Leber und Nieren, sondern auch Blutgefäße ohne Anwendung spezieller Puls-Sequenzen in vivo dargestellt werden, womit sie unter anderem als Perfusionsagentien Verwendung finden können.

Als Beispiel für die im Porphyringerüst gebundenen Ionen seien die Metalle Mangan, Eisen, Cobalt, Nickel, Kupfer, Zink und Technetium genannt. Bevorzugt sind die Metalle Eisen, Technetium, Zink und insbesondere Mangan.

Sofern eines der im Porphyrin gebundenen Ionen in einer höheren Oxidationsstufe als +2 vorliegt, so wird (werden) die überschüssige(n) Ladung(en) z.B. durch Anionen von organischen oder anorganischen Säuren, bevorzugt durch Acetat-, Chlorid-, Oxidoder Nitrid-Ionen ausgeglichen.

Als Beispiel fiir die an den Komplexbildner K gebundenen Ionen seien die Übergangsmetalle der Ordnungszahlen 21-30, 37, 39, und 43 sowie die Elemente 57-83 genannt. Bevorzugt ist das Gadolinium-, Dysprosium-, Holmium, Erbium- und das Mangan-Ion.

Gewünschtenfalls können die Carboxylgruppen, die nicht für die Komplexierung der Metallionen benötigt werden, als Ester, als Amide oder als Salze anorganischer oder organischer Basen vorliegen. Geeignete Esterreste sind solche mit 1 bis 6 C-Atomen vorzugsweise die Ethylester; geeignete anorganische Kationen sind beispielsweise das Lithium- und das Kalium-Ion und insbesondere das Natrium-Ion. Geeignete Kationen organischer Basen sind solche von primären sekundären oder tertiären Aminen, wie zum Beispiel Ethanolamin, Diethanolamin, Morpholin, Glucamin, N,N-Dimethylglucamin insbesondere das Meglumin.

Als Beispiele für die Gruppe $-(NR^4)_o-(A)_q-(NR^5)-$, die quasi als "Linker" zwischen dem Porphyringerüst und dem Komplexbildner K dient, seien beispielhaft genannt die -NHNH-, $-NH(CH_2)_2NH-$, $-NH(CH_2)_3NH-$, $-NH(CH_2)_4NH-$, $-NH(CH_2)_2O(CH_2)_2NH-$, $-NH-CH_2-C_6H_4-CH_2-NH-$ und die $-CH_2-O-C_6H_4-NH-$Gruppe.

Als Komplexbildnerreste K seien vorzugsweise Derivate der Diethylentriaminpentaessigsäure und der 1,4,7,10-Tetraazacyclododecan-1,4,7-triessigsäure genannt, die über einen "Linker" an das jeweilige Porphyrin gebunden sind.

Die Herstellung der erfindungsgemäßen Komplexverbindungen erfolgt dadurch, daß man durch

a) Reduktion eines Porphyrins der allgemeinen Formel (IIIa)

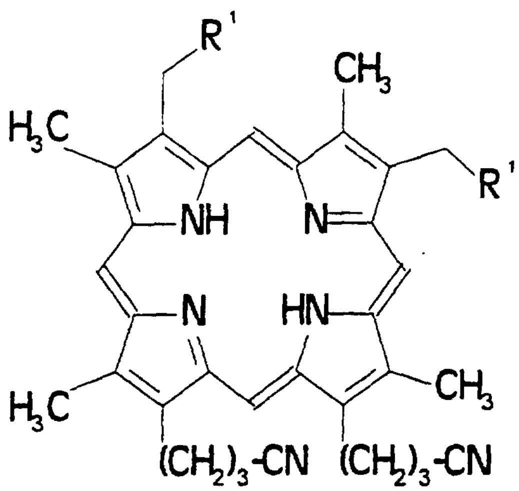

(IIIa)

oder durch

b) Umsetzung eines Porphyrins der allgemeinen Formel (IIIb)

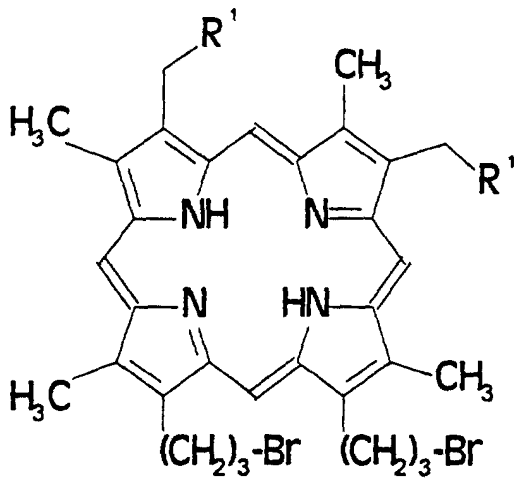

(IIIb)

mit Aminophenol, oder durch

c) Umsetzung eines Porphyrins der allgemeinen Formel (IIIc)

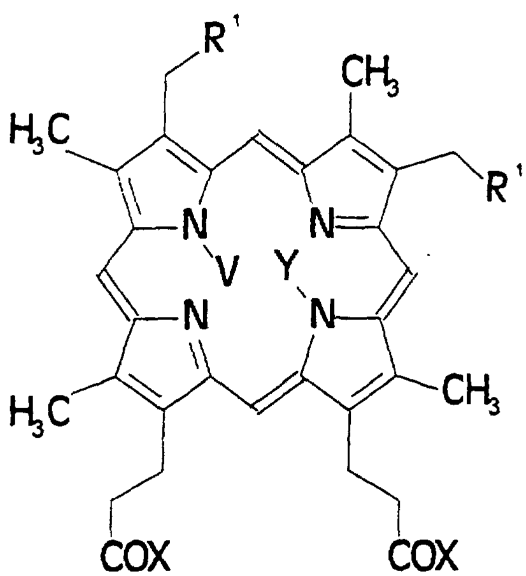

(IIIc)

worin $R^1$ die angegebene Bedeutung hat,

V und Y jeweils für ein Wasserstoffatom oder gemeinsam für ein mehrwertiges Metall-Ion eines Elementes der Ordnungszahl 21-32, 37-39, 42-51 oder 57-83 , vorzugsweise für ein Zink-(II)- oder ein Mangan-(III)-ion stehen und

X für ein Halogenatom, eine Gruppe -OR' oder für eine Gruppe -O-COOR' mit

R' in der genannten Bedeutung steht, mit Verbindungen $H\text{-}NR^4\text{-}(A)_q\text{-}NR^4\text{-}H$,

worin A, $R^4$ und q die angegebene Bedeutung haben, gewünschtenfalls anschließende Reduktion der Carbonylgruppen oder Hofmann'schen Abbau des Amids zunächst ein Porphyrin der allgemeinen Formel IV

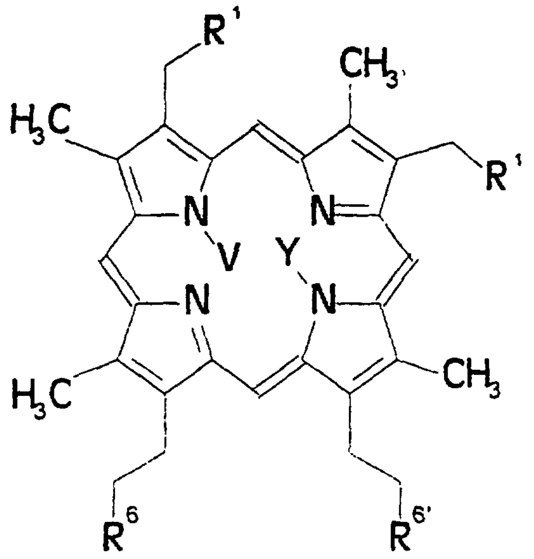

(IV)

erhält,
worin $R^6$ fiir eine Gruppe $-(C{=}M)-(NR^4)_o-(A)_q-NR^4-H$ steht,
worin M, $R^4$, A, o und q die angegebene Bedeutung haben und
worin $R^{6'}$ die gleiche Bedeutung wie $R^6$ hat oder für eine Gruppe -OR' steht, das anschließend

a) mit einem Komplexbildner der allgemeinen Formel V,

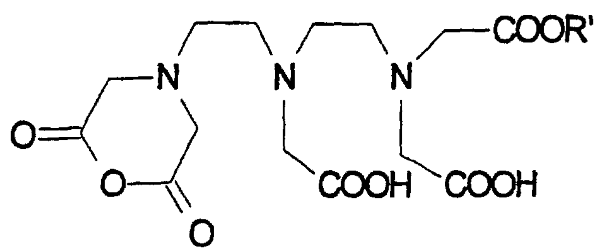

(V)

worin R' die angegebene Bedeutung hat, umgesetzt wird und gewünschtenfalls die vorhandenen Estergruppen verseift werden
oder
b) mit einer Verbindung der Formel VI

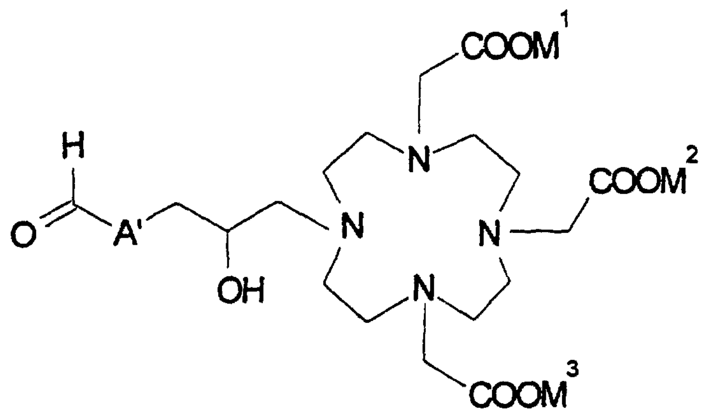

(VI)

worin A' eine um ein Kohlenstoffatom verkürzte Gruppe A ist und $M^1$, $M^2$ und $M^3$ unabhängig voneinander für R' oder ein Metallionenäquivalent der Elemente der Ordnungszahlen 21-32, 37-39, 42-51 oder 57-83 stehen, unter den Bedingungen einer reduktiven Aminierung umgesetzt wird, danach das so erhaltene Produktgegebenenfalls nach vollständiger oder teilweiser Abspaltung der Estergruppen - mit (einem) Metalloxid(en) oder Metallsalz(en) der Elemente der oben genannten Ordnungszahlen umsetzt, anschließend mit einem Reagenz Nucleofug-D' acyliert, (wobei die letztgenannten Schritte vertauscht werden können) worin D' die unter D angegebene Bedeutung hat, unter der Maßgabe, daß D' nicht für Wasserstoff steht und abschließend gewünschtenfalls eventuell noch vorhandene acide Wasserstoffe durch Kationen anorganischer oder organischer Basen vollständig oder teilweise substituiert.

Als Nucleofug-D' seien beispielhaft genannt Acetylchlorid, Acetanhydrid, Bernsteinsäureanhydrid oder Diglycolsäureanhydrid.

Somit erfolgt die Funktionalisierung der C-13 und C-17 Seitenketten zu C-13,17-Amino- oder Amidoalkyl-substituierten Porphyrin-Derivaten (die als Edukte für die erfindungsgemäßen Porphyrinkomplexe, die den Komplexbildner K enthalten, dienen) entweder durch

a) Reduktion des betreffenden 13,17-Bis-(3-cyanopropyl)-porphyrins zum Amin in an sich bekannter Weise, zum Beispiel mit Lithiumborhydrid/Trimethylsilylchlorid in einem organischen polaren Ether, vorzugsweise Tetrahydrofuran (A. Giannis, K. Sandhoff, Angew. Chem.; **101** (1989) 220/22) oder
b) Umsetzung des betreffenden 13,17-Bis-(3-brompropyl)-porphyrins mit einem Aminophenol in einem dipolaren aprotischen Lösungsmittel wie z.B. Dimethylformamid oder Dimethylsulfoxid oder
c) Umsetzung der gewünschten C-13,17-Propionsäureketten-tragenden Porphyrine, die gegebenenfalls in aktiver Form z.B. als Säurechloride, Ester oder gemischte Anhydride vorliegen können, gewünschtenfalls anstelle der pyrrolischen Wasserstoffe bereits ein Metallatom enthalten, mit gegebenenfalls substituierten Hydrazinen oder mit endständigen Alkylendiaminen, die gegebenenfalls durch einen $C_1$-$C_6$ Alkyl-, Aryl- oder $C_7$-$C_{12}$ Aralkyl-Rest substituiert sein können und von denen eine Aminogruppe gegebenenfalls z.B. in Form des Carbobenzoxy- oder t-Butoxycarbonylrestes geschützt ist. Die Entfernung der Schutzgruppen erfolgt anschließend nach literaturbekannten Methoden, z.B. durch Hydrierung oder Behandlung mit Trifluoressigsäure bzw. mit Salzsäure/Eisessig.

Sollen unsymetrisch substituierte Porphyrine hergestellt werden, d.h. Porphyrine in denen die Reste $R^2$ und $R^3$ ungleich sind, so kann dies über die Reaktionszeit und die Stöchiometrie gesteuert werden.

Die Einführung des Komplexbildner-Restes K der allgemeinen Formel IIa in die so funktionalisierten C-13, C-17 Amino- oder Amidoalkyl-substituierten Porphyrin-Derivate erfolgt in an sich bekannter Weise durch Umsetzung mit den entsprechenden Säureanhydriden in flüssiger Phase. Geeignete Reaktionsmedien sind beispielsweise Wasser, dipolare aprotische Lösungsmittel wie Dimethylformamid, Dimethylsulfoxid oder Hexamethylphosphorsäuretriamid in Gegenwart einer anorganischen oder organischen Base, wie Alkali- oder Erdalkali-hydroxide und -carbonate und tertiäre Amine wie z.B. Trimethylamin, Triethylamin, N,N'-Dimethylaminopyridin.

Geeignete Reaktionstemperaturen liegen zwischen -10 und 120°C, vorzugsweise zwischen 0 und 50°C.

Die Einführung des Restes K der allgemeinen Formel IIb erfolgt in an sich bekannter Weise, indem ein entsprechendes Vorprodukt , das gegebenenfalls bereits durch ein Metall substituiert sein kann, durch Glycolspaltung (z. B. mit Perjodat) zunächst zu einem Aldehyd der allgemeinen Formel VI umgesetzt wird und anschließend mit dem betreffenden C-13, C-17 Amino- oder Amidoalkyl-substituierten Porphyrin-Derivat umgesetzt wird. Diesem Reaktionsschritt schließt sich eine Reduktion z.B. mit Natriumcyanoborhydrid an.

Verbleibende sekundäre Amine können durch Umsetzung mit aktivierten Säurederivaten (Nucleofug-D')

acyliert werden.

Die Einführung der gewünschten Metalle (z.B. Mn, Fe, Co, Ni, Cu, Zn, Tc, Sm, Eu, Gd, Bi) in die Porphyrine erfolgt nach literaturbekannten Methoden (The Porphyrins, ed. D. Dolphin, Academic Press, New York 1980, Vol. V, p. 459), wobei im wesentlichen zu nennen sind:

a) die Substitution der pyrrolischen NH's (durch Erwärmen des metallfreien Liganden mit dem entsprechenden Metallsalz , vorzugsweise dem Acetat, gegebenenfalls unter Zusatz von säurepuffernden Mitteln, wie z.B. Natriumacetat , in einem polaren Lösungsmittel) oder

b) die "Umkomplexierung". bei der ein bereits vom Liganden komplexiertes Metall durch das gewünschte Metall verdrängt wird. Als Lösungsmittel sind vor allem polare Solventien, wie z.B. Methanol, Eisessig, Dimethylformamid, Chloroform und Wasser geeignet.

Die Einführung des Porphyrinmetalls kann vor oder nach Anknüpfung des Komplexbildner-Restes K sowie auch vor oder nach Chelatisierung dieses Komplexbildners mit einem Metall erfolgen. Dadurch wird eine besonders flexible Vorgehensweise für die Synthese der erfindungsgemäßen Verbindungen ermöglicht, so daß z.B. Metall-Isotope geringer Halbwertszeit (zum Beispiel 99m-Technetium), sei es in den Porphyrinliganden oder in den Komplexbildner, erst im letzten Syntheseschritt eingeführt werden können.

Die Chelatisierung des Restes K erfolgt in literaturbekannter Weise (siehe z.B. DE 34 01 052) indem das Metalloxid oder -salz (z.B. das Nitrat, Acetat, Carbonat, Chlorid oder Sulfat) des jeweils gewünschten Metalls, in polaren Lösungsmitteln wie Wasser oder wäßrigen Alkoholen suspendiert oder gelöst wird und mit der entsprechenden Menge des komplexbildenden Liganden umgesetzt wird. Soweit gewünscht, können vorhandene acide Wasserstoffatome oder Säuregruppen durch Kationen anorganischer und/oder organischer Basen oder Aminosäuren substituiert werden.

Die Neutralisation erfolgt dabei mit Hilfe anorganischer Basen wie z.B. Alkali- oder Erdalkali-hydroxiden, -carbonaten oder -bicarbonaten und/oder organischer Basen wie unter anderem primärer, sekundärer und tertiärer Amine, wie z.B. Ethanolamin, Morpholin, Glucamin, N-Methyl- und N,N-Dimethylglucamin, sowie basischer Aminosäuren, wie z.B. Lysin, Arginin und Ornithin oder von Amiden ur-sprünglich neutraler oder saurer Aminosäuren.

Zur Herstellung der neutralen Komplexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (z.B. Methanol, Ethanol, Isopropanol), niederen Ketonen (z.B. Aceton), polaren Ethern (z.B. Tetrahydrofuran, Dioxan, 1,2-Dimethoxyethan) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Enthalten die sauren Komplexverbindungen mehrere freie acide Gruppen, so ist es oft zweckmäßig, neutrale Mischsalze herzustellen, die sowohl anorganische als auch organische Kationen als Gegenionen enthalten.

Dies kann beispielsweise geschehen, indem man den komplexbildenden Liganden in wäßriger Suspension oder Lösung mit dem Oxid oder Salz des das Zentralion liefernden Elements und der Hälfte des zur neutralisation benötigten Menge einer organischen Base umsetzt, das gebildete Komplexsalz isoliert, es gewünschtenfalls reinigt und dann zur vollständigen Neutralisation mit der benötigten Menge anorganischer Base versetzt. Die Reihenfolge der Basenzugabe kann auch umgekehrt werden.

Eine andere Möglichkeit, zu neutralen Komplexverbindungen zu kommen, besteht darin, die verbleibenden Säuregruppen im Komplex ganz oder teilweise in Ester zu überführen. Dies kann durch nachträgliche Reaktion am fertigen Komplex geschehen (z.B. durch erschöpfende Umsetzung der freien Carboxy-Gruppen mit Dimethylsulfat).

Im Falle der Verwendung von Radioisotope enthaltenden Komplexverbindungen kann deren Herstellung nach den in "Radiotracers for Medical Applications", Volume 1, CRC-Press, Boca Raton, Florida beschriebenen Methoden vorgenommen werden.

Die Herstellung der erfindungsgemäßen pharmazeutischen Mittel erfolgt ebenfalls in an sich bekannter Weise, indem man die erfindungsgemäßen Komplexverbindungengegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie z.B. Tromethamin), geringe Zusätze von Komplexbildnern (wie z.B. Diethylentriaminpentaessigsäure) oder, falls erforderlich, Elektrolyte wie z. B. Natriumchlorid oder, falls erforderlich, Antioxidantien wie z.B. Ascorbinsäure.

Sind für die enterale Verabreichung oder andere Zwecke Suspensionen oder Lösungen der erfindungsgemäßen Mittel in Wasser oder in physiologischer Salzlösung erwünscht, werden sie mit einem oder mehreren in der Galenik üblichen Hilfsstoff(en) (z.B. Methylcellulose, Lactose, Mannit) und/oder Tensid(en) (z.B. Lecithine, Tween®, Myrj®) und/oder Aromastoffen zur Geschmackskorrektur (z.B. etherischen Ölen) gemischt.

Prinzipiell ist es auch möglich, die erfindungsgemäßen pharmazeutischen Mittel auch ohne Isolierung der

Komplexsalze herzustellen. In jedem Fall muß besondere Sorgfalt darauf verwendet werden, die Chelatbildung so vorzunehmen, daß die erfindungsgemäßen Salze und Salzlösungen praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexsalzes.

Um unerwünschte Photoreaktionen der Porphyrine zu vermeiden, sollten die erfindungsgemäßen Verbindungen und Mittel möglichst unter Lichtausschluß gelagert und gehandhabt werden.

Die erfindungsgemäßen pharmazeutischen Mittel enthalten vorzugsweise 20 µMol/L bis 200 mmol/L des Komplexsalzes und werden in der Regel in Mengen von 0,01 µMol bis 2 mMol/kg Körpergewicht dosiert. Sie sind zur enteralen und parenteralen Applikation bestimmt.

Die erfindungsgemäßen Komplexverbindungen kommen zur Anwendung

1. in der NMR-Diagnostik in Form ihrer Komplexe mit den Ionen der Elemente mit den Ordnungszahlen 21-30, 42, 44 und 57-83;
2. in der Radiodiagnostik und -Therapie in Form ihrer Komplexe mit den Isotopen der Elemente mit den Ordnungszahlen 27, 29-32, 37-39, 42-51, 62, 64, 70, 75, 77, 82 oder 83.

Die erfindungsgemäßen Mittel erfüllen die vielfältigen Voraussetzungen für die Eignung als Kontrastmittel für die Kernspintomographie. So sind sie hervorragend dazu geeignet, nach enteraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten und die gute Verträglichkeit, die notwendig ist, um den nichtinvasiven Charakter der Untersuchungen aufrechtzuerhalten.

Die gute Wasserlöslichkeit der erfindungsgemäßen Mittel erlaubt es hochkonzentrierte Lösungen herzustellen, damit die Volumenbelastung des Kreislaufs in vertretbaren Grenzen zu halten und die Verdünnung durch Körperflüssigkeit auszugleichen. Weiterhin weisen die erfindungsgemäßen Mittel nicht nur eine hohe Stabilität In vitro auf, sondern auch eine überraschend hohe Stabilität in vivo, so daß eine Freigabe oder ein Austausch der in den Komplexen nicht konvalent gebundenen - an sich giftigen-Ionen innerhalb der Zeit, in der die neuen Kontrastmittel vollständig wieder ausgeschieden werden, zu vernachlässigen ist.

Details der Anwendung werden zum Beispiel in H.J. Weinmann et al., Am. J. of Roentgenology **142**, 619 (1984) diskutiert.

Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet. Details bezüglich der Anwendung und Dosierung der radioaktive Metallionen tragenden erfindungsgemäßen Komplexverbindungen im Bereich der Radiodiagnostik, werden z.B. in "Radiotracers for Medical Applications", CRS-Press, Boca Raton, Florida beschrieben.

Eine weitere bildgebende Methode mit Radioisotopen ist die Positronen-Emissions-Tomographie, die positronenemittierende Isotope wie z.B. $^{43}Sc$, $^{44}Sc$, $^{52}Fe$, $^{55}Co$ und $^{68}Ga$ verwendet (Heiss, W.D., Phelps, M.E., Positron Emission Tomography of Brain, Springer Verlag Berlin, Heidelberg, New York 1983).

Die erfindungsgemäßen Verbindungen können auch in der Radioimmuno- oder Strahlentherapie verwendet werden. Diese unterscheidet sich von der entsprechenden Diagnostik nur durch die Art und die Menge des verwendeten Isotops. Ziel ist dabei die Zerstörung von Tumorzellen durch energiereiche kurzwellige Strahlung mit einer möglichst geringen Reichweite. Geeignete β-emittierende Ionen sind zum Beispiel $^{46}Sc$, $^{47}Sc$, $^{48}Sc$, $^{72}Ga$, $^{73}Ga$ und $^{90}Y$. Geeignete geringe Halbwertszeiten aufweisende α-emittierende Ionen sind z.B. $^{211}Bi$, $^{212}Bi$, $^{213}Bi$, $^{214}Bi$, wobei das $^{212}Bi$ bevorzugt ist. Ein geeignetes photonen- und elektronenemittierendes Ion ist $^{158}Gd$, das aus $^{157}Gd$ durch Neutroneneinfang erhalten werden kann.

Ist das erfindungsgemäße Mittel zur Anwendung in der von R.L. Mills et al. [Nature Vol. 336, (1988), S. 787] vorgeschlagenen Variante der Strahlentherapie bestimmt, so muß (müssen) sich das (die) komplexierte(n) Ion(en) von einem Mößbauer-Isotop wie beispielsweise $^{57}Fe$ oder $^{151}Eu$ ableiten.

Bei der in vivo Applikation der erfindungsgemäßen therapeutischen Mittel können diese zusammen mit geeigneten Trägern wie z.B. Serum oder pysiologischer Kochsalzlösung und zusammen mit einem anderen Protein wie z.B. Human Serum Albumin verabreicht werden. Die Dosierung ist dabei abhängig von der Art der zellulären Störung, dem benutzten Metallion und der Art der Methode, z.B. Brachytherapie.

Die erfindungsgemäßen therapeutischen Mittel werden parenteral appliziert.

Details der Anwendung von Radiotherapeutika werden z.B. in R.W. Kozak et al. TIBTEC, Oktober 1986, 262 diskutiert.

Die Erfindung wird durch die nachfolgenden Beispiele erläutert:

**Beispiel 1**

a) N,N'-Bis[9-carboxy-2,5-bis(carboxymethyl)-8-(ethoxycarboxymethyl-2,5,8-triazanonyl-carbamoyl]-mesoporphyrin-IX-13,17-diamid

806,8 mg (2 mmol) 3-Ethoxy-carbonylmethyl-6-[2-(2,6-dioxomorpholino)ethyl]-3,6-diazaoctandi-säure (DTPA-monoethylester-monoanhydrid) werden in 250 ml absolutem Dimethylformamid suspendiert. Man überschichtet mit Stickstoff, setzt 1,01 g (10 mmol) Triethylamin und 595 mg (1 mmol) Mesoporphyrin-IX-13,17-dihydrazid (hergestellt analog zu H. Fischer, E. Haarer und F. Stadler, Z. Physiol. Chem. 241, 209 (1936) zu und rührt das resultierende Reaktionsgemisch 3 Tage bei Raumtemperatur. Nach beendeter Reaktion wird filtriert, das Lösungsmittel im Vakuum abgezogen und das verbleibende Öl mit 500 ml Diethylether verrieben. Der ausgefallene Feststoff wird abfiltriert und mit Diethylether und n-Hexan gewaschen. Zur Reinigung wird an Kieselgel RP-18 chromatographiert (Eluens: $H_2O$/Tetrahydrofuran; Tetrahydrofuran: 0-30 %).
Ausbeute: 1,21 g (86,3 % d. Th.) rotbraunes Pulver

| Analyse (bezogen auf wasserfreie Substanz): | | | | |
|---|---|---|---|---|
| C 56,56<br>C 56,25 | H 6,62<br>H 6,89 | N 13,99<br>N 13,70 | O 22,83 | ber. |

b) N,N'-Bis[9-carboxylato-2.5-bis(carboxylatomethyl)-8-(ethoxycarboxymethyl-2,5,8-triazononyl-carbamoyl]-mesoporphyrin-IX-13,17-diamid, Digadoliniumkomplex

1,40 g (1 mmol) des unter Beispiel 1a hergestellten Liganden werden in 400 ml Wasser gelöst. Durch Zugabe von 2n wäßriger Natronlauge wird pH 7 eingestellt und abwechselnd portionsweise 894,2 mg (2,2 mmol) Gadoliniumacetat-tetrahydrat und 2n wäßrige Natronlauge zugesetzt, so daß der ph-Wert des Reaktionsgemisches stets zwischen 6,8 und 7,2 pendelt. Ist alles Gadoliniumacetat zugesetzt, wird über Nacht bei Raumtemperatur nachgerüht. Zur Aufarbeitung wird das Lösungsmittel im Vakuum abgezogen und der Rückstand an Kieselgel RP-18 chromatographiert (Eluens: $H_2O$/Tetrahydrofuran; Tetrahydrofuran: 0-30 %).
Ausbeute: 1,01 g (59,1 % d. Th.) rotbraunes Pulver

| Analyse (bezogen auf wasserfreie Substanz): | | | | | |
|---|---|---|---|---|---|
| C 46,36<br>C 46,08 | H 5,07<br>H 5,29 | N 11,47<br>N 11,27 | Gd 18,39<br>Gd 18,05 | O 18,71 | ber. |

c) N,N'-Bis[9-Carboxylato-2,5,8-tris-(carboxylatomethyl)-2,5,8-triazanonylcarbamoyl]-mesoporphyrin-IX-13,17-diamid, Digadoliniumkomplex, Dinatriumsalz

1,4 g (1 mmol) des unter Beispiel 1a hergestellten Liganden werden in 400 ml Wasser gelöst. Durch Zugabe von 10 molarer wäßriger Natronlauge wird pH 13 eingestellt und 5 Stunden bei Raumtemperatur gerührt. Nach vollzogener Verseifung der Estergruppen wird mit konzentrierter Salzsäure pH 7,0 eingestellt und wie unter Beispiel 1b beschrieben, mit 894,2 mg (2,2 mmol) Gadoliniumacetat-tetrahydrat komplexiert, aufgearbeitet und gereinigt.
Ausbeute: 1,19 g (70,1 % d. Th.) rotbraunes Pulver

| Analyse (bezogen auf wasserfreie Substanz): | | | | | |
|---|---|---|---|---|---|
| C 43,88<br>C 43,71 | H 4,51<br>H 4,30 | Gd 18,53<br>Gd 18,28 | Na 2,71<br>Na 2,80 | O 18,85 | ber. |

**Beispiel 2**

a) N,N'-Bis[13-Carboxy-4-oxo-6,9-bis-(carboxymethyl)-12-(ethoxycarboxymethyl)-3,6,9,12-tetraazatridecyl]-mesoporphyrin-IX-13,17-diamid

806,8 mg (2 mmol) 3-Ethoxy-carbonylmethyl-6-[2-(2,6-dioxomorpholino)ethyl]-3,6-diazaoctandisäure (DTPA-monoethylester-monoanhydrid) werden in 250 ml absolutem Dimethylformamid suspendiert. Man überschichtet mit Stickstoff, setzt 1,01 g (10 mmol) Triethylamin und 650,9 mg (1 mmol) N,N'-Bis(2-aminoethyl)-mesoporphyrin-IX-diamid

(hergestellt analog zu H. Fischer, E. Haarer und F. Stadler, Z. Physiol. Chem. 241, 209 (1936)) zu und rührt das resultierende Reaktionsgemisch 3 Tage bei Raumtemperatur. Nach beendeter Reaktion wird filtriert, das Lösungsmittel im Vakuum abgezogen und das verbleibende Öl mit 500 ml Diethylether verrieben. Der ausgeschiedene Feststoff wird mit Diethylether und Hexan gewaschen. Zur Reinigung wird an Kieselgel RP-18 chromatographiert (Eluens: $H_2O$/Tetrahydrofuran; Tetrahydrofuran: 0-30 %).
Ausbeute: 1,21 g (82,3 % d. Th.) rotbraunes Pulver

| Analyse (bezogen auf wasserfreie Substanz): | | | | |
|---|---|---|---|---|
| C 57,68<br>C 57,39 | H 6,92<br>H 7,18 | N 13,45<br>N 13,22 | O 21,95 | ber. |

b) N,N'-Bis[13-Carboxy-4-oxo-6,9-bis-(carboxymethyl)-12-(ethoxycarboxymethyl)-3,6,9,12-tetraazatridecyl]-mesoporphyrin-IX-13,17-diamid, Digadoliniumkomplex

1,458 g (1 mmol) des unter Beispiel 2a hergestellten Liganden werden in 400 ml Wasser gelöst. Durch Zugabe von 2n wäßriger Natronlauge wird pH 7 eingestellt und abwechselnd, portionsweise 894,2 mg (2,2 mmol) Gadoliniumacetat-tetrahydrat und 2n wäßrige Natronlauge zugesetzt, so daß der pH-Wert des Reaktionsgemisches stets zwischen 6,8 und 7,2 pendelt. Ist alles Gadoliniumacetat zugesetzt, wird über Nacht bei Raumtemperatur nachgerührt. Zur Aufarbeitung wird filtriert, das Lösungsmittel im Vakuum abgezogen und der Rückstand an Kieselgel RP-18 chromatographiert (Eluens: $H_2O$/Tetrahydrofuran; Tetrahydrofuran: O-30 %).
Ausbeute: 1,33 g (73,3 % d. Th.) rotbraunes Pulver

| Analyse (bezogen auf wasserfreie Substanz): | | | | | |
|---|---|---|---|---|---|
| C 47,61<br>C 47,32 | H 5,37<br>H 5,52 | N 11,10<br>N 10,85 | Gd 17,81<br>Gd 17,69 | O 18,12 | ber. |

c) N,N'-Bis[13-Carboxylato-4-oxo-6,8,12-tris-(carboxylatomethyl)-3,6,9,12-tetraazatridecyl]-mesoporphyrin-IX-13,17-diamid, Digadoliniumkomplex, Dinatriumsalz

1,458 g (1 mmol) des unter Beispiel 2a hergestellten Liganden werden in 400 ml Wasser gelöst. Durch Zugabe von 10 molarer wäßriger Natronlauge wird pH 13 eingestellt und 5 Stunden bis Raumtemperatur gerührt. Nach vollzogener Verseifung der Estergruppen wird mit konzentrierter Salzsäure pH 7 eingestellt und wie unter Beispiel 2b beschrieben mit 894,2 mg (2 mmol) Gadoliniumacetat-tetrahydrat komplexiert, aufgearbeitet und gereinigt.
Ausbeute: 980 mg (55,9 % d. Th.) rotbraunes Pulver

| Analyse (bezogen auf wasserfreie Substanz): | | | | | | |
|---|---|---|---|---|---|---|
| C 45,20<br>C 44,92 | H 4,83<br>H 5,09 | N 11,18<br>N 10,99 | Gd 17.93<br>Gd 17,71 | Na 2,62<br>Na 2,71 | O 18,24 | ber. |

**Beispiel 3**

a) N,N'-Bis-(3-Aminopropyl)-mesoporphyrin-IX-13,17-diamid

1 g (1,68 mmol) Mesoporphyrin-IX-dimethylester werden im Bombenrohr in 300 ml 1,3-Diaminopropan und 200 ml absolutem Pyridin suspendiert. Nach Überschichten mit Stickstoff wird im Bombenrohr 3 Tage auf 150°C erhitzt. Nach beendeter Reaktion wird im Vakuum eingedampft und der Rückstand wiederholt aus Pyridin/Diethylether umkristallisiert.
Ausbeute: 930 mg (81,5 % d. Th.) rotbraunes Pulver

| Analyse (bezogen auf wasserfreie Substanz): | | | | |
|---|---|---|---|---|
| C 70,76<br>C 70,49 | H 8,02<br>H 8,12 | N 16,50<br>N 16,31 | O 4,71 | ber. |

b) N,N'-Bis[14-Carboxy-5-oxo-7,10-bis-(carboxymethyl)-13-(ethoxycarboxymethyl)-4,7,10,13-tetraazatetradecyl]-mesoporphyrin-IX-13,17-diamid

806,8 mg (2 mmol) 3-Ethoxy-carbonylmethyl-6-[2-(2,6-dioxomorpholino)ethyl]-3,6-diazaoctandisäure (DTPA-monoethylester-monoanhydrid) werden in 250 ml absolutem Dimethylfarmamid suspendiert. Man überschichtet mit Stickstoff, setzt 1,01 g (10 mmol) Triethylamin und 679 mg (1 mmol) N,N'-Bis(3-Aminopropyl)-mesoporphyrin-IX-13,17-diamid (Beispiel 3a) zu und rührt das resultierende Reaktionsgemisch 3 Tage bei Raumtemperatur. Nach beendeter Reaktion wird filtriert, das Lösungsmittel im Vakuum abgezogen und das verbleibende Öl mit 500 ml Diethylether verrieben. Der ausgeschiedene Feststoff wird mit Diethylether und Hexan gewaschen. Zur Reinigung wird an Kieselgel RP-18 chromatographiert (Eluens: $H_2O$/Tetrahydrofuran; Tetrahydrofuran: 0-30 %).
Ausbeute: 1,20 g (80,8 % d. Th.) rotbraunes Pulver

| Analyse (bezogen auf wasserfreie Substanz): | | | | |
|---|---|---|---|---|
| C 58,21<br>C 57,93 | H 7,06<br>H 7,24 | N 13,20<br>N 13,01 | O 21,40 | ber. |

c) N,N'-Bis[14-Carboxylato-5-oxo-7,10-bis-(carboxylatomethyl)-13-(ethoxycarboxymethyl)-4,7,10,13-tetraazatetradecyl]-mesoporphyrin-IX-13,17-diamid, Digadoliniumkomplex

1,49 g (1 mmol) des unter Beispiel 3b) hergestellten Liganden werden in 400 ml Wasser gelöst. Durch Zugabe von 2n wäßriger Natronlauge wird pH 7 eingestellt und abwechselnd, portionsweise 894,2 mg (2,2 mmol) Gadoliniumacetat-tetrahydrat und 2n wäßrige Natronlauge zugesetzt, so daß der pH-Wert des Reaktionsgemisches stets zwischen 6,8 und 7,2 pendelt. Ist alles Gadoliniumacetat zugesetzt, wird über Nacht bei Raumtemperatur nachgerührt. Zur Aufarbeitung wird filtriert, das Lösungsmittel im Vakuum abgezogen und der Rückstand an Kieselgel RP-18 chromatographiert (Eluens: $H_2O$/Tetrahydrofuran; Tetrahydrofuran: 0-30 %).
Ausbeute: 1,01 g (56,3 % d. Th.) rotbraunes Pulver

| Analyse (bezogen auf wasserfreie Substanz): | | | | | |
|---|---|---|---|---|---|
| C 48,20<br>C 47,95 | H 5,51<br>H 5,71 | N 10,93<br>N 10,71 | Gd 17,53<br>Gd 17,24 | O 17,83 | ber. |

d) N,N'-Bis[14-Carboxylato-5-oxo-7,10,13-tris-(carboxylatomethyl)-4,7,10,13-tetraazatetradecyl]-mesoporphyrin-IX-13,17-diamid, Digadoliniumkomplex, Dinatriumsalz

1,49 g (1 mmol) des unter Beispiel 3b) hergestellten Liganden werden in 400 ml Wasser gelöst. Durch Zugabe von 10 molarer wäßriger Natronlauge wird pH 13 eingestellt und 5 Stunden bei Raumtemperatur gerührt. Nach vollzogener Verseifung der Estergruppen wird mit konzentrierter Salzsäure pH 7 eingestellt und wie unter Beispiel 3c beschrieben mit 894,2 mg (2,2 mmol) Gadoliniumacetat-tetrahydrat komplexiert, aufgearbeitet und gereinigt.
Ausbeute: 935 mg (52,5 % d. Th.) rotbraunes Pulver

| Analyse (bezogen auf wasserfreie Substanz): | | | | | | |
|---|---|---|---|---|---|---|
| C 45,83<br>C 45,58 | H 4,98<br>H 5,13 | N 11,00<br>N 10,79 | Gd 17,65<br>Gd 17,45 | Na 2,58<br>Na 2,72 | O 17,96 | ber. |

**Beispiel 4**

a) N,N'-Bis(4-Aminobutyl)-mesoporphyrin-IX-13,17-diamid

1 g (168 mmol) Mesoporphyrin-IX-dimethylester werden im Bombenrohr in 300 ml geschmolzenem 1,4-Diaminobutan und 200 ml absolutem Pyridin suspendiert. Nach Überschichten mit Stickstoff wird im Bombenrohr 3 Tage auf 150°C erhitzt. Man dampft im Vakuum ein, versetzt den Rückstand mit 500 ml Diethylether, filtriert den ausgeschiedenen Feststoff ab und wäscht mit reichlich Diethylether nach. Das Rohprodukt wird durch Umkristallisation aus Pyridin/Diethylether gereinigt.
Ausbeute: 953 mg (80,21 % d. Th.) rotbraunes Pulver

| Analyse (bezogen auf wasserfreie Substanz): | | | | |
|---|---|---|---|---|
| C 71,35<br>C 71,08 | H 8,27<br>H 8,10 | N 15,85<br>N 15,57 | O 4,53 | ber. |

b) N,N'-Bis[15-Carboxy-6-oxo-8,11-bis-(carboxymethyl)-14-(ethoxycarboxymethyl)-5,8,11,14-tetraazapentadecyl]-mesoporphyrin-IX-13,17-diamid

806,8 mg (2 mmol) 3-Ethoxy-carbonylmethyl-6-[2-(2,6-dioxomorpholino)ethyl]-3,6-diazaoctandisäure (DTPA-monoethylester-monoanhydrid) werden in 250 ml absolutem Dimethylformamid suspendiert. Man überschichtet mit Stickstoff, setzt 1,01 g (10 mmol) Triethylamin und 707 mg (1 mmol) N,N'-Bis-(4-Aminobutyl)-mesoporphyrin-IX-13,17-diamid (Beispiel 4a) zu und rührt das resultierende Reaktionsgemisch 3 Tage bei Raumtemperatur. Nach beendeter Reaktion wird filtriert, das Lösungsmittel im Vakuum abgezogen und das verbleibende Öl mit 500 ml Diethylether verreiben. Der ausgeschiedene Feststoff wird abfiltriert und mit Diethylether und Hexan gewaschen. Zur Reinigung wird an Kieselgel RP-18 chromatographiert (Eluens: Tetrahydrofuran/$H_2O$; Tetrahydrofuran: 0-30 %).
Ausbeute: 1,24 g (81,9 % d. Th.) rotbraunes Pulver

| Analyse (bezogen auf wasserfreie Substanz): | | | | |
|---|---|---|---|---|
| C 58,72<br>C 58,51 | H 7,19<br>H 7,28 | N 12,95<br>N 12,68 | O 21,14 | ber. |

c) N,N'-Bis[15-Carboxylato-6-oxo-8,11-bis-(carboxylatomethyl)-14-(ethoxycarboxymethyl)-5,8,11,14-tetraazapentadecyl]-mesoporphyrin-IX-13,17-diamid, Digadoliniumkomplex

1,51 g (1 mmol) des unter Beispiel 4b hergestellten Liganden werden in 400 ml Wasser gelöst. Durch Zugabe von 2n wäßriger Natronlauge wird pH 7 eingestellt und abwechselnd, portionsweise 894,2 mg (2,2 mmol) Gadoliniumacetat-tetrahydrat und 2n wäßrige Natronlauge zugesetzt, so daß der pH-Wert des Reaktionsgemisches stets zwischen 6,8 und 7,2 pendelt. Ist alles Gadoliniumacetat zugesetzt, wird über Nacht bei Raumtemperatur nachgerührt. Zur Aufarbeitung wird filtriert, das Lösungsmittel im Vakuum abgezogen und der Rückstand an Kieselgel RP-18 chromatographiert.
(Eluens: $H_2O$/Tetrahydrofuran; Tetrahydrofuran: 0-30 %).
Ausbeute: 1,31 g (71,9 % d. Th.) rotbraunes Pulver

| Analyse (bezogen auf wasserfreie Substanz): | | | | | |
|---|---|---|---|---|---|
| C 48,78<br>C 48,49 | H 5,64<br>H 5,83 | N 10,76<br>N 10,48 | Gd 17,26<br>Gd 17,06 | O 17,56 | ber. |

d) N,N'-Bis[15-Carboxylato-6-oxo-8,11,14-tris-(carboxylatomethyl)-5,8,11,14-tetraazapentadecyl]-mesoporphyrin-IX-13,17-diamid, Digadoliniumkomplex, Dinatriumsalz

1,51 g (1 mmol) des unter Beispiel 4b hergestellten Liganden werden in 400 ml Wasser gelöst. Durch Zugabe von 10 molarer wäßriger Natronlauge wird pH 13 eingestellt und 5 Stunden bei Raumtemperatur gerührt. Nach vollzogener Verseifung der Estergruppen wird mit konzentrierter Salzsäure pH 7 eingestellt und wie unter Beispiel 4c beschrieben mit 894,2 mg (2,2 mmol) Gadoliniumacetat-tetrahydrat komplexiert, aufgearbeitet und gereinigt.
Ausbeute: 1,29 g (71,3 % d. Th.) rotbraunes Pulver

| Analyse (bezogen auf wasserfreie Substanz): | | | | | | |
|---|---|---|---|---|---|---|
| C 46,45<br>C 46,42 | H 5,12<br>H 5,23 | N 10,83<br>N 10,75 | Gd 17,37<br>Gd 17,27 | Na 2,54<br>Na 2,61 | O 17,68 | ber. |

**Beispiel 5**

a) N,N'-Bis(5-Aminopentyl)-mesoporphyrin-IX-13,17-diamid

1 g (1,68 mmol) Mesoporphyrin-IX-dimethylester werden im Bombenrohr in 300 ml geschmolzenem 1,5-Diaminopentan und 200 ml absolutem Pyridin suspendiert. Nach überschichten mit Stickstoff wird im Bombenrohr 3 Tage auf 150°C erhitzt. Man dampft im Feinvakuum ein, versetzt den Rückstand mit 500 ml Diethylether, filtriert den ausgeschiedenen Feststoff ab und wäscht mit reichlich Diethylether nach. Das Rohprodukt wird durch Umkristallisation aus Pyridin/Diethylether gereinigt.
Ausbeute: 932 mg (75,5 % d. Th.) rotbraunes Pulver

| Analyse (bezogen auf wasserfreie Substanz): | | | | |
|---|---|---|---|---|
| C 71,90 | H 8,50 | N 15,24 | O 4,35 | ber. |
| C 71,92 | H 8,35 | N 14,99 | | |

b) N,N'-Bis[16-Carboxy-7-oxo-9,12-bis-(carboxymethyl)-15-(ethoxycarboxymethyl)-6,9,12,15-tetraazahexadecyl]-mesoporphyrin-IX-13,17-diamid

806,8 mg (2 mmol) 3-Ethoxy-carbonylmethyl-6-[2(2,6-dioxomorpholino)ethyl]-3,6-diazaoctandisäure (DTPA-monoethylester-monoanhydrid) werden in 250 ml absolutem Dimethylformamid suspendiert. Man überschichtet mit Stickstoff, setzt 1,01 g (10 mmol) Triethylamin und 735 mg (1 mmol) N,N'-Bis(5-Aminopentyl)-mesoporphyrin-IX-13,17-diamid (Beispiel 5a) zu und rührt das resultierende Reaktionsgemisch 3 Tage bei Raumtemperatur. Nach beendeter Reaktion wird filtriert, das Lösungsmittel im Vakuum abgezogen und das verbleibende Öl mit 500 ml Diethylether verrieben. Der ausgefallene Feststoff wird abfiltriert und mit Diethylether und Hexan gewaschen. Zur Reinigung wird an Kieselgel RP-18 chromatographiert (Eluens: Tetrahydrofuran/H2O; Tetrahydrofuran: 0-30 %)
Ausbeute: 1,31 g (85,0 % d. Th.) rotbraunes Pulver

| Analyse (bezogen auf wasserfreie Substanz): | | | | |
|---|---|---|---|---|
| C 59,21 | H 7,32 | N 12,72 | O 20,75 | ber. |
| C 59,00 | H 7,51 | N 12,47 | | |

c) N,N'-Bis[16-Carboxylato-7-oxo-9,12-bis-(carboxylatomethyl)-15-(ethoxycarboxymethyl)-6,9,12,15-tetraazahexadecyl]-mesoporphyrin-IX-13,17-diamid, Digadoliniumkomplex

1,54 g (1 mmol) des unter Beispiel 5b hergestellten Liganden werden in 400 ml Wasser gelöst. Durch Zugabe von 2n wäßriger Natronlauge wird pH 7 eingestellt und abwechselnd, portionsweise 894,2 mg (2,2 mmol) Gadoliniumacetat-tetrahydrat und 2n wäßrige Natronlauge zugesetzt, so daß der pH-Wert des Reaktionsgemisches stets zwischen 6,8 und 7,2 pendelt. Ist alles Gadoliniumacetat zugesetzt, wird über Nacht bei Raumtemperatur nachgerührt. Zur Aufarbeitung wird filtriert, das Lösungsmittel im Vakuum abgezogen und der Rückstand an Kieselgel RP-18 chromatographiert (Eluens: Tetrahydrofuran/$H_2O$; Tetrahydrofuran: 0-30 %)
Ausbeute: 1,26 g (68,1 % d. Th.) rotbraunes Pulver

| Analyse (bezogen auf wasserfreie Substanz): | | | | | |
|---|---|---|---|---|---|
| C 49,34 | H 5,77 | N 10,60 | Gd 17,00 | O 17,29 | ber. |
| C 49,08 | H 6,02 | N 10,41 | Gd 16,73 | | |

d) N,N'-Bis[16-Carboxylato-7-oxo-9,12,15-tris-(carboxylatomethyl)-6,9,12,15-tetraazahexadecyl]-mesoporphyrin-IX-13,17-diamid, Digadoliniumkomplex, Dinatriumsalz

1,54 g (1 mmol) des unter Beispiel Sb hergestellten Liganden werden in 400 ml Wasser gelöst. Durch Zugabe von 10 molarer wäßriger Natronlauge wird pH 13 eingestellt und 5 Stunden bei Raumtemperatur gerührt. Nach vollzogener Verseifung der Estergruppen wird mit konzentrierter Salzsäure pH 7 eingestellt und wie unter Beispiel 5c beschrieben, mit 894,2 mg (2,2 mmol) Gadoliniumacetat-tetrahydrat komplexiert, aufgearbeitet und gereinigt.
Ausbeute: 1,49 g (81,1 % d. Th.) rotbraunes Pulver

| Analyse (bezogen auf wasserfreie Substanz): | | | | | | |
|---|---|---|---|---|---|---|
| C 47,05 | H 5,26 | N 10,67 | Gd 17,11 | Na 2,50 | O 17,11 | ber. |
| C 39,87 | H 5,45 | N 10,38 | Gd 17,01 | Na 2,73 | | |

**Beispiel 6**

a) N,N'-Bis-(4-Aminomethylbenzyl)-mesoporphyrin-IX-13,17-diamid

1 g (1,68 mmol) Mesoporphyrin-IX-dimethylester werden in 300 ml geschmolzenem p-Xylylendiamin und 200 ml absolutem Pyridin im Bombenrohr suspendiert. Nach überschichten mit Stickstoff wird das Reaktionsgemisch 3 Tage bei 150°C gerührt. Nach beendeter Reaktion wird das Pyridin und der Überschuß an p-Xylylendiamin im Feinvakuum abdestilliert und der Rückstand wiederholt aus Pyridin/Diethylether umkristallisiert.
Ausbeute: 921 mg (68,3 % d. Th.) rotbraunes Pulver

| Analyse (bezogen auf wasserfreie Substanz): | | | | |
|---|---|---|---|---|
| C 74,78 | H 7,28 | N 13,95 | O 3,98 | ber. |
| C 74,51 | H 7,15 | N 13,78 | | |

b) N,N'-Bis{4-[12-Carboxy-3-oxo-5,8-bis-(carboxymethyl)-11-(ethoxycarboxymethyl)-2,5,8,11-tetraazadodecyl]benzyl}-mesoporphyrin-IX-13,17-diamid

806,8 mg (2 mmol) 3-Ethoxy-carbonylmethyl-6-[2-(2,6-dioxomorpholino)ethyl]-3,6-diazaoctandisäure (DTPA-monoethylestermonoanhydrid) werden in 250 ml absolutem Dimethylformamid suspendiert. Man überschichtet mit Stickstoff, setzt 1,01 g (10 mmol) Triethylamin und 803 mg (1 mmol) N,N'-Bis-(4-Aminomethylbenzyl)-mesoporphyrin-IX-13,17-diamid (Beispiel 6a) zu und rührt das resultierende Reaktionsgemisch 3 Tage bei Raumtemperatur. Nach beendeter Reaktion wird filtriert, das Lösungsmittel im Vakuum abgezogen und das verbleibende Öl mit 500 ml Diethylether verrieben. Der ausgeschiedene Feststoff wird abfiltriert und mit Diethylether und Hexan gewaschen. Zur Reinigung wird an Kieselgel RP-18 chromatographiert (Eluens: Tetrahydrofuran/$H_2O$; Tetrahydrofuran: 0-30 %).
Ausbeute: 923 mg (57,3 % d. Th.) rotbraunes Pulver

| Analyse (bezogen auf wasserfreie Substanz): | | | | |
|---|---|---|---|---|
| C 61,18 | H 6,76 | N 12,18 | O 19,88 | ber. |
| C 61,02 | H 6,92 | N 11,98 | | |

c) N,N'-Bis{4-[12-Carboxy-3-oxo-5,8-bis-(carboxylatomethyl)-11-(ethoxycarboxymethyl)-2,5,8,11-tetraazadodecyl] benzyl}-mesoporphyrin-IX-13,17-diamid, Digadoliniumkomplex

1,61 g (1 mmol) des unter Beispiel 6b hergestellten Liganden werden in 400 ml Wasser gelöst. Durch Zugabe von 2n wäßriger Natronlauge wird pH 7 eingestellt und abwechselnd portionsweise 894,2 mg (2,2 mmol) Gadoliniumacetat-tetrahydrat und 2n wäßrige Natronlauge zugesetzt, so daß der pH-Wert des Reaktionsgemisches stets zwischen 6,8 und 7,2 pendelt. Ist alles Gadoliniumacetat zugesetzt, wird über Nacht bei Raumtemperatur nachgerührt. Zur Aufarbeitung wird filtriert, das Lösungsmittel im Vakuum abgezogen und der Rückstand an Kieselgel RP-18 chromatographiert (Eluens: Tetrahydrofuran/$H_2O$; Tetrahydrofuran: 0-30 %).
Ausbeute: 985 mg (51,3 % d. Th.) rotbraunes Pulver

| Analyse (bezogen auf wasserfreie Substanz): | | | | | |
|---|---|---|---|---|---|
| C 51,34 | H 5,36 | N 10,22 | Gd 16,39 | O 16,68 | ber. |
| C 51,08 | H 5,47 | N 9,91 | Gd 16,22 | | |

d) N,N'-Bis{4-[12-carboxylato-3-oxo-5,8,11-tris-(carboxylatomethyl)-2,5,8,11-tetraazadodecyl]benzyl}-mesoporphyrin-IX-13,17-diamid, Digadoliniumkomplex, Dinatriumsalz

1,61 g (1 mmol) des unter Beispiel 6b hergestellten Liganden werden in 400 ml Wasser gelöst. Durch Zugabe

von 10 molarer wäßriger Natronlauge wird pH 13 eingestellt und 5 Stunden bei Raumtemperatur gerührt. Nach vollzogener Verseifung der Estergruppen wird mit konzentrierter Salzsäure pH 7 eingestellt und wie unter Beispiel 6c beschrieben mit 894,2 mg (2,2 mmol) Gadoliniumacetat-tetrahydrat komplexiert, aufgearbeitet und gereinigt.
Ausbeute: 1,23 g (64,5 % d. Th.) rotbraunes Pulver

| Analyse (bezogen auf wasserfreie Substanz): | | | | | | |
|---|---|---|---|---|---|---|
| C 49,15<br>C 48,98 | H 4,87<br>H 4,95 | N 10,29<br>N 10,12 | Gd 16,50<br>Gd 16,31 | Na 2,41<br>Na 2,53 | O 16,79 | ber. |

**Beispiel 7**

a) N,N'-Bis-butyl-mesoporphyrin-IX-13,17-diamid

1 g (1,68 mmol) Mesoporphyrin-IX-dimethylester werden im Bombenrohr in 100 ml absolutem Pyridin und 300 ml n-Butylamin suspendiert. Man überschichtet mit Stickstoff und erhitzt das Reaktionsgemisch drei Tage auf 150°C. Nach beendeter Reaktion wird im Vakuum eingedampft, der Rückstand in 500 ml Chloroform aufgenommen, dreimal mit 100 ml 10%iger wäßriger Citronensäure, dreimal mit 100 ml gesättigter Natriumhydrogencarbonatlösung gewaschen, über Natriumsulfat getrocknet und das Lösungsmittel im Vakuum abgezogen. Die Reinigung des Amids erfolgt durch Chromatographie an Aluminiumoxid (Eluens: Chloroform/Methanol 99:1).
Ausbeute: 1,03 g (90,5 % d. Th.) violettes Pulver

| Analyse (bezogen auf wasserfreie Substanz): | | | | |
|---|---|---|---|---|
| C 74,52<br>C 74,28 | H 8,34<br>H 8,44 | N 12,41<br>N 12,32 | O 4,73 | ber. |

b) 3,8-Diethyl-2,7,12,18-tetramethyl-13,17-bis(n-butylaminopropyl)-porphyrin

677 mg (1 mmol) N,N'-Bis-butyl-mesoporphyrin-IX-13,17-diamid (Beispiel 7a) werden unter Stickstoffatmosphäre in 150 ml absolutem Tetrahydrofuran gelöst. Man versetzt mit 350 mg (16,1 mmol) Lithiumborhydrid, 2 ml Trimethylsilylchlorid und rührt das resultierende Reaktionsgemisch 3 Tage bei Raumtemperatur. Nach beendeter Reaktion werden 20 ml Methanol und anschließend 200 ml Wasser zugetropft, mit 2 molarer Salzsäure pH 1 eingestellt, 0,5 Stunden gerührt, mit 2 molarer wäßriger Natriumhydroxidlösung pH 13 eingestellt und erneut 0,5 Stunden gerührt. Man extrahiert das Reaktionsprodukt dreimal mit 100 ml Chloroform und trocknet die organische Phase über Natriumsulfat. Die Reinigung des Amins geschieht durch Chromatographie an Kieselgel (Eluens: Chloroform/Methanol; Methanol: 0-50 %).
Ausbeute: 362 mg (55,8 % d. Th.) violettes Pulver

| Analyse (bezogen auf wasserfreie Substanz): | | | |
|---|---|---|---|
| C 77,73<br>C 77,48 | H 9,32<br>H 9,30 | N 12,95<br>N 12,76 | ber. |

c) 3,8-Diethyl-2,7,12,18-tetramethyl-13,17-bis[4,7,10,13-tetraaza-4-butyl-5-oxo-7,10,13-tris(carboxymethyl)-13-ethoxycarboxymethyl-tridecyl)-porphyrin

806,8 mg (2 mmol) 3-Ethoxy-carbonylmethyl-6-[2-(2,6-dioxomorpholino)ethyl]-3,6-diazaoctandisäure (DTPA-monoethylester-monoanhydrid) werden in 250 ml absolutem Dimethylformamid suspendiert. Man überschichtet mit Stickstoff, setzt 1,01 g (10 mmol) Triethylamin und 649 mg (1 mmol) 3,8-Diethyl-2,7,12,18-tetramethyl-13,17-bis(n-butylaminopropyl)-porphyrin (Beispiel 7b) zu und rührt das resultierende Reaktionsgemisch drei Tage bei Raumtemperatur. Nach beendeter Reaktion wird filtriert, das Lösungsmittel im Vakuum abgezogen und das verbleibende Öl mit 500 ml Diethylether verrieben. Der ausgefallene Feststoff wird abfiltriert und mit Diethylether und n-Hexan gewaschen. Zur Reinigung wird an Kieselgel RP-18 chromatographiert (Eluens: $H_2O$/Tetrahydrofuran;
Tetrahydrofuran: 0-30 %).
Ausbeute: 1.05 g (72,1 % d. Th.) rotbraunes Pulver

| Analyse (bezogen auf wasserfreie Substanz): | | | | |
|---|---|---|---|---|
| C 61,05<br>C 60,89 | H 7,62<br>H 7,72 | N 11,55<br>N 11,31 | O 19,78 | ber. |

d) 3,8-Diethyl-2,7,12,18-tetramethyl-13,17-bis-[4,7,10,13-tetraaza-4-butyl-5-oxo-7,10,13-tris(carboxylatomethyl)-13-ethoxycarboxymethyl-tridecyl]-porphyrin, Digadoliniumkomplex

1,46 (1 mmol) des unter Beispiel 7c hergestellten Liganden werden in 400 ml Wasser gelöst. Durch Zugabe von zweimolarer Natronlauge wird pH 7 eingestellt und abwechselnd, portionsweise 894,2 mg (2,2 mmol) Gadoliniumacetat-tetrahydrat und zweimolare Natronlauge zugesetzt, so daß der pH-Wert des Reaktionsgemisches stets zwischen 6,8 und 7,2 pendelt. Ist alles Gadoliniumaceta zugesetzt, wird über Nacht bei Raumtemperatur nachgerührt. Zur Aufarbeitung wird das Lösungsmittel im Vakuum abgezogen und der Rückstand an Kieselgel R 18 chromatographiert (Eluens: $H_2O$/Tetrahydrofuran; Tetrahydrofuran: 0-30 %).
Ausbeute: 721 mg (40,9 % d. Th.), rotbraunes Pulver

| Analyse (bezogen auf wasserfreie Substanz): | | | | | |
|---|---|---|---|---|---|
| C 50,38<br>C 50,02 | H 5,94<br>H 5,99 | N 9,53<br>N 9,38 | Gd 17,83<br>Gd 17,73 | O 16,32 | ber. |

e) 3,8-Diethyl-2,7,12,18-tetramethyl-13,17-bis[4,7,10,13-tetraaza-4-butyl-5-oxo-7,10,13,13,-tetra(carboxylatomethyl)-tridecyl]-porphyrin, Digadoliniumkomplex, Dinatriumsalz

1,46 g (1 mmol) des unter Beispiel 7c hergestellten Liganden werden in 400 ml Wasser gelöst. Durch Zugabe von 10 molarer wäßriger Natronlauge wird pH 13 eingestellt und 5 Stunden bei Raumtemperatur gerührt. Nach vollzogener Verseifung der Estergruppen wird mit konzentrierter Salzsäure pH 7,0 eingestellt und wie unter Beispiel 7d beschrieben mit 894,2 mg (2,2 mmol) Gadoliniumacetat-tetrahydrat komplexiert, aufgearbeitet und gereinigt.
Ausbeute: 1,32 g (75,3 % d. Th.) rotbraunes Pulver

| Analyse (bezogen auf wasserfreie Substanz): | | | | | | |
|---|---|---|---|---|---|---|
| C 47,99<br>C 47,76 | H 5,41<br>H 5,63 | N 9,59<br>N 9,31 | Gd 17,95<br>Gd 17,81 | Na 2,62<br>Na 2,79 | O 16,44 | ber. |

**Beispiel 8**

a) N,N'-Bis-benzyl-mesoporphyrin-IX-13,17-diamid

1 g (1,68 mmol) Mesoporphyrin-IX-dimethylester werden in 500 ml Benzylamin unter Stickstoffatmosphäre 48 Stunden unter Rückfluß gekocht. Nach beendeter Reaktion wird im Vakuum eingedampft, der Rückstand in 500 ml Chloroform aufgenommen, die organische Phase dreimal mit 150 ml 5 %iger wässriger Citronensäure gewaschen, über Natriumsulfat getrocknet und im Vakuum eingedampft. Man kristallisiert aus Chloroform/Methanol.
Ausbeute: 920 mg (73,5 % d. Th.) rotviolettes Pulver

| Analyse (bezogen auf wasserfreie Substanz): | | | |
|---|---|---|---|
| C 77,39<br>C 77,41 | H 7,04<br>H 6,93 | N 11,28<br>N 11,42 | ber. |

b) 3,8-Diethyl-2,7,12,18-tetramethyl-13,17-bis(benzylaminopropyl)-porphyrin

745 mg (1 mmol) N,N'-Bis-benzyl-mesoporphyrin-13,17-diamid werden unter Stickstoffatmosphäre in 150 ml absolutem Tetrahydrofuran gelöst. Man versetzt mit 350 mg (16,1 mmol) Lithiumborhydrid, 2 ml Trimethylsilylchlorid und rührt das resultierende Reaktionsgemisch 3 Tage bei Raumtemperatur. Nach beendeter Reaktion werden 20 ml Methanol und anschließend 200 ml Wasser zugetropft, mit 2 molarer Salzsäure pH 1 eingestellt, 0,5 Stunden gerührt, mit 2 molarer Natronlauge pH 13 eingestellt und erneut 0,5 Stunden gerührt. Man extrahiert das Reaktionsprodukt

dreimal mit 100 ml Chloroform und trocknet die organische Phase über Natriumsulfat. Die Reinigung des Amins geschieht durch Chromatographie an Kieselgel (Eluens: Chloroform/Methanol; Methanol: 0-50 %).
Ausbeute: 425 mg (59,3 % d. Th.) rotviolettes Pulver

| Analyse (bezogen auf wasserfreie Substanz): | | | |
|---|---|---|---|
| C 80,41<br>C 80,20 | H 7,87<br>H 7,92 | N 11,72<br>N 11,45 | ber. |

c) 3,8-Diethyl-2,7,12,18-tetramethyl-13,17-bis[4,7,10,13-tetraaza-4-benzyl-5-oxo-7,10,13-tris(carboxymethyl)-13-ethoxycarboxymethyl-tridecyl]-porphyrin

806,8 mg (2 mmol) 3-Ethoxy-carbonylmethyl-6-[2-(2,6-dioxomorpholino)ethyl]-3,6-diazactandisäure (DTPA-monoethylester-monoanhydrid) werden in 250 ml absolutem Dimethylformamid suspendiert. Man überschichtet mit Stickstoff, setzt 1,01 g (10 mmol) Triethylamin und 717mg(1 mmol) 3,8-Diethyl-2,7,12,18-tetramethyl-13,17-bis(benzylaminopropyl)porphyrin (Beispiel 8b) zu und rührt das resultierende Reaktionsgemisch drei Tage bei Raumtemperatur. Nach beendeter Reaktion wird filtriert, das Lösungsmittel im Vakuum abgezogen und das verbleibende Öl mit 500 ml Diethylether verrieben. Der ausgefallene Feststoff wird abfiltriert und mit Diethylether und n-Hexan gewaschen. Zur Reinigung wird an Kieselgel RP-18 chromatographiert (Eluens: $H_2O$/Tetrahydrafuran;
Teuahydrofuran: 0-30 %).
Ausbeute: 1,25 g (82 % d. Th.) rotbraunes Pulver

| Analyse (bezogen auf wasserfreie Substanz): | | | | |
|---|---|---|---|---|
| C 63,06<br>C 62,89 | H 7,01<br>H 7,14 | N 11,03<br>N 11,18 | O 18,90 | ber. |

d) 3,8-Diethyl-2,7,12,18-tetramethyl-13,17-bis[4,7,10,13-tetraaza-4-benzyl-5-oxo-7,10,13-tris(carboxylatomethyl)-13-ethoxycarboxymethyl-tridecyl]-porphyrin, Digadoliniumkomplex

1,52 g (1 mmol) des unter Beispiel 8c hergestellten Liganden werden in 400 ml Wasser gelöst. Durch Zugabe von zweimolarer Natronlauge wird pH 7 eingestellt und abwechselnd, portionsweise 894,2 mg (2,2 mmol) Gadoliniumacetat-tetrahydrat und zweimolare Natronlauge zugesetzt, so daß der pH-Wert des Reaktionsgemisches stets zwischen 6,8 und 7,2 pendelt. Ist alles Gadoliniumacetat zugesetzt, wird über Nacht bei Raumtemperatur nachgerührt. Zur Aufarbeitung wird das Lösungsmittel im Vakuum abgezogen und der Rückstand an Kieselgel RP-18 chromatographiert. (Eluens: $H_2O$/Tetrahydrofuran; Tetrahydrofuran: 0-30 %).
Ausbeute: 1,01 g (55,1 % d. Th.) rotbraunes Pulver

| Analyse (bezogen auf wasserfreie Substanz): | | | | | |
|---|---|---|---|---|---|
| C 52,44<br>C 52,17 | H 5,50<br>H 5,81 | N 9,17<br>N 9,03 | Gd 17,16<br>Gd 17,02 | O 15,72 | ber. |

e) 3,8-Diethyl-2,7,12,18-tetramethyl-13,17-bis[4,7,10,13-tetraaza-4-benzyl-5-oxo-7,10,13,13-tetra(carboxylatomethyl)-tridecyl]-porphyrin, Digadoliniumkomplex, Dinatriumsalz

1,52 g (1 mmol) des unter Beispiel 8c hergestellten Liganden werden in 400 ml Wasser gelöst. Durch Zugabe von 10 molarer, wäßriger Natronlauge wird pH 13 eingestellt und 5 Stunden bei Raumtemperatur gerührt. Nach vollzogener Verseifung der Estergruppen wird mit konzentrierter Salzsäure pH 7 eingestellt und wie unter Beispiel 8d beschrieben, mit 894,2 mg (2,2 mmol) Gadoliniumacetat-tetrahydrat komplexiert, aufgearbeitet und gereinigt.
Ausbeute: 1,12 g (61,5 % d. Th.) rotbraunes Pulver

| Analyse (bezogen auf wasserfreie Substanz): | | | | | | |
|---|---|---|---|---|---|---|
| C 50,15<br>C 50,02 | H 4,98<br>H 4,99 | N 9,23<br>N 9,05 | Gd 17,28<br>Gd 17,03 | Na 2,53<br>Na 2,68 | O 15,82 | ber. |

**Beispiel 9**

a) 10-[2,6,7-Trihydroxy-4-oxa-heptyl]-1,4,7-tris-carboxymethyl-1,4,7,10-tetraazacyclododecan

19,56 g (103,92 mmol) 2,2-Dimethyl-4-(2',3'-epoxy)-propoxy-methyl-1,3-dioxolan und 10 g (28,86 mmol) 1,4,7-Triscarboxymethyl-1,4,7,10-tetraazacyclododecan (=DO3A) werden in einer Mischung aus 50 ml Dioxan/80 ml Wasser gelöst, und der pH-Wert mit 6N Kalilauge auf pH 10 gebracht. Man rührt 24 Stunden bei 70°C. Man dampft zur Trockne ein, nimmt den Rückstand mit 200 ml Wasser/50 ml Methanol auf und extrahiert 2 mal mit 100 ml tert. -Butyl-methylether. Die wäßrige Lösung wird mit 5N-Salzsäure auf pH 3 gestellt und zur Trockne eingedampft. Der Rückstand wird mit 200 ml Methanol/80 ml Dichlormethan ausgekocht (extrahiert). Man kühlt im Eisbad ab und filtriert vom ausgefallenen Kaliumchlorid ab. Das Filtrat wird im Vakuum eingedampft, der Rückstand in 45 ml Wasser/20 ml Ethanol gelöst und anschließend auf eine Säule aus Poly-(4-vinylpyridin) gegeben. Das Produkt wird mit einer Lösung aus Ethanol/Wasser 1:3 eluiert. Nach Eindampfen im Vakuum wird der Rückstand an einer Reversed Phase-Säule (RP-18/Lauftnittel, Gradient aus Wasser/Tetrahydrofuran) chromatographiert. Nach Eindampfen der Hauptfraktion erhält man 10,13 g (71 % d. Th.) eines stark hygroskopischen, glasigen Feststoffes.

| Analyse (bezogen auf wasserfreie Substanz): | | | |
|---|---|---|---|
| C 48,57 | H 7,74 | N 11,33 | ber. |
| C 48,46 | H 7,81 | N 11,24 | |

b) Gd-Komplex des 10-(2,6,7-Trihydroxy-4-oxa-heptyl)-1,4,7-tris-carboxymethyl-1,4,7,10-tetraazacyclododecans

8,56 g (17,3 mmol) der Titelverbindung aus Beispiel 9a werden in 50 ml entionisiertem Wasser gelöst und 3,13 g (8,65 mmol) Gadoliniumoxid zugesetzt. Es wird 3 Stunden auf 90°C erhitzt. Die abgekühlte Lösung wird eine Stunde mit 3 ml saurem Ionenaustauscher (AMB 252c) und 3 ml schwach basischem Austauscher (IRA 67) gerührt. Es wird vom Austauscher abfiltriert und das Filtrat gefriergetrocknet.
Ausbeute: 11,0 g (98 % d. Th.) eines farblosen amorphen Pulvers

| Analyse (bezogen auf wasserfreie Substanz): | | | | |
|---|---|---|---|---|
| C 37,03 | H 5,44 | N 8,64 | Gd 24,24 | ber. |
| C 37,00 | H 5,51 | N 8,57 | Gd 24,18 | |

In analoger Weise erhält man mit Yttriumoxid den entsprechenden Yttrium-Komplex des 10-(2,6,7-Trihydroxy-4-oxa-heptyl)-1,4,7-tris-carboxymethyl-1,4,7,10-tetraazacyclododecans.

c) N,N'-Bis{4,7,10-tris(carboxylatomethyl)-1,4,7,10-tetraazacyclodecyl}-5-hydroxy-3-oxahexylamino}-mesoporphyrin-IX-13,17-diamid, Digadoliniumkomplex

3,89 g (6 mmol) des Gd-Komplexes aus Beispiel 9b werden in 40 ml Methanol gelöst, mit 2,57 g (12 mmol) Natriumperiodat versetzt und 4 Stunden unter Lichtausschluß verrührt. Dann wird vom Ungelösten abfiltriert und das Filtrat gefriergetrocknet. Der Rückstand wird mit 500 ml absolutem Dimethylformamid versetzt. Man überschichtet mit Stickstoff, setzt 6,06 g (60 mmol) Triethylamin und 1,78 g (3 mmol) Mesoporphyrin-IX-13,17-dihydrazid zu und läßt 3 Tage bei Raumtemperatur rühren. Man engt im Vakuum zur Trockne ein, versetzt den Rückstand mit 75 ml Puffer pH 9,0 (Riedel de Haen, Borax/HCl). gibt 1,13 g (18 mmol) Natriumcyanoborhydrid zu und läßt nochmals 6 Tage unter Stickstoff bei Raumtemperatur rühren. Nach Chromatographie der neutralen Lösung an Kieselgel
RP-18 erhält man 2,8 g (52 % d. Th.) rotbraunes Pulver

| Analyse (bezogen auf wasserfreie Substanz): | | | | |
|---|---|---|---|---|
| C 48,20 | H 5,73 | N 12,49 | Gd 17,52 | ber. |
| C 48,38 | H 5,90 | N 12,27 | Gd 17,63 | |

Aus dem Yttriumkomplex (Beispiel 9b) erhält man in analoger Weise den Di-Yttriumkomplex.

d) N,N'-Bis{4,7,10-tris(carboxylatomethyl)-1,4,7,10-tetraazacyclododecyl}-5-hydoxy-3-oxahexyl-(1'-oxo-3'-oxa-4'-carboxylatobutyl)-amino}-mesoporphyrin-IX-13,17-diamid, Digadoliniumkomplex, Dinatriumsalz

2,5 g (1,41 mmol) der Titelverbindung aus Beispiel 9c werden in 200 ml N,N-Dimethylfomamid gelöst und 10 ml Pyridin zugesetzt. Dann gibt man 3,48 g (30 mmol) Diglykolsäureanhydrid zu und rührt 4 Tage bei Raumtemperatur. Man engt im Vakuum zur Trockne ein, nimmt den Rückstand mit 20 ml 20 %iger wässriger Essigsäure auf und dampft erneut zur Trockene ein. Der Rückstand wird an Kieselgel RP-18 chromatographiert. Die Hauptfraktionen werden vereinigt und zur Trockne eingedampft. Der Rückstand wird in 200 ml Wasser gelöst und der pH-Wert der Lösung mit 0,1 N Natronlauge auf pH 7,2 eingestellt. Die Lösung wird filtiert und das Filtrat getrocknet. Man erhält 2,17 g (75 % d. Th.) eines rotbraunes Pulvers.

| Analyse (bezogen auf wasserfreie Substanz): | | | | | |
|---|---|---|---|---|---|
| C 45,74<br>C 45,51 | H 5,41<br>H 5,60 | N 10,94<br>N 10,72 | Gd 15,35<br>Gd 15,14 | Na 2,24<br>Na 2,01 | ber. |

**Beispiel 10**

a) 3,8-Bis-(hydroxymethyl)-deuteroporphyrin-IX-dimethylester

1,00 g (1,67 mmol) 3,8-Diformyl-deuteroporphyrin-IX-dimethylester (H. Fischer, K.O. Deilmann, Hoppe Seyler's Z.phys.Chem. 280, 186-216 (1944)) werden in einem Gemisch aus 700 ml Chloroform und 300 ml Methanol gelöst. Die Lösung wird auf 0°C gekühlt und unter Rühren portionsweise mit 1,00 g (26,43 mmol) Natriumborhydrid versetzt. Man läßt den Ansatz auf Raumtemperatur aufwärmen, neutralisiert mit halbkonzentrierter Essigsäure und engt im Vakuum bis zur Trockne ein. Der Rückstand wird in Methylenchlorid aufgenommen, mit gesättigter Natriumhydrogencarbonatlösung ausgeschüttelt, über Natriumsulfat getrocknet, filtriert, eingedampft und aus Methylenchlorid/Diethylether umkristallisiert.
Ausbeute: 0,92 g (92 % d. Th.) rotbraunes Pulver

| Analyse (bezogen auf wasserfreie Substanz): | | | | |
|---|---|---|---|---|
| C 68,21<br>C 68,01 | H 6,40<br>H 6,35 | N 9,36<br>N 9,22 | O 16,03 | ber. |

b) 3,8-Bis-(methoxymethyl)-deuteroporphyrin-IX-dimethylester

0,90 g (1,44 mmol) 3,8-Bis-(hydroxymethyl)-deuteroporphyrin-IX-dimethylester (Beispiel 10a) werden in einem Gemisch aus 40 ml Orthoameisensäuretrimethylester, 40 ml Methanol und 8 ml Schwefelsäure 2 Stunden lang unter Rückfluß erhitzt. Nach dem Abkühlen wird die Lösung mit Natriumhydrogencarbonat neutralisiert, in Methylenchlorid aufgenommen und mit Wasser gewaschen. Nach Trocknen über Natriumsulfat, Filtrieren und Eindampfen wird der feste Rückstand aus Methylenchlorid/Diethylether umkristallisiert.
Ausbeute: 0,78 g (86 % d. Th.) rotbraunes Pulver

| Analyse (bezogen auf wasserfreie Substanz): | | | | |
|---|---|---|---|---|
| C 68,90<br>C 68,81 | H 6,75<br>H 6,71 | N 8,94<br>N 8,85 | O 15,32 | ber. |

c) N,N'-Bis-(2-aminoethyl)-3,8-bis-(methoxymethyl)-deuteroporphyrin-IX-13,17-diamid

0,70 g (1,12 mmol) 3,8-Bis-(methoxymethyl)-deuteroporphyrin-IX-dimethylester (Beispiel 10b) werden im Autoklaven in einem Gemisch aus 80 ml Pyridin und 20 ml 1,2-Diaminoethan nach überschichten mit Stickstoff 24 Stunden auf 150°C erhitzt. Man dampft den Ansatz bis zur Trockne ein und reinigt das feste Rohprodukt durch Umkristallisieren aus Pyridin/Diethylether.
Ausbeute: 0,63 g (83 % d. Th.) rotbraunes Pulver

| Analyse (bezogen auf wasserfreie Substanz): | | | | |
|---|---|---|---|---|
| C 66,84<br>C 66,68 | H 7,38<br>H 7,11 | N 16,41<br>N 16,42 | O 9,37 | ber. |

d) N,N'-Bis-[13-carboxy-4-oxo-6,9-bis-(carboxymethyl)-12-(ethoxycarbonylmethyl)-3,6,9,12-tetraazatridecyl]-3,8-bis-(methoxymethyl)-deuteroporphyrin-IX-13,17-diamid

0,71 g (1,76 mmol) 3-Ethoxy-carbonylmethyl-6-[2-(2,6-dioxomorpholino)-ethyl]-3,6-diazaoctandisäure (DTPA-Monoanhydridmonoethylester) werden in 100 ml wasserfreiem Dimethylformamid suspendiert. Man überschichtet mit Stickstoff, setzt 0,89 g (8,80 mmol) Triethylamin und 0,60 g (0,88 mmol) N,N'-Bis-(2-aminoethyl)-3,8-bis-(methoxymethyl)-deuteroporphyrin-IX-13,17-diamid (Beispiel 10c) zu und rührt 3 Tage bei Raumtemperatur. Nach Filtrieren und Eindampfen im Vakuum wird der Rückstand über Kieselgel RP-18 mit Wasser/Tetrahydrofuran chromatographiert und im Vakuum zur Trockne eingeengt.
Ausbeute: 1,09 g (83 % d. Th.) rotbraunes Pulver

| Analyse (bezogen auf wasserfreie Substanz): | | | | |
|---|---|---|---|---|
| C 56,44<br>C 56,18 | H 6,77<br>H 6,63 | N 13,16<br>N 13,27 | O 23,63 | ber. |

e) N,N'-Bis-[13-carboxylato-4-oxo-6,9-bis-(carboxylatomethyl)-12-(ethoxycarbonylmethyl)-3,6,9,12-tetraazatridecyl]-3,8-bis-(methoxymethyl)-deuteroporphyrin-IX-13,17-diamid, Digadoliniumkomplex

1,00 g (0,67 mmol) des unter Beispiel 10d) hergestellten Liganden werden in 250 ml Wasser gelöst. Durch Zugabe von zweinormaler wäßriger Natronlauge wird pH 7,0 eingestellt, portionsweise 0,60 g (1,47 mmol) Gadoliniumacetat-tetrahydrat und zweinormale wäßrige Natronlauge zugesetzt, so daß der pH-Wert des Reaktionsgemisches stets zwischen 6,8 und 7,2 pendelt. Ist alles Gadoliniumacetat zugesetzt, wird über Nacht bei Raumtemperatur nachgerührt. Zur Aufarbeitung wird filtriert, das Lösungsmittel im Vakuum abgezogen, der Rückstand über Kieselgel RP-18 mit Wasser/Tetrahydrofuran chromatographiert und im Vakuum zur Trockne eingeengt.
Ausbeute: 0,87 g (72 % d. Th.) rotbraunes Pulver

| Analyse (bezogen auf wasserfreie Substanz): | | | | | |
|---|---|---|---|---|---|
| C 46,76<br>C 46,54 | H 5,27<br>H 5,23 | Gd 17,49<br>Gd 17,42 | N 10,91<br>N 10,75 | O 19,58 | ber. |

f) N,N'-Bis-[13-carboxylato-4-oxo-6,9,12-tris-(carboxylatomethyl)-3,6,9,12-(tetraazatridecyl)-3,8-bis-(methoxymethyl)-deuteroporphyrin-IX-13,17-diamid, Digadoliniumkomplex, Dinatriumsalz

1,00 g (0,67 mmol) des unter Beispiel 10d hergestellten Liganden werden in 200 ml Wasser gelöst. Durch Zugabe von 10normaler wäßriger Natronlauge wird pH 13 eingestellt und 5 Stunden bei Raumtemperatur gerührt. Nach vollständiger Verseifung des Esters wird mit konzentrierter Salzsäure pH 7 eingestellt und wie unter Beispiel 10e beschrieben, mit 0,60 g (1,47 mmol) Gadoliniumacetat-tetrahydrat komplexiert, aufgearbeitet und gereinigt.
Ausbeute: 1,01 g (84 % d. Th.)

| Analyse (bezogen auf wasserfreie Substanz): | | | | | | |
|---|---|---|---|---|---|---|
| C 44,39<br>C 44,17 | H 4,74<br>H 4,68 | N 10.98<br>N 10,89 | Gd 17,61<br>Gd 17,65 | Na 2,57<br>Na 2,63 | O 19,71 | ber |

**Beispiel 11**

a) N-(2-Aminoethyl)-3,8-bis-(methoxymethyl)-deuteroporphyrin-IX-monoamidmonomethylester (Isomerengemisch)

0,70 g (1,12 mmol) 3,8-Bis-(methoxymethyl)-deuteroporphyrin-IX-dimethylester (Beispiel 10 b) werden im Autoklaven in einem Gemisch aus 90 ml Pyridin und 10 ml 1,2-Diaminoethan nach überschichten mit Stickstoff 12

Stunden lang auf 150°C erhitzt. Man dampft den Ansatz ein und reinigt das feste Rohprodukt durch Umkristallisation aus Pyridin/Diethylether.
Ausbeute: 0,56 g (86 % d. Th.) rotbraunes Pulver

b) N-(2-Aminoethyl)-3,8-bis-(methoxymethyl)-deuteroporphyrin-IX-monoamidmononatriumsalz (Isomerengemisch)

0,50 g (0,75 mmol) der unter Beispiel 11a hergestellten Verbindung werden in einem Gemisch aus 100 ml Pyridin und 100 ml einnormaler Natronlauge zwei Stunden bei 50°C gerührt; dann wird im Vakuum eingedampft. Der Rückstand wird in 100 ml Wasser suspendiert, die Suspension mit 0,1 normaler Salzsäure neutralisiert, der Niederschlag abgesaugt, mit Wasser gewaschen und im Vakuum über Phosphorpentoxid getrocknet.
Ausbeute: 0,41 g (81 % d. Th.) rotbraunes Pulver

c) N-[13-carboxy-4-oxo-6,9-bis-(carboxymethyl)-12-(ethoxycarbonylmethyl)-3,6,9,12-tetraazatridecyl]-3,8-bis-(methoxymethyl)-deuteroporphyrin-IX-monoamid-Natriumsalz (Isomerengemisch)

0,24 g (0,60 mmol) 3-Ethoxy-carbonylmethyl-6-[2-(2,6-dioxomorpholino)-ethyl]-3,6-diazaoctandisäure (DTPA-Monoanhydridmonoethylester) werden in 80 ml wasserfreiem Dimethylformamid suspendiert. Man überschichtet mit Stickstoff, setzt 0,30 g (3,00 mmol) Triethylamin und 0,40 g (0,60 mmol) der in Beispiel 11b hergestellten Verbindung zu und rührt 3 Tage bei Raumtemperatur. Nach Filtrieren und Eindampfen im Vakuum wird der Rückstand in wenig Wasser suspendiert, durch Zugabe von zweinormaler Natronlauge bei pH 7,2 gelöst, über Kieselgel RP-18 mit Wasser/Tetrahydrofuran chromatographiert und das Eluat im Vakuum zur Trockne eingeengt.
Ausbeute: 0,37 g (58 % d. Th.) rotbraunes Pulver

| Analyse (bezogen auf wasserfreie Substanz): | | | | | |
|---|---|---|---|---|---|
| C 58,58<br>C 58,49 | H 6,43<br>H 6,40 | N 11,82<br>N 11,58 | Na 2,16<br>Na 2,35 | O 21,01 | ber. |

d) N-[13-carboxylato-4-oxo-6,9-bis-(carboxylatomethyl)-12-(ethoxycarbonylmethyl)-3,6,9,12-tetraazatridecyl)-3,8-bis-(methoxymethyl)-deuteroporphyrin-IX-monoamid, Gadoliniumkomplex, Natriumsalz (Isomerengemisch)

0,35 g (0,33 mmol) des unter Beispiel 11c hergestellten Liganden werden in 100 ml Wasser gelöst. Durch Zugabe von zweinormaler wäßriger Natronlauge wird pH 7,0 eingestellt, portionsweise 0,15 g (0,36 mmol) Gadoliniumacetat-tetrahydrat und zweinormale wäßrige Natronlauge zugesetzt, so daß der pH-Wert des Reaktionsgemisches stets zwischen 6,8 und 7,2 pendelt. Nachdem alles Gadoliniumacetat zugesetzt ist, wird über Nacht bei Raumtemperatur nachgerührt. Zur Aufarbeitung wird filtriert, das Lösungsmittel im Vakuum abgezogen, der Rückstand über Kieselgel RP-18 mit Wasser/Tetrahydrofuran chromatographiert und das Eluat im Vakuum zur Trockne eingeengt.
Ausbeute: 0,33 g (81 % d. Th.) rotbraunes Pulver

| Analyse (bezogen auf wasserfreie Substanz): | | | | | | |
|---|---|---|---|---|---|---|
| C 51,18<br>C 50,88 | H 5,37<br>H 5,35 | N 10,33<br>N 10,24 | Na 1,88<br>Na 1,91 | Gd 12,89<br>Gd 12,90 | O 18,35 | ber. |

e) N-[13-carboxylato-4-oxo-6,9,12-tris-(carboxylatomethyl)-3,6,9,12-tetraazatridecyl]-3,8-bis-(methoxymethyl)-deuteroporphyrin-IX-monoamid, Gadoliniumkomplex, Dinatriumsalz (Isomerengemisch)

0,35 g (0,33 mmol) des unter Beispiel 11 chergestellten Liganden werden in 100 ml Wasser gelöst. Durch Zugabe von 10molarer wäßriger Natronlauge wird pH 13 eingestellt und fünf Stunden bei Raumtemperatur gerührt. Nach vollständiger Verseifung des Esters wird mit konzentrierter Salzsäure pH 7 eingestellt und wie unter Beispiel 11d beschrieben, mit 0,15 g (0,36 mmol) Gadoliniumacetat-tetrahydrat komplexiert, aufgearbeitet und gereinigt.
Ausbeute: 0,33 g (83 % d. Th.) rotbraunes Pulver

| Analyse (bezogen auf wasserfreie Substanz): | | | | | | |
|---|---|---|---|---|---|---|
| C 49,46<br>C 49,35 | H 4,98<br>H 5,06 | N 10,38<br>N 10,27 | Na 3,79<br>Na 3,85 | Gd 12,95<br>Gd 12,89 | O 18,45 | ber. |

**Beispiel 12**

a) Mangan(III)-[N,N'-Bis-(2-aminoethyl)-3,8-bis-(methoxymethyl)-deuteroporphyrin-IX-13,17-diamid]-acetat

0,60 g (0,88 mmol) der in Beispiel 10c hergestellten Verbindung werden mit 3,00 g Mangan(II)-acetat eine Stunde in 120 ml Essigsäure unter Rückfluß gekocht. Dann wird im Vakuum eingedampft, der Rückstand in Wasser aufgeschlämmt, abfiltriert und mit Wasser gewaschen. Das getrocknete Rohprodukt wird aus Pyridin/Diethylether umkristallisiert.
Ausbeute: 0,64 g (91 % d. Th.) rotbraunes Pulver

| Analyse (bezogen auf wasserfreie Substanz): | | | | | |
|---|---|---|---|---|---|
| C 60,45<br>C 60,18 | H 6,47<br>H 6,51 | Mn 6,91<br>Mn 6,90 | N 14,10<br>N 13,97 | O 12,08 | ber. |

b) Mangan(III)-{N,N'-Bis-[13-carboxy-4-oxo-5,9-bis-(carboxymethyl)-12-(ethoxycarbonylmethyl)-3,6,9,12-tetraazatridecyl]-3,8-bis-(methoxymethyl)-deuteroporphyrin-IX-13,17-diamid}-acetat

0,61 g (1,50 mmol) 3-Ethoxy-carbonylmethyl-6-[2-(2,6-dioxomorpholino)-ethyl]-3,6-diazaoctandisäure (DTPA-Monoanhydridmonomethylester) werden in 100 ml wasserfreiem Dimethylformamid suspendiert. Man überschichtet mit Stickstoff, setzt 0,76 g (7,50 mmol) Triethylamin und 0,60 g (0,75 mmol) der in Beispiel 12a hergestellten Verbindung zu und rührt 3 Tage bei Raumtemperatur. Nach Filtrieren und Eindampfen im Vakuum wird der Rückstand über Kieselgel RP-18 mit Wasser/Tetrahydrofuran chromatographiert und das Eluat im Vakuum zur Trockne eingeengt.
Ausbeute: 1,01 g (84 % d. Th.) rotbraunes Pulver

| Analyse (bezogen auf wasserfreie Substanz): | | | | | |
|---|---|---|---|---|---|
| C 54,00<br>C 53,79 | H 6,36<br>H 6,31 | Mn 3,43<br>Mn 3,11 | N 12,24<br>N 12,03 | O 23,98 | ber. |

c) Mangan(III)-{N,N'-Bis-[13-carboxylato-4-oxo-6,9-bis-(carboxylatomethyl)-12-(ethoxycarbonylmethyl)-3,6,9,12-tetraazatridecyl]-3,8-bis-(methoxymethyl)-deuteroporphyrin-IX-13,17-diamid}-acetat, Digadoliniumkomplex

1,00 g (0,62 mmol) des unter Beispiel 12b hergestellten Liganden werden in 250 ml Wasser gelöst. Durch Zugabe von zweinormaler Natronlauge wird pH 7,0 eingestellt, portionsweise 0,55 g (1,36 mmol) Gadoliniumacetat und zweinormale Natronlauge zugesetzt, so daß der pH-Wert des Reaktionsgemisches stets zwischen 6,8 und 7,2 pendelt. Ist alles Gadoliniumacetat zugesetzt, wird über Nacht bei Raumtemperatur nachgerührt. Zur Aufarbeitung wird filtriert, das Lösungsmittel im Vakuum abgezogen, der Rückstand über Kieselgel RP-18 mit Wasser/Tetrahydrofuran chromatographiert und das Eluat im Vakuum zur Trockne eingeengt.
Ausbeute: 0,85 g (72 % d. Th.) rotbraunes Pulver

| Analyse (bezogen auf wasserfreie Substanz): | | | | | | |
|---|---|---|---|---|---|---|
| C 45,28<br>C 45,07 | H 5,01<br>H 4,88 | N 10,27<br>N 10,16 | Gd 16,47<br>Gd 16,51 | Mn 2,88<br>Mn 2,80 | O 20,10 | ber. |

d) Mangan(III)-{N,N'-Bis-[13-carboxylato-4-oxo-6,9,12-tris-(carboxylatomethyl)-3,6,9,12-tetraazatridecyl]-3,8-bis-(methoxymethyl)-deuteroporphyrin-IX-13,17-diamid}-acetat, Digadoliniumkomplex, Dinatriumsalz

1,00 g (0,52 mmol) des unter Beispiel 12b hergestellten Liganden werden in 250 ml Wasser gelöst. Durch Zugabe von 10molarer wäßriger Natronlauge wird pH 13 eingestellt und 5 Stunden bei Raumtemperatur gerührt. Nach vollständiger Verseifung des Esters wird mit konzentrierter Salzsäure pH 7,0 eingestellt und wie unter Beispiel 12c beschrieben, mit 0,47 (1,15 mmol) Gadoliniumacetat-tetrahydrat komplexiert und gereinigt.
Ausbeute: 80 g (81 % d. Th.)

| Analyse (bezogen auf wasserfreie Substanz): | | | | | | |
|---|---|---|---|---|---|---|
| C 43,03 | H 4,51 | N 10,33 | Na 2,42 | Gd 16,57 | Mn 2,89 O 20,23 | ber. |

(fortgesetzt)

| Analyse (bezogen auf wasserfreie Substanz): | | | | | | |
|---|---|---|---|---|---|---|
| C 42,88 | H 4,36 | N 10,30 | Na 2,24 | Gd 16,49 | Mn 2,82 | |

**Beispiel 13**

a) 3,8-Bis-(methoxymethyl)-deuteroporphyrin-IX-13,17-dihydrazid

1,00 g (1,60 mmol) der unter Beispiel 10b hergestellten Verbindung werden in 80 ml wasserfreiem Pyridin unter Argon gelöst und mit 15 ml Hydrazin versetzt. Nach 3 Tagen Rühren bei Raumtemperatur wird im Vakuum eingedampft, der Rückstand in halbkonzentrierter wäßriger Salzsäure eingerührt und mit 6 normaler Natronlauge zum Einstellen von pH 7,0 versetzt.. Der Niederschlag wird abfiltriert, mit Wasser gewaschen und aus Pyridin/Ether umkristallisiert.
Ausbeute: 0,81 g (81 % d. Th.) rotbraunes Pulver

| Analyse (bezogen auf wasserfreie Substanz): | | | | |
|---|---|---|---|---|
| C 65,16<br>C 64,87 | H 6,75<br>H 6,62 | N 17,88<br>N 17,64 | O 10,21 | ber. |

b) N,N'-Bis-[11-carboxy-2-oxo-4,7-bis-(carboxymethyl)-10-(ethoxycarbonylmethyl)-1,4,7,10-tetraazaundecyl]-3,8-bis-(methoxymethyl)-deuteroporphyrin-IX-13,17-diamid

0,90 g (2,23 mmol) 3-Ethoxy-carbonylmethyl-6-[2,6-dioxomorpholino)-ethyl]-3,6-diazaoctandisäure (DTPA-Monoanhydridmcnoethylester) werden in 250 ml wasserfreiem Dimethylformamid suspendiert. Man überschichtet mit Stickstoff, setzt 0,9 g (11,17 mmol) Triethylamin und 0,70 g (1,12 mmol) der unter Beispiel 13a hergestellten Verbindung zu und rührt 3 Tage bei Raumtemperatur. Nach Filtrieren und Eindampfen im Vakuum wird der Rückstand über Kieselgel RP-18 mit Wasser/Tetrahydrofuran chromatographiert und das Eluat zur Trockne eingeengt.
Ausbeute: 1,35 g (84 % d. Th.) rotbraunes Pulver

| Analyse (bezogen auf wasserfreie Substanz): | | | | |
|---|---|---|---|---|
| C 55,30<br>C 55,01 | H 6,47<br>H 6,42 | N 13,68<br>N 13,36 | O 24,55 | ber. |

c) N,N'-Bis-[11-carboxylato-2-oxo-4,7-bis-(carboxylatomethyl)-10-(ethoxycarbonylmethyl)-1,4,7,10-tetraazaundecyl]-3,8-bis-(methoxymethyl)-deuteroporphyrin-IX-13,17-diamid, Digadoliniumkomplex

1,30 g (0,91 mmol) des unter Beispiel 13b hergestellten Liganden werden in 250 ml Wasser gelöst. Durch Zugabe von zweimolarer wäßriger Natronlauge wird pH 7,0 eingestellt, portionsweise 0,80 g (2,00 mmol). Gadoliniumacetat-tetrahydrat und zweinormale wäßrige Natronlauge zugesetzt, so daß der pH-Wert des Reaktionsgemisches stets zwischen 6,8 und 7,2 pendelt. Ist alles Gadoliniumacetat zugesetzt, wird über Nacht bei Raumtemperatur nachgerührt. Zur Aufarbeitung wird filtriert, das Lösungsmittel im Vakuum abgezogen, der Rückstand über Kieselgel RP-18 mit Wasser/Tetrahydrofuran chromatographiert und das Eluat zur Trockne eingeengt.
Ausbeute: 1,27 g (80 % d. Th.) rotbraunes Pulver

| Analyse (bezogen auf wasserfreie Substanz): | | | | | |
|---|---|---|---|---|---|
| C 45,51<br>C 45,43 | H 4,98<br>H 4,86 | N 11,26<br>N 10,97 | Gd 18,05<br>Gd 17,87 | O 20,21 | ber. |

d) N,N'-Bis-[11-carboxylato-2-oxo-4,7,10-tris-(carboxylatomethyl)-1,4,7,10-tetraazaundecyl]-3,8-bis-(methoxymethyl)-deuteroporphyrin-IX-13,17-diamid Digadoliniumkomplex, Dinatriumsalz

1,00 g (0,70 mmol) des unter Beispiel 13b hergestellten Liganden werden in 200 ml Wasser gelöst. Durch Zugabe von 10molarer wäßriger Natronlauge wird pH 13 eingestellt und 5 Stunden bei Raumtemperatur gerührt. Nach vollständiger Verseifung des Esters wird mit konzentrierter Salzsäure pH 7,0 eingestellt und wie unter Beispiel 13c

beschrieben, mit 0,62 g (1,53 mmol) Gadoliniumacetat-tetrahydrat komplexiert, aufgearbeitet und gereinigt.
Ausbeute: 0,86 g (71 % d. Th.) rotbraunes Pulver

| Analyse (bezogen auf wasserfreie Substanz): | | | | | | |
|---|---|---|---|---|---|---|
| C 43,05<br>C 42,88 | H 4,42<br>H 4,31 | N 11,34<br>N 11,12 | Na 2,65<br>Na 2,71 | Gd 18,18<br>Gd 18,09 | O 20,35 | ber. |

**Beispiel 14**

a) Zn-[3,8-Bis-(1-propenyl)-deuteroporphyrin-IX-dimethylester]

2,48 g (6,69 mmol) Ethyltriphenylphosphoniumbromid in 600 ml wasserfreiem Tetrahydrofuran werden unter Argon bei Raumtemperatur mit einer Lösung von 0,43 g (6,69 mmol) nButyllithium versetzt. Nach vollständiger Umsetzung gibt man 2,00 g (3,04 mmol) Zn-[3,8-Diformyl-deuteroporphyrin-IX-dimethylester] (Kevin M. Smith, Eugene M. Fujinari, Kevin C. Langry, Daniel W. Parish and Hani D. Tabba, J. Am. Chem. Soc. 105, 6638-6646 (1983) zu und rührt zwei Stunden lang nach. Nach Zugabe von 30 ml Methanol wird das Lösungsmittel im Vakuum weitgehend abgezogen und der Rückstand mit Methylenchlorid und halbkonzentrierter, wäßriger Natriumhydrogencarbonatlösung ausgeschüttelt. Die organische Phase wird über Natriumsulfat getrocknet, eingedampft und an Aluminiumoxid (Merck, Aktivitätsstufe 2-3) mit Methylenchlorid/Methanol chromatographiert. Das Eluat wird im Vakuum zur Trockne eingeengt.
Ausbeute: 1,78 g (86 % d. Th.) rotbraunes Pulver

| Analyse (bezogen auf wasserfreie Substanz): | | | | | |
|---|---|---|---|---|---|
| C 66,91<br>C 66,62 | H 5,91<br>H 5,79 | N 8,21<br>N 8,07 | Zn 9,58<br>Zn 9,55 | O 9,38 | ber. |

b) 3,8-Bis-(n-propyl)-deuteroporphyrin-IX-dimethylether

1,70 g (2,49 mmol) des unter Beispiel 14a hergestellten Verbindung werden unter den zur Darstellung von Mesoporphyrin-IX-dimethylester aus Protoporphyrin-IXdimethylester angewandten Bedingungen (H. Muir u. A. Neuberger, Biochem. J., 45, 163 (1949)) unter gleichzeitiger Entmetallierung katalytisch hydriert bis das UV-Spektrum dem Etio-Typ entspricht und entsprechend dieser Literaturvorschrift aufgearbeitet und umkristallisiert.
Ausbeute: 1,42 g (92 % d. Th.) rotbraunes Pulver

| Analyse (bezogen auf wasserfreie Substanz): | | | | |
|---|---|---|---|---|
| C 73,28<br>C 72,97 | H 7,44<br>H 7,38 | N 9,00<br>N 8,85 | O 10,28 | ber. |

c) 3,8-Bis-(n-propyl)-deuteroporphyrin-IX-13,17-dihydrazid

1,40 g (2,25 mmol) der unter Beispiel 14b hergestellten Verbindung werden in 110 ml wasserfreien Pyridin unter Argon gelöst und mit 20 ml Hydrazin versetzt. Nach 3 Tagen Rühren bei 20°C wird im Vakuum eingedampft, der Rückstand in halbkonzentrierte wässrige Salzsäure eingerührt und mit 6normaler Natronlauge durch Einstellen von pH 7,0 wieder gefällt. Der Niederschlag wird abfiltriert, mit Wasser gewaschen und aus Pyridin/Ether umkristallisiert.
Ausbeute: 1,25 g (89 % d. Th.) rotbraunes Pulver

| Analyse (bezogen auf wasserfreie Substanz): | | | | |
|---|---|---|---|---|
| C 69,43<br>C 69,19 | H 7,44<br>H 7,37 | N 17,99<br>N 17,65 | O 5,38 | ber. |

d) N,N'-Bis-[11-carboxy-2-oxo-4,7-bis-(carboxymethyl)-10-(ethoxycarbonylmethyl)-1,4,7,10-tetraazaundecyl] - 3,8-bis-(n-propyl)-deuteroporphyrin-IX-13,17-diamid

1,56 g (3,86 mmol) 3-Ethoxy-carbonylmethyl-6-[2-(2,6-dioxomorpholino)-ethyl]-3,6-diazaoctandisäure (DTPA-Monoanhydridmonoethylester) werden in 250 ml wasserfreiem Dimethylformamid suspendiert. Man überschichtet

mit Stickstoff, setzt 1,95 g (19,30 mmol) Triethylamin und 1,20 g (1,93 mmol) der unter Beispiel 14c hergestellten Verbindung zu und rührt 3 Tage bei Raumtemperatur. Nach Filtrieren und Eindampfen im Vakuum wird der Rückstand über Kieselgel RP-18 mit Wasser/Tetrahydrofuran chromatographiert und das Eluat im Vakuum zur Trockne eingeengt.
Ausbeute: 4,75 g (86 % d. Th.) rotbraunes Pulver

| Analyse (bezogen auf wasserfreie Substanz): | | | | |
|---|---|---|---|---|
| C 57,13<br>C 56,86 | H 6,77<br>H 6,69 | N 13,72<br>N 13,65 | O 22,38 | ber. |

e) N,N'-Bis-[11-carboxylato-2-oxo-4,7-bis-(carboxylatomethyl)-10-(ethoxycarbonylmethyl)-1,4,7,10-tetraazaundecyl]-3,8-bis-(n-propyl)-deuteroporphyrin-IX-13,17-diamid, Digadoliniumkomplex

4,70 g (3,29 mmol) des unter Beispiel 14d hergestellten Liganden werden in 1000 ml Wasser gelöst. Durch Zugabe von zweinormaler wäßriger Natronlauge wird pH 7,0 eingestellt, portionsweise 2,94 g (7,24 mmol) Gadoliniumacetat-tetrahydat und zweinormale wäßrige Natronlauge zugesetzt, so daß der pH-Wert des Reaktionsgemisches stets zwischen 6,8 und 7,2 pendelt. Ist alles Gadoliniumacetat zugesetzt, wird über Nacht bei Raumtemperatur nachgerührt. Zur Aufarbeitung wird filtriert, das Lösungsmittel im Vakuum abgezogen und der Rückstand über Kieselgel RP-18 mit Wasser/Tetrahydrofuran chromatographiert. Das Eluat wird im Vakuum zur Trockne eingeengt.
Ausbeute: 4,35 g (76 % d. Th.) rotbraunes Pulver

| Analyse (bezogen auf wasserfreie Substanz): | | | | | |
|---|---|---|---|---|---|
| C 46,99<br>C 46,75 | H 5,22<br>H 5,16 | N 11,28<br>N 11,02 | Gd 18,09<br>Gd 18,02 | O 18,41 | ber. |

f) N,N'-Bis-[11-carboxylato-2-oxo-4,7,10-tris-(carboxylatomethyl)-1,4,7,10-tetraazaundecyl]-3,8-bis-X-(1-propyl)-deuteroporphyrin-IX-13,17-diamid, Digadoliniumkomplex, Dinatriumsalz

2,00 g (1,40 mmol) des unter Beispiel 14d hergestellten Liganden werden in 200 ml Wasser gelöst. Durch Zugabe von 10 molarer wäßriger Natronlauge wird pH 13 eingestellt und 5 Stunden bei Raumtemperatur gerührt. Nach vollständiger Verseifung des Esters wird mit konzentrierter Salzsäure pH 7,0 eingestellt und wie unter Beispiel 14e beschrieben, mit 1,36 g (3,08 mmol) Gadoliniumacetat-tetrahydrat komplexiert und gereinigt.
Ausbeute: 1,80 g (75 % d. Th.) rotbraunes Pulver

| Analyse (bezogen auf wasserfreie Substanz): | | | | | |
|---|---|---|---|---|---|
| C 44,54<br>C 44,26 | H 4,67<br>H 4,57 | N 11,36<br>N 11,13 | Gd 18,22<br>Gd 18,13 | O 18,54 | ber. |

**Beispiel 15**

a) Zink(II)-[3,8-Bis-(1-butenyl)-deuteroporphyrin-IX-dimethylester]

2,58 g (6,69 mmol) Propyltriphenylphosphoniumbromid in 600 ml wasserfreiem Tetrahydrofuran werden unter Argon bei Raumtemperatur mit einer Lösung von 0,43 g (6,69 mmol) Butylithium versetzt. Nach vollständiger Umsetzung zum Ylid gibt man 2,00 g (3,04 mmol) Zn-[3,8-Diformyl-deuteroporphyrin-IX-dimethylester] (Kevin M. Smith, Eugene M. Fujinari, Kevin C. Langry, Daniel W. Parish and Hani D. Tabba, J. Am. Chem. Soc. 105, 6638-6646 (1983)) und rührt zwei Stunden lang nach. Nach Zugabe von 30 ml Methanol wird das Lösungsmittel im Vakuum weitgehend abgezogen und der Rückstand mit Methylenchlorid und halbkonzentrierter wäßriger Natriumhydrogencarbonatlösung ausgeschüttelt. Die organische Phase wird über Natriumsulfat getrocknet, eingedampft und an Aluminiumoxid (Merck, Aktivitätsstufe 2-3) mit Methylenchlorid/Methanol chromatographiert. Das Eluat wird im Vakuum zur Trockne eingeengt.
Ausbeute: 1,75 g (81 % d. Th.) rotbraunes Pulver

| Analyse (bezogen auf wasserfreie Substanz): | | | | | |
|---|---|---|---|---|---|
| C 67,65<br>C 67,41 | H 62,44<br>H 62,27 | N 7,89<br>N 7,63 | Zn 9,20<br>Zn 9,15 | O 9,01 | ber. |

b) 3,8-Bis-(n-butyl)-deuteroporphyrin-IX-dimethylester

1,6 g (2,25 mmol) der unter Beispiel 15a hergestellten Verbindung werden unter den zur Darstellung von Mesoporphyrin-IX-dimethylester aus Protoporphyrin-IXdimethylester angewandten Bedingungen (H. Muir u. A. Neuberger, Biochem. J., 45, 163 (1949)) unter gleichzeitiger Entmetallierung katalytisch hydriert bis das UV-Spektrum dem Etio-Typ entspricht und entsprechend dieser Literaturvorschrift aufgearbeitet und umkristallisiert.
Ausbeute: 1,39 g (95 % d. Th.) rotbraunes Pulver

| Analyse (bezogen auf wasserfreie Substanz): | | | | |
|---|---|---|---|---|
| C 73,82<br>C 73,55 | H 7,74<br>H 7,62 | N 8,61<br>N 8,48 | O 9,83 | ber. |

c) 3,8-Bis-(n-butyl)-deuteroporphyrin-IX-13,17-dihydrazid

1,30 g (2,00 mmol) der unter Beispiel 15b hergestellten Verbindung werden in 100 ml wasserfreien Pyridin unter Argon gelöst und mit 20 ml Hydrazin versetzt. Nach 3 Tagen Rühren bei 20°C wird im Vakuum eingedampft, der Rückstand in halbkonzentrierte wäßrige Salzsäure eingerührt und mit 6normaler Natronlauge durch Einstellen von pH 7,0 wieder gefällt. Der Niederschlag wird abfiltriert, mit Wasser gewaschen und aus Pyridin/Ether umkristallisiert.
Ausbeute: 1,18 g (91 % d. Th.) rotbraunes Pulver

| Analyse (bezogen auf wasserfreie Substanz): | | | | |
|---|---|---|---|---|
| C 70,13<br>C 69,85 | H 7,74<br>H 7,68 | N 17,22<br>M 17,01 | O 4,92 | ber. |

d) N,N'-Bis-[11-carboxy-2-oxo-4,7-bis-(carboxymethyl)-10-(ethoxycarbonylmethyl)-1,4,7,10-tetraazaundecyl]-3,8-bis-(n-butyl)-deuteroporphyrin-IX-13,17-diamid

1,24 g (3,07 mmol) 3-Ethoxy-carbonylmethyl-6-[2-(2,6-dioxomorpholino)-ethyl]-3,6-diazaoctandisäure (DTPA-Monoanhydridmonoethylester) werden in 200 ml wasserfreiem Dimethylformamid suspendiert. Man überschichtet mit Stickstoff, setzt 1,55 g (15,36 mmol) Triethylamin und 1,00 g (1,54 mmol) der unter Beispiel 15c hergestellten Verbindung zu und rührt 3 Tage bei Raumtemperatur. Nach Filtrieren und Eindampfen im Vakuum wird der Rückstand über Kieselgel RP-18 mit Wasser/Tetrahydrofuran chromatographiert. Das Eluat wird im Vakuum zur Trockne eingeengt.
Ausbeute: 2,00 g (89 % d. Th.) rotbraunes Pulver

| Analyse (bezogen auf wasserfreie Substanz): | | | | |
|---|---|---|---|---|
| C 57,68<br>C 57,38 | H 6,91<br>H 6,77 | N 13,45<br>N 13,19 | O 21,95 | ber. |

e) N,N'-Bis-[11-carboxylato-2-oxo-4,7-bis-(carboxylatomethyl)-10-(ethoxycarbonylmethyl)-1,4,7,10-tetraazaundecyl]-3,8-bis-(n-butyl)-deuteroporphyrin-IX-13,17-diamid, Digadoliniumkomplex

1,00 g (0,69 mmol) des unter Beispiel 15d hergestellten Liganden werden in 200 ml Wasser gelöst. Durch Zugabe von zweinormaler wäßriger Natronlauge wird pH 7,0 eingestellt, portionsweise 0,62 g (1,52 mmol) Gadoliniumacetat-tetrahydrat und zweinormale wäßrige Natronlauge zugesetzt, so daß der pH-Wert des Reaktionsgemisches stets zwischen 6,8 und 7,2 pendelt. Ist alles Gadoliniumacetat zugesetzt, wird über Nacht bei Raumtemperatur nachgerührt. Zur Aufarbeitung wird filtriert, das Lösungsmittel im Vakuum abgezogen und der Rückstand über Kieselgel RP-18 mit Wasser/Tetrahydrofuran chromatographiert und das Eluat im Vakuum zur Trockne eingeengt.
Ausbeute: 0,87 g (71 % d. Th.) rotbraunes Pulver

| Analyse (bezogen auf wasserfreie Substanz): | | | | | |
|---|---|---|---|---|---|
| C 47,61<br>C 47,65 | H 5,36<br>H 5,51 | N 11,10<br>N 11,23 | Gd 17,81<br>Gd 17,71 | O 18,12 | ber. |

f) N,N'-Bis-[11-carboxylato-2-oxo-4,7-tris-(carboxylatomethyl)-1,4,7,10-tetraazaundecyl]-3,8-bis-(n-butyl)-deuteroporphyrin-IX-13,17-diamid, Digadoliniumkomplex, Dinatriumsalz

1,00 g (0,69 mmol) des unter Beispiel 15d hergestellten Liganden werden in 200 ml Wasser gelöst. Durch Zugabe von 1Omolare wäßriger Natronlauge wird pH 13 eingestellt und 5 Stunden bei Raumtemperatur gerührt. Nach vollständiger Verseifung des Esters wird mit konzentrierter Salzsäure pH 7,0 eingestellt und wie unter Beispiel 15e beschrieben, mit 0,62 g (1,52 mmol) Gadoliniumacetat-tetrahydrat komplexiert und gereinigt.
Ausbeute: 0,88 g (73 % d. Th.) rotbraunes Pulver

| Analyse (bezogen auf wasserfreie Substanz): | | | | | | |
|---|---|---|---|---|---|---|
| C 45,20<br>C 44,93 | H 4,83<br>H 4,76 | N 11,18<br>N 11,01 | Na 2,61<br>Na 2,52 | Gd 17,93<br>Gd 17,82 | O 18,25 | ber. |

**Beispiel 16**

a) Zink(II)-[3,8-Bis-(1-heptenyl)-deuteroporphyrin-IX-dimethylester]

2,86 g (6,69 mmol) Hexyltriphenylphosphoniumbromid in 600 ml wasserfreiem Tetrahydrofuran werden unter Argon bei Raumtemperatur mit einer Lösung von 0,43 g (6,69 mmol) Butylithium versetzt. Nach vollständiger Umsetzung zum Ylid gibt man 2,00 g (3,04 mmol) Zn-[3,8-Diformyl-deuteroporphyrin-IX-dimethylester] (Kevin M. Smith, Eugene M. Fujinari, Kevin C. Langry, Daniel W. Parish and Hani D. Tabba, J. Am. Chem. Soc. 105, 6638-6646 (1983)) zu und rührt zwei Stunden lang nach. Nach Zugabe von 30 ml Methanol wird das Lösungsmittel im Vakuum weitgehend abgezogen und mit Methylenchlorid und halbkonzentrierter wäßriger Natriumhydrogencarbonatlösung ausgeschüttelt. Die organische Phase wird über Natriumsulfat getrocknet, eingedampft und an Aluminiumoxid (Merck, Aktivitätsstufe 2-3) mit Methylenchlorid/Methanol chromatographiert. Das Eluat wird im Vakuum zur Trockne eingeengt.
Ausbeute: 1,91 g (79 % d. Th.) rotbraunes Pulver

| Analyse (bezogen auf wasserfreie Substanz): | | | | | |
|---|---|---|---|---|---|
| C 69,56<br>C 69,11 | H 7,11<br>H 6,88 | N 7,05<br>N 6,79 | Zn 8,23<br>Zn 8,16 | O 8,06 | ber. |

b) 3,8-Bis-(n-heptyl)-deuteroporphyrin-IX-dimethylester

1,50 g (1,89 mmol) der unter Beispiel 16a hergestellten Verbindung werden unter den zur Darstellung von Mesoporphyrin-IX-dimethylester aus Protoporphyrin-IXdimethylester angewandten Bedingungen (H. Muir u. A. Neuberger, Biochem. J., 45, 163 (1949)) unter gleichzeitiger Entmetallierung katalytisch hydriert bis das UV-Spektrum dem Etio-Typ entspricht und entsprechend dieser Literaturvorschrift aufgearbeitet und umkristallisiert.
Ausbeute: 1,32 g (95 % d. Th.) rotbraunes Pulver

| Analyse (bezogen auf wasserfreie Substanz): | | | | |
|---|---|---|---|---|
| C 75,17<br>C 74,83 | H 8,50<br>H 8,39 | N 7,62<br>N 7,37 | O 8,71 | ber. |

c) 3,8-Bis-(I-heptyl)-deuteroporphyrin-IX-13,17-dihydrazid

1,30 g (1,77 mmol) der unter Beispiel 16b hergestellten Verbindung werden in 100 ml wasserfreien Pyridin unter Argon gelöst und mit 20 ml Hydrazin versetzt. Nach 3 Tagen Rühren bei 20°C wird im Vakuum eingedampft, der Rückstand in halbkonzentrierte wässrige Salzsäure eingerührt und mit 6normaler Natronlauge durch Einstellen von pH 7,0 wieder gefällt. Der Niederschlag wird abfiltriert, mit Wasser gewaschen und aus Pyridin/Ether umkristallisiert.
Ausbeute: 1,14 g (88 % d. Th.) rotbraunes Pulver

| Analyse (bezogen auf wasserfreie Substanz): | | | | |
|---|---|---|---|---|
| C 71,90<br>C 71,78 | H 8,50<br>H 8,39 | N 15,24<br>N 14,97 | O 4,35 | ber. |

d) N,N'-Bis-[11-carboxy-2-oxo-4,7-bis-(carboxymethyl)-10-(ethoxycarbonylmethyl)-1,4,7,10-tetraazaundecyl]-3,8-bis-(n-heptyl)-deuteroporphyrin-IX-13,17-diamid

1,21 g (3,00 mmol) 3-Ethoxy-carbonylmethyl-6-[2-(2,6-diaxomorpholino)-ethyl]-3,6-diazaoctandisäure (DTPA-Monoanhydridmonoethylester werden in 200 ml wasserfreiem Dimethylformamid suspendiert. Man überschichtet mit Stickstoff, setzt 1,52 g (15,00 mmol) Triethylamin und 1,10 g (1,50 mmol) der unter Beispiel 16 c hergestellt Verbindung zu und rührt 3 Tage bei Raumtemperatur. Nach Filtrieren und Eindampfen im Vakuum wird der Rückstand über Kieselgel RP-18 mit Wasser/Tetrahydroruran chromatographiert und das Eluat im Vakuum zur Trockne eingeengt.
Ausbeute: 2,06 g (89 % d. Th.) rotbraunes Pulver

| Analyse (bezogen auf wasserfreie Substanz): | | | | |
|---|---|---|---|---|
| C 59,21<br>C 58,97 | H 7,32<br>H 7,11 | N 12,72<br>N 12,61 | O 20,75 | ber. |

e) N,N'-Bis-[11-carboxylato-2-oxo-4,7-bis-(carboxylatomethyl)-10-ethoxycarbonylmethyl-1,4,7,10-tetraazaundecyl]-3,8-bis-(n-heptyl)-deuteroporphyrin-IX-13,17-diamid, Digadoliniumkomplex

1,00 g (0,65 mmol) des unter Beispiel 16d hergestellten Liganden werden in 300 ml Wasser gelöst. Durch Zugabe von zweinormaler wäßriger Natronlauge wird pH 7,0 eingestellt, portionsweise 0,58 g (1,43 mmol) Gadoliniumacetat-tetrahydrat und zweinormale wäßrige Natronlauge zugesetzt, so daß der pH-Wert des Reaktionsgemisches stets zwischen 6,8 und 7,2 pendelt. Ist alles Gadoliniumacetat zugesetzt, wird über Nacht bei Raumtemperatur nachgerührt. Zur Aufarbeitung wird filtriert, das Lösungsmittel im Vakuum abgezogen, der Rückstand über Kieselgel RP-18 mit Wasser/Tetrahydrofuran chromatographiert und das Eluat im Vakuum zur Trockne eingeengt.
Ausbeute: 0,87 g (72 % d. Th.) rotbraunes Pulver

| Analyse (bezogen auf wasserfreie Substanz): | | | | | |
|---|---|---|---|---|---|
| C 49,34<br>C 49,07 | H 5,77<br>H 5,68 | N 10,60<br>N 10 42 | Gd 17,00<br>Gd 16,89 | O 17,29 | ber. |

f) N,N'-Bis-[11-carboxylato-2-oxo-4,7,10-tris-(carboxylatomethyl)-1,4,7,10-tetraazaundecyl]-3,8-bis-(n-heptyl)-deuteroporphyrin-IX-13,17-diamid, Digadoliniumkomplex, Dinatriumsalz

1,00 g (0,65 mmol) des unter Beispiel 16d hergestellten Liganden werden in 300 ml Wasser gelöst. Durch Zugabe von 10molarer wäßriger Natronlauge wird pH 13 eingestellt und 5 Stunden bei Raumtemperatur gerührt. Nach vollständiger Verseifung des Esters wird mit konzentrierter Salzsäure pH 7,0 eingestellt und wie unter Beispiel 16e beschrieben, mit 0,58 g (1,43 mmol) Gadoliniumacetat-tetrahydrat komplexiert und gereinigt.
Ausbeute: 0,91 g (76 % d. Th.) rotbraunes Pulver

| Analyse (bezogen auf wasserfreie Substanz): | | | | | |
|---|---|---|---|---|---|
| C 47,05<br>C 46,86 | H 5,26<br>H 5,07 | N 10,67<br>N 10,42 | Na 2,49<br>Na 2,51 | Gd 17,11<br>Gd 17,09 | O 17,41ber. |

**Beispiel 17**

a) Zink(II)-[3,8-Bis-(2-phenylethenyl)-deuteroporphyrin-IX-dimethylester]

2,60 g (6,69 mmol) Benzyltriphenylphosphaniumchlorid in 600 ml wasserfreiem Tetrahydrofuran werden unter Argon bei Raumtemperatur mit einer Lösung von 0,43 g (6,69 mmol) Butyllithium versetzt. Nach vollständiger Umsetzung zum Ylid gibt man 2,00 g (3,04 mmol) Zn-[3,8-Diformyl-deuteroporphyrin-IX-dimethylester] (Kevin M. Smith, Eugene M. Fujinari, Kevin C. Langry, Daniel W. Parish and Hani D. Tabba, J. Am. Chem. Soc. 105, 6638-6646 (1983)) und rührt zwei Stunden lang nach. Nach Zugabe von 30 ml Methanol wird das Lösungsmittel im Vakuum weitgehend abgezogen und der Rückstand mit Methylenchlorid und halbkonzentrierter wäßriger Natriumhydrogencarbonatlösung ausgeschüttelt. Die organische Phase wird über Natriumsulfat getrocknet, eingedampft, über Aluminiumoxid (Merck, Aktivitätsstufe 2-3) mit Methylenchlorid/Methanol chromatographiert und das Eluat zur Trockne eingeengt.
Ausbeute: 2,23 g (91 % d. Th.) rotbraunes Pulver

| Analyse (bezogen auf wasserfreie Substanz): | | | | | |
|---|---|---|---|---|---|
| C 71,51<br>C 71,24 | H 5,50<br>H 5,39 | N 6,95<br>N 6,68 | Zn 8,11<br>Zn 8,06 | O 7,94 | ber. |

b) Zink(II)-[3,8-Bis-(2-phenylethyl)-deuteroporphyrin-IX-dihydrazid]

2,10 g (2,60 mmol) der unter Beispiel 17a hergestellten Verbindung werden in 130 ml Pyridin gelöst und mit 30 ml Hydrazin versetzt. Nach 3 Tagen Rühren im offenen Kolben bei 20°C wird im Vakuum eingedampft, der Rückstand in Wasser aufgeschlämmt, abgesaugt, im Vakuum getrocknet und aus Pyridin/Ether umkristallisiert.
Ausbeute: 1,96 g (93 % d. Th.) rotbraunes Pulver

| Analyse (bezogen auf wasserfreie Substanz): | | | | | |
|---|---|---|---|---|---|
| C 68,19<br>C 67,86 | H 5,97<br>H 5,83 | N 13,83<br>N 13,61 | Zn 8,07<br>Zn 7,94 | O 3,95 | ber. |

c) Mangan(III)-[3,8-Bis-(2-phenylethyl)-deuteroporphyrin-IX-dihydrazid]-acetat

1,90 g (2,34 mmol) der unter Beispiel 17b hergestellten Verbindung werden mit 9,50 g Mangan(II)acetat eine Stunde in 500 ml Essigsäure unter Rückfluß gekocht. Dann wird im Vakuum eingedampft, der Rückstand in Wasser aufgeschlämmt, abfiltriert und mit Wasser gewaschen. Das getrocknete Rohprodukt wird aus Dimethylformamid umkristallisiert.
Ausbeute: 1,65 g (82 % d. Th.) rotbraunes Pulver

| Analyse (bezogen auf wasserfreie Substanz): | | | | | |
|---|---|---|---|---|---|
| C 67,12<br>C 66,93 | H 5,98<br>H 5,91 | Mn 6,40<br>Mn 6,32 | N 13,05<br>N 12,89 | O 7,45 | ber. |

d) Mangan(III)-{N,N'-Bis-[11-carboxy-2-oxo-4,7-bis-(carboxymethyl)-10-(ethoxycarbonylmethyl)-1,4,7,10-tetraazaundecyl]-3,8-bis-(2-phenylethyl)-deuteroporphyrin-IX-13,17-diamid}-acetat

1,41 g (3,49 mmol) 3-Ethoxy-carbonylmethyl-6-[2-(2,6-dioxomorpholino)-ethyl]-3,6-diazaoctandisäure (DTPA-Monoanhydridmonoethylester) werden in 250 ml wasserfreiem Dimethylformamid suspendiert. Man überschichtet mit Stickstoff, setzt 1,77 g (17,50 mmol) Triethylamin und 1,50 g (1,75 mmol) der unter Beispiel 17c hergestellten Verbindung zu und rührt 3 Tage bei Raumtemperatur. Nach Filtrieren und Eindampfen im Vakuum wird der Rückstand über Kieselgel RP-18 mit Wasser/Tetrahydrofuran chromatographiert und das Eluat im Vakuum zur Trockne eingeengt.
Ausbeute: 2,10 g (72 % d. Th.) rotbraunes Pulver

| Analyse (bezogen auf wasserfreie Substanz): | | | | | |
|---|---|---|---|---|---|
| C 57,69<br>C 57,42 | H 6,11<br>H 5,99 | N 11,77<br>N 11,61 | Mn 3,30<br>Mn 3,24 | O 21,13 | ber. |

e) Mangan(III)-{N,N'-Bis-[11-carboxylato-2-oxo-4,7,10-tris-(carboxylatomethyl)-1,4,7,10-tetraazaundecyl]-3,8-bis-(2-phenylethyl)-deuteroporphyrin-IX-13,17-diamid}-acetat, Digadoliniumkomplex, Dinatriumsalz

2,00 g (1,20 mmol) des unter Beispiel 17d hergestellten Liganden werden in 400 ml Wasser suspendiert. Durch Zugabe von 10molarer wäßriger Natronlauge wird pH 13 eingestellt und 5 Stunden bei Raumtemperatur gerührt. Nach vollständiger Verseifung des Esters wird pH 7,0 eingestellt, 1,06 g (2,64 mmol) Gadoliniumacetat-tetrahydrat zugesetzt und durch Zugabe von 2normaler Natronlauge der pH-Wert auf 7,0 gehalten. Nach Rühren über Nacht wird filtriert, das Lösungsmittel im Vakuum abgezogen, der Rückstand über Kieselgel RP-18 mit Wasser/Tetrahydrofuran chromatographiert und das Eluat im Vakuum zur Trockne eingeengt.
Ausbeute: 1,62 g (69 % d. Th.) rotbraunes Pulver

| Analyse (bezogen auf wasserfreie Substanz): | | | | | | | |
|---|---|---|---|---|---|---|---|
| C 46,53 | H 4,37 | N 10,00 | Gd 16,03 | Mn 2,80 | Na 2,33 | O 17,94 | ber. |
| C 46,33 | H 4,28 | N 9,86 | Gd 17,86 | Mn 2,67 | Na 2,41 | | |

**Beispiel 18**

a) Zink(II)-[3,8-Bis-(2-(napht-1-yl)-ethenyl)-deuteroporphyrin-IX-dimethylester]

3,23 g (6,69 mmol) Napht-1-yl-methyl-triphenylphosphoniumbromid in 600 ml wasserfreiem Tetrahydrofuran werden unter Argon bei Raumtemperatur mit einer Lösung von 0,43 g (6,69 mmol) Butyllithium versetzt. Nach vollständiger Umsetzung zum Ylid gibt man 2,00 g (3,04 mmol) Zn-[3,8-Diformyldeuteroporphyrin-IX-dimethylester] (Kevin M. Smith, Eugene M. Fujinari, Kevin C. Langry, Daniel W. Parish and Hani D. Tabba, J. Am. Chem. Soc. 105, 6638-6646 (1983)) zu und rührt zwei Stunden lang nach. Nach Zugabe von 30 ml Methanol wird das Lösungsmittel im Vakuum weitgehend abgezogen und der Rückstand mit Methylenchlorid und halbkonzentrierter wäßriger Natriumhydrogencarbonatlösung ausgeschüttelt. Die organische Phase wird über Natriumsulfat getrocknet, eingedampft, an Aluminiumoxid (Merck, Aktivitätsstufe 2-3) mit Methylenchlorid/Methanol chromatographiert und das Eluat zur Trockne eingeengt.
Ausbeute: 2,53 g (92 %) rotbraunes Pulver

| Analyse (bezogen auf wasserfreie Substanz): | | | | | |
|---|---|---|---|---|---|
| C 74,21 | H 5,34 | N 6,18 | Zn 7,21 | O 7,06 | ber. |
| C 74,00 | H 5,19 | N 5,99 | Zn 7,11 | | |

b) Zink(II)-[3,8-Bis-(2-(napht-1-yl)-ethyl)-deuteroporphyrin-IX-dihydrazid]

2,50 g (2,76 mmol) der unter Beispiel 18a hergestellten Verbindung werden in 130 ml Pyridin gelöst und mit 30 ml Hydrazin versetzt. Nach 3 Tagen Rühren bei Raumtemperatur im offenen Kolben bei 20°C wird im Vakuum eingedampft, der Rückstand in Wasser aufgeschlämmt, abgesaugt, im Vakuum getrocknet und aus Pyridin/Ether umkristallisiert.
Ausbeute: 2,39 g (95 % d. Th.) rotbraunes Pulver

| Analyse (bezogen auf wasserfreie Substanz): | | | | | |
|---|---|---|---|---|---|
| C 71,24 | H 5,76 | N 12,31 | Zn 7,18 | O 3,51 | ber. |
| C 70,93 | H 5,59 | N 12,01 | Zn 7,09 | | |

c) Zink(II)-{N,N'-Bis[11-carboxy-2-oxo-4,7-bis-(carboxymethyl)-10-(ethoxycarbonylmethyl)-1,4,7,10-tetraazaundecyl]-3,8-bis-(2-(napht-1-yl)-ethyl)-deuteroporphyrin-IX-13,17-diamid}

1,77 g (4,40 mmol) 3-Ethoxy-carbonylmethyl-6-[2-(2,6-dioxomorpholino)-ethyl]-3,6-diazaoctandisäure (DTPA-Monoanhydridmonoethylester) werden in 300 ml wasserfreiem Dimethylformamid suspendiert. Man überschichtet mit Stickstoff, setzt 2,20 g (21,75 mmol) Triethylamin und 2,00 g (2,20 mmol) der unter Beispiel 18b hergestellten Verbindung zu und rührt 3 Tage bei Raumtemperatur. Nach Filtrieren und Eindampfen im Vakuum wird der Rückstand über Kieselgel RP-18 mit Wasser/Tetrahydrofuran chromatographiert. Das Eluat wird im Vakuum zur Trockne eingeengt.
Ausbeute: 2,91 g (77 % d. Th.) rotbraunes Pulver

| Analyse (bezogen auf wasserfreie Substanz): | | | | | |
|---|---|---|---|---|---|
| C 60,15 | H 5,99 | N 11,42 | Zn 3,81 | O 18,63 | ber. |
| C 59,89 | H 5,81 | N 11,26 | Zn 3,72 | | |

d) Zink(II)-{N,N'-Bis[11-carboxylato-2-oxo-4,7,10-tris-(carboxylatomethyl)-1,4,7,10-tetraazaundecyl]-3,8-bis-(2-(napht-1-yl)-ethyl)-deuteroporphyrin-IX-13,17-diamid}, Digadoliniumcomplex, Dinatriumsalz

2,00 g (1,16 mmol) des unter Beispiel 18c hergestellten Liganden werden in 400 ml Wasser suspendiert.

Durch Zugabe von 10molare wäßriger Natronlauge wird pH 13 eingestellt und 5 Stunden bei Raumtemperatur gerührt. Nach vollständiger Verseifung des Esters wird pH 7,0 eingestellt, 1,06 g (2,64 mmol) Gadoliniumacetat-tetrahydrat zugesetzt und durch Zugabe von 2normaler Natronlauge der pH-Wert auf 7,0 gehalten. Nach Rühren über Nacht wird filtriert, das Lösungsmittel im Vakuum abgezogen, der Rückstand über Kieselgel RP-18 mit Wasser/Tetrahydrofuran chromatographiert und das Eluat im Vakuum zur Trockne eingeengt.
Ausbeute: 1,47 g (63 % d. Th.) rotbraunes Pulver

| Analyse (bezogen auf wasserfreie Substanz): | | | | | | | |
|---|---|---|---|---|---|---|---|
| C 48,92 | H 4,31 | N 9,74 | Na 2,27 | Gd 15,62 | Zn 3,25 | O 15,89 | ber. |
| C 48,66 | H 4,21 | N 9,48 | Na 2,31 | Gd 15,46 | Zn 3,18 | | |

**Beispiel 19**

a) 13,17-Bis-(4-aminobutyl)-3,8-diethyl-2,7,12,18-tetramethylporphyrin

2,00 g (3,59 mmol) 13,17-Bis-(3-cyanopropyl)-3,8-diethyl-2,7,12,18-tetramethylprophyrin werden portionsweise zu dem aus 0,32 g (14,5 mmol) Lithiumborhydrid und 3,18 g (29,0 mmol) Trimethylsilylchlorid gebildetem Reduktionsmittel (A. Giannis, K. Sandhoff, Angewandte Chemie 101 Nr. 2, 220-222 (1989)) in 100 ml wasserfreiem Tetrahydrofuran gegeben und unter Argon 24 Stunden bei 25°C gerührt. Falls per Dünnschichtchromatographie noch Edukt nachweisbar ist, wird weiteres Reduktionsmittel zugesetzt und weitergerührt. Zur Aufarbeitung tropft man vorsichtig
20 ml Methanol zu, stellt mit halbkonzentrierter Salzsäure pH 1,0 ein und läßt eine Stunde rühren. Anschließend wird der Ansatz durch Zugabe von 20 ml 30 %iger Natronlauge stark alkalisch gestellt, 30 Minuten gerührt und zweimal mit Methylenchlorid ausgeschüttelt. Die organischen Phasen werden vereinigt, über wasserfreiem Natriumsulfat getrocknet, filtriert und eingedampft. Der rotbraune, feste Rückstand wird als Rohprodukt weiterverwendet.
Ausbeute: 1,80 g (89 % d. Th.) rotbrauner Feststoff

b) 13,17-Bis-[4-N-(benzyloxycarbonyl)-aminobutyl]-3,8-diethyl-2,7,12,18-tetramethylporphyrin

50 mg (0,09 mmol) der unter Beispiel 19a hergestellten Verbindung werden in 5 ml wasserfreiem Pyridin gelöst. Zu der bei -10°C unter Argon gerührten Lösung tropft man 34 mg (0,20 mmol)Benzyloxycarbonylchlorid zu und läßt den Ansatz bei Raumtemperatur 12 Stunden weiterrühren. Dann zieht man das Lösungsmittel ab, nimmt im Methylenchlorid auf und schüttelt einmal mit zweinormaler Citronensäurelösung und einmal mit konzentrierter Natriumhydrogencarbonatlösung aus und trocknet die organische Phase über Natriumsulfat. Man filtriert, engt ein, chromatographiert an Aluminiumoxid mit Methylenchlorid/Methanol und dampft zur Trockne ein.
Ausbeute: 61 mg (81 % d. Th.) rotbrauner Feststoff

| Analyse (bezogen auf wasserfreie Substanz): | | | | |
|---|---|---|---|---|
| C 74,97 | H 7,26 | N 10,09 | O 7,68 | ber. |
| C 74,76 | H 7,19 | N 9,88 | | |

c) 13,17-Bis-[15-carboxy-6-oxo-8,11-bis-(carboxymethyl)-14-(ethoxycarbonylmethyl)-5,8,11,14-tetraazapentadecyl]-3,8-diethyl-2,7,12,18-tetramethylporphyrin

1,21 g (3,00 mmol) 3-Ethoxy-carbonylmethyl-6-[2-(2,6-dioxomorpholino)-ethyl]-3,6-diazaoctandisäure (DTPA-Monoanhydridmonoethylester) werden in 200 ml wasserfreiem Dimethylformamid suspendiert. Man überschichtet mit Stickstoff, setzt 1,52 g (15,00 mmol) Triethylamin und 0,85 g (1,50 mmol) der unter Beispiel 19a hergestellten Verbindung zu und rührt 3 Tage bei Raumtemperatur. Nach Filtrieren und Eindampfen im Vakuum wird der Rückstand über Kieselgel RP-18 mit Wasser/Tetrahydrofuran chromatographiert und das Eluat im Vakuum eingedampft.
Ausbeute: 1,50 g (73 % d. Th.) rotbraunes Pulver

| Analyse (bezogen auf wasserfreie Substanz): | | | | |
|---|---|---|---|---|
| C 59,55 | H 7,20 | N 12,25 | O 21,00 | ber. |
| C 59,41 | H 7,14 | N 12,13 | | |

d) 13,17-Bis-[15-carboxylato-6-oxo-8,11-bis-(carboxylatomethyl)-14-(ethoxycarbonylmethyl)-5,8,11,14-tetraazapentadecyl]-3,8-diethyl-2,7,12,18-tetramethylporphyrin, Digadoliniumkomplex

1,20 g (0,87 mmol) des unter Beispiel 19c hergestellten Liganden werden in 400 ml Wasser gelöst. Durch Zugabe von zweinormaler wässriger Natronlauge wird pH 7,0 eingestellt, portionsweise 0,78 g (1,91 mmol) Gadoliniumacetat-tetrahydrat und zweinormale wässrige Natronlauge zugesetzt, so daß der pH-Wert des Reaktionsgemisches stets zwischen 6,8 und 7,2 pendelt. Ist alles Gadoliniumacetat zugesetzt, wird über Nacht bei Raumtemperatur nachgerührt. Zur Aufarbeitung wird filtriert, das Lösungsmittel im Vakuum abgezogen und der Rückstand über Kieselgel RP-18 mit Wasser/Tetrahydrofuran chromatographiert und das Eluat im Vakuum zur Trockne eingeengt.
Ausbeute: 1,01 g (69 % d. Th.) rotbraunes Pulver

| Analyse (bezogen auf wasserfreie Substanz): | | | | | |
|---|---|---|---|---|---|
| C 48,62<br>C 48,46 | H 5,52<br>H 5,47 | Gd 18,72<br>Gd 18,75 | N 10,10<br>N 9,83 | O 17,14 | ber. |

e) 13,17-Bis-[15-carboxylato-6-oxo-8,11,14-tris-(carboxylatomethyl)-5,8,11,14-tetraazapentadecyl]-3,8-diethyl-2,7,12,18-tetramethylporphyrin, Digadoliniumkomplex, Dinatriumsalz

0,90 g (0,66 mmol) des unter Beispiel 19c hergestellten Liganden werden in 300 ml Wasser gelöst. Durch Zugabe von 10molarer wäßriger Natronlauge wird pH 13,0 eingestellt und 5 Stunden bei Raumtemperatur gerührt. Nach vollständiger Verseifung des Esters wird mit konzentrierter Salzsäure pH 7,0 eingestellt und wie unter Beispiel 19d beschrieben, mit 0,59 g (1,44 mmol) Gadoliniumacetat-tetrahydrat komplexiert und gereinigt.
Ausbeute: 0,91 g (83 % d. Th.) rotbraunes Pulver

| Analyse (bezogen auf wasserfreie Substanz): | | | | | | |
|---|---|---|---|---|---|---|
| C 46,09<br>C 45,85 | H 4,96<br>H 4,87 | Gd 18,86<br>Gd 18,72 | N 10,08<br>N 9,84 | Na 2,75<br>Na 2.54 | O 17,27 | ber. |

**Beispiel 20**

a) 13,17-Bis-[4-(4-aminophenyl)-4-oxabutyl]-3,8-diethyl-2,7,12,18-tetramethylporphyrin

3,28 g (30,10 mmol) 4-Aminophenol und 1,69 g (30,10 mmol) gepulvertes Kaliumhydroxid werden in 50 ml wasserfreiem Dimethylformamid unter Argon 30 Minuten bei Raumtemperatur gerührt und anschließend mit 2,00 g (3,01 mmol) 13,17-Bis-(3-brompropyl)-3, 8-diethyl-2,7,12,18-tetramethylporphyrin (CA RN 112635-99-1) versetzt. Nach 30 Stunden Rühren bei Raumtemperatur wird filtriert, im Vakuum weitgehend eingedampft, der Rückstand in Methylenchlorid aufgenommen und jeweils einmal mit Wasser und mit gesättigter wässriger Natriumhydrogencarbonatlösung ausgeschüttelt. Die organische Phase wird über Natriumsulfat getrocknet, filtriert und eingedampft und der Rückstand an Aluminiumoxid mit Methylenchlorid/Methanol chromatographiert. Nach dem Eindampfen erhält man das Produkt als rotbraunen Feststoff, der aus Methylenchlorid/Ether umkristallisiert werden kann.
Ausbeute: 1,97 g (91 % d. Th.) rotbraunes Pulver

| Analyse (bezogen auf wasserfreie Substanz): | | | | |
|---|---|---|---|---|
| C 76,64<br>C 76,48 | H 7,27<br>H 7,21 | N 11,66<br>N 11,54 | O 4,44 | ber. |

b) 13,17-Bis-{4-(4-(11-carboxy-2-oxo-4,7-bis-(carboxymethyl)-10-(ethoxycarbonylmethyl)-1,4,7,10-tetraazaundecyl)-phenyl]4-oxabutyl}-3,8-diethyl-2,7,12,18-tetramethylporphyrin

1,21 g (3,00 mmol) 3-Ethoxy-carbonylmethyl-6-[2-(2,6-dioxomorpholino)-ethyl]-3,6-diazaoctandisäure (DTPA-Monoanhydridmonoethylester) werden in 200 ml wasserfreiem Dimethylformamid suspendiert. Man überschichtet mit Stickstoff, setzt 1,52 g (15,00 mmol) Triethylamin und 1,08 g (1,50 mmol) der unter Beispiel 20a hergestellten Verbindung zu und rührt 3 Tage bei Raumtemperatur. Nach Filtrieren und Eindampfen im Vakuum wird der Rückstand über Kieselgel RP-18 mit Wasser/Tetrahydrofuran chromatographiert und im Vakuum zur Trockne eingeengt.
Ausbeute: 1,89 g (82 % d. Th.) rotbraunes Pulver

| Analyse (bezogen auf wasserfreie Substanz): | | | | |
|---|---|---|---|---|
| C 61,32<br>C 61,09 | H 6,73<br>H 6,64 | N 11,00<br>N 10,87 | O 20,95 | ber. |

c) 13,17-Bis-{4-(4-(11-carboxylato-2-oxo-4,7-bis-(carboxylatomethyl)-10-(ethoxycarbonylmethyl)-1,4,7,10-tetraazaundecyl)-phenyl]-4-oxabutyl}-3,8-diethyl-2,7,12,18-tetramethylporphyrin, Digadoliniumkomplex

1,50 g (0,98 mmol) des unter Beispiel 20b hergestellten Liganden werden in 350 ml Wasser gelöst. Durch Zugabe von zweinormaler wäßriger Natronlauge wird pH 7,0 eingestellt, portionsweise 0,88 g (2,16 mmol) Gadoliniumacetat-tetrahydrat und zweinormale wäßrige Natronlauge zugesetzt, so daß der pH-Wert des Reaktionsgemisches stets zwischen 6,8 und 7,2 pendelt. Ist alles Gadoliniumacetat zugesetzt, wird über Nacht bei Raumtemperatur nachgerührt. Zur Aufarbeitung wird filtriert, das Lösungsmittel im Vakuum abgezogen und der Rückstand über Kieselgel RP-18 mit Wasser/Tetrahydrofuran chromatographiert. Das Eluat wird im Vakuum zur Trockne eingeengt.
Ausbeute: 1,44 g (80 % d. Th.) rotbraunes Pulver

| Analyse (bezogen auf wasserfreie Substanz): | | | | | |
|---|---|---|---|---|---|
| C 51,02<br>C 50,83 | H 5,27<br>H 5,18 | Gd 17,13<br>Gd 17,11 | N 9,15<br>N 8,94 | O 17,43 | ber. |

d) 13,17-Bis-{4-(4-(11-carboxylato-2-oxo-4,7,10-tris-(carboxylatomethyl)-1,4,7,10-tetraazaundecyl)-phenyl]-4-oxabutyl}-3,8-diethyl-2,7,12,18-tetramethylporphyrin, Digadoliniumkomplex, Dinatriumsalz

1,50 g (0,98 mmol) des unter Beispiel 20b hergestellten Liganden werden in 350 ml Wasser gelöst. Durch Zugabe von 10molarer wäßriger Natronlauge wird pH 13,0 eingestellt und 5 Stunden bei Raumtemperatur gerührt. Nach vollständiger Verseifung des Esters wird mit konzentrierter Salzsäure pH 7,0 eingestellt und wie unter Beispiel 20c beschrieben, mit 0,88 g 2,16 mmol) Gadoliniumacetat-tetrahydrat komplexiert, aufgearbeitet und gereinigt.
Ausbeute: 1,41 g (79 % d. Th.) rotbraunes Pulver

| Analyse (bezogen auf wasserfreie Substanz): | | | | | | |
|---|---|---|---|---|---|---|
| C 48,73<br>C 48,61 | H 4,75<br>H 4,72 | Gd 17,24<br>Gd 17,25 | N 9,22<br>N 9,13 | Na 2,51<br>Na 2,39 | O 17,54 | ber. |

Beispiel 21

a) Mangan(III)-{N,N'-Bis-[11-carboxy-2-oxo-4,7-bis-(carboxymethyl)-10-(ethoxycarbonylmethyl)-1,4,7,10-tetraazaundecyl]-3,8-bis-(1-propyl)-deuteroporphyrin-IX-13,17-diamid}-acetat

2,00 g (1,40 mmol) des unter Beispiel 14d hergestellten Liganden und 10,00 g Mangan(II)acetat-Tetrahydrat wurden in 150 ml Essigsäure eine Stunde lang unter Rückfluß gekocht. Man dampft im Vakuum bis zur Trockne ein und chromatographiert den Rückstand in einem Gemisch aus Dichlormethan, Methanol, Essigsäure und Wasser. Die produkthaltigen Fraktionen ergeben nach dem Eindampfen und Trocknen im Vakuum ein feinkristallines, rotbraunes Pulver.
Ausbeute: 2,01 g (93 % d. Th.)

| Analyse (bezogen auf wasserfreie Substanz): | | | | | |
|---|---|---|---|---|---|
| C 54,54<br>C 54,63 | H 6,34<br>H 6,41 | Mn 3,56<br>Mn 3,42 | N 12,72<br>N 12,58 | O 22,83 | ber. |

b) Mangan(III)-{N,N'-Bis-[11-carboxylato-2-oxo-4,7-bis-(carboxylatomethyl)-10-(ethoxycarbonylmethyl)-1,4,7,10-tetraazaundecyl]-3,8-bis-(1-propyl)-deuteroporphyrin-IX-13,17-diamid}-acetat, Digadoliniumkomplex

1,92 g (1,25 mmol) des unter Beispiel 21a hergestellten Liganden werden in 300 ml Wasser gelöst. Durch Zugabe von zweinormaler Natronlauge wird pH 7,0 eingestellt, portionsweise 1,18 g (2,90 mmol) Gadoliniumacetat-tetrahydrat und zweinormale wässrige Natronlauge angesetzt, so daß der pH-Wert stets zwischen-6,8 und 7,2 pendelt. Ist alles Gadoliniumacetat zugesetzt, wird über Nacht bei Raumtemperatur nachgerührt. Zur Aufarbeitung wird filtriert, das Lösungsmittel im Vakuum abgezogen und der Rückstand über Kieselgel RP-18 mit Wasser/Tetrahydrofuran chromatographiert. Die im Produkt enthaltenden Fraktionen werden im Vakuum zur Trockne eingedampft.
Ausbeute: 1,89 g (82 % d. Th.) rotbraunes Pulver

| Analyse (bezogen auf wasserfreie Substanz): | | | | | | |
|---|---|---|---|---|---|---|
| C 45,45 | H 4,96 | Gd 17,00 | Mn2,97 | N 10,60 | O 19,03 | ber. |
| C 45,22 | H 4,83 | Gd 16,89 | Mn 3,05 | N 10,50 | | |

c) Mangan(III)-{N,N'-Bis-[11-Carboxylato-2-oxo-4,7-bis-(carboxylatomethyl)-10-(ethoxycarbonylmethyl)-1,4,7,10-tetraazaundecyl]-3,8-bis-(1-propyl)-deuteroporphyrin-IX-13,17-diamid}-acetat, Digadoliniumkomplex, Dinatriumsalz

1,55 g (1,01 mmol) des unter Beispiel 21a hergestellten Manganporphyrins werden in 150 ml Wasser gelöst. Durch Zugabe von 10 molarer wässriger Natronlauge wird pH 13 eingestellt und 5 Stunden bei Raumtemperatur gerührt. Nach vollständiger Verseifung des Esters wird mit konzentrierter Salzsäure pH 7,0 eingestellt und wie unter Beispiel 21b beschrieben, mit 0,90 g (2,21 mmol) Gadoliniumacetat-tetrahydrat komplexiert und gereinigt.
Ausbeute: 1,45 g (78 % d. Th.) rotbraunes Pulver

| Analyse (bezogen auf wasserfreie Substanz): | | | | | | | |
|---|---|---|---|---|---|---|---|
| C 43,13 | H 4,44 | Gd 17,11 | Mn 2,99 | N 10,67 | Na 2,50 | O 19,15 | ber. |
| C 43,24 | H 4,52 | Gd 17,03 | Mn 2,84 | N 10,39 | Na 2,81 | | |

**Beispiel 22**

a) 9 Nacktmäusen (Balb/c nu/nu) mit subcutan implantierten Coloncarcinomen (HT 29, WiDr) wurden je 0.1 mmol/kg des Mesoporhpyrinderivats Beispiel 1b (10 mmol/l gelöst in Aqua bidest., pH 7.2, 37°C) i.v. appliziert. Nach 0.5 h, 3 h und 24 h wurden die Tiere getötet und präpariert. Die Gewebekonzentrationen wurden nach entsprechender Aufarbeitung ($HNO_3$ konz.) mittels ICP-AES bestimmt und auf % der applizierten Dosis pro Gramm Gewebefrischgewicht berechnet.

Tabelle 1

| % der Dosis/g Organ | | | |
|---|---|---|---|
| | über 0.5 Std. MW ± S | über 3 Std. MW ± S | über 24 Std. MW ± S |
| Blut | 19.34 ± 1.06 | 6.11 ± 1.13 | 0.21 ± 0.04 |
| Leber | 9.26 ± 0.10 | 8.68 ± 0.53 | 10.47 ± 0.64 |
| Nieren | 42.29 ± 7.05 | 6.71 ± 0.79 | 3.93 ± 1.01 |
| Milz | 7.02 ± 0.53 | 3.21 ± 0.46 | 3.85 ± 0.63 |
| Muskel | 3.13 ± 0.68 | 0.85 ± 0.15 | 0.53 ± 0.04 |
| Dann | 3.53 ± 0.07 | 2.03 ± 0.13 | 0.95 ± 0.30 |
| Haut | 8.63 ± 0.91 | 2.81 ± 0.30 | 2.71 ± 0.42 |
| Lunge | 13.83 ± 1.43 | 4.57 ± 0.67 | 1.84 ± 0.38 |
| HT29-Tumor | 4.80 ± 1.23 | 3.03 ± 0.22 | 1.95 ± 0.52 |
| WiDr-Tumor | 4.54 ± 3.37 | 3.23 ± 0.95 | 2,71 ± 0.57 |
| Restkörper | 4.84 ± 0.48 | 1.57 ± 0.21 | 1.20 ± 0.45 |

Schon 0.5 h p.i. beträgt die Konzentration in der Leber 10 % der applizierten Dosis pro Gramm Gewebefrischgewicht. Während in allen anderen untersuchten Geweben die Konzentration innerhalb von 24 h drastisch abfällt, bleibt die Porphyrinkonzentration im Lebergewebe konstant bei 10 %.

Tabelle 2:

| Mittelwerte (Enhancement): | | | | | |
|---|---|---|---|---|---|
| Zeit (min) | HT29 | WiDr | Leber | Niere | Muskel |
| 0 | 1,00 | 1,00 | 1,00 | 1,00 | 1,00 |
| 5 | 1,36 | 1,16 | 1,43 | 2,89 | 1,41 |
| 15 | 1,49 | 1,16 | 1,43 | 2,70 | 1,41 |
| 30 | 1,54 | 1,18 | 1,40 | 2,65 | 1,39 |
| 45 | 1,55 | 1,25 | 1,42 | 2,79 | 1,40 |
| 60 | 1,59 | 1,05 | 1,38 | 2,56 | 1,29 |
| 75 | 1,97 | 1,23 | 1,51 | 2,53 | 1,47 |
| 90 | 1,51 | 1,28 | 1,43 | 2,40 | 1,50 |
| 120 | 1,64 | 1,22 | 1,47 | 2,15 | 1,34 |
| 24 h | 1,05 | 1,64 | 1,19 | 1,51 | 1,24 |

Schon 5 min p.i. zeigte sich eine deutliche Signalintensitätssteigerung im Lebergewebe und nach 30 min im untersuchten Tumor. Der maximale Enhancement (Leber: +50 %, Tumor: +100 %) wurde 75 min p.i. erreicht. Im Muskel als Referenzgewebe war dagegen nur eine geringe Änderung festzustellen. In den Nieren war eine stärkere Signalintensitätänderung festzustellen.

**Beispiel 23**

a) An einem Bruker Biospec Kernspintomographen (2.35 Tesla) wurde die Signalintenstätsänderung in verschiedenen Geweben von tumortragenden (HT29-Coloncarcinom) Nacktmäusen (Balb/c nu/nu) nach i. v. Gabe von 0.05 mmol/kg der Verbindung nach Beispiel 1c (10 mmol/l, pH 7.2; 37°C in Aqua bidest.) untersucht. Die Messung erfolgt mit einer multi slice single echo Sequenz (MSSE). Aufnahmeparameter: TR: 400 ms, TE: 30 ms, Schichtdicke: 4 mm, Number of averages: 4/slice, Matrix $256^2$. Nach einer Voraufnahme wurden 0.05 mmol/kg der Substanz appliziert und die Signalintensitätsänderung (bezogen auf einen internen Standard) 5, 15, 30, 45, 60, 75, 90 und 120 min p.i. untersucht.

Tabelle 3:

| Enhancement nach intravenöser Applikation von 0.05 mmol/kg Körpergewicht | | | | |
|---|---|---|---|---|
| Zeit (min) | Leber | Muskel | HT 29 | Niere |
| 0 | 100 | 100 | 100 | 100 |
| 5 | 122 ± 31 | 119 ± 8 | 116 ± 19 | 185 ± 34 |
| 60 | 164 ± 22 | 129 ± 7 | 128 ± 21 | 240 ± 26 |
| 90 | 202 ± 37 | 143 ± 21 | 148 ± 22 | 248 ± 18 |
| 120 | 194 ± 33 | 147 ± 17 | 155 ± 30 | 246 ± 6 |
| 150 | 197 ± 17 | 146 ± 8 | 167 ± 29 | 225 ± 12 |
| 180 | 212 ± 9 | 157 ± 8 | 161 ± 22 | 212 ± 16 |

Schon 5 min p.i. zeigte sich eine deutliche Signalintensitätsteigerung im Lebergewebe, während in Tumor und Muskel als Referenzgewebe nur eine geringe Änderung festzustellen war. Dieser Effekt blieb während des gesamten Beobachtungszeitraums bestehen. Lediglich in den Nieren ist bis 3 h p.i. eine noch stärkere Signalintensitätsänderung festzustellen.

b) An einem Bruker Biospec Kernspintomographen (2.35 Tesla) wurde die Signalintensitätsänderung in verschiedenen Geweben von Ratten mit einem DMBAinduziertem Mamacarcinom nach i.v. Gabe von 0.05 mmol/kg bzw. 0.1 mmol Verbindung Beispiel 1c (20 mmol/l, pH 7.2; 37°C in Aqua bidest.) untersucht. Die Messungen erfolgten mit einer multi slice variable echo Sequenz (MSVE).
Aufnahmeparameter: TR: 400 ms, TE: 25 ms, Schichtdicke: 4 mm, Number of averages: 4/slice, Matrix $256^2$.
Nach einer Voraufnahme wurden 0.05 mmol/kg bzw. 0.1 mmol/kg der Substanz appliziert und die Signalintensitätsänderung (bezogen auf einen internen Standard) untersucht.
Die Ergebnisse sind in den nachfolgenden Tabelle zusammengefaßt. Die $^1$H-NMR-Bilder vom DMBA-induzierten Mammacarcinom nach Gabe von 0,1 mmol/Kg i.v. nach 5 Minuten bzw. 3 oder 24 Stunden p.i. sind in Figur 1 wiedergegeben.

Tabelle 4:

| Enhancement nach intravenöser Applikation von 0,05 mmol/kg (Mittelwerte) | | | | |
|---|---|---|---|---|
| Zeit (min) | Tumor | Leber | Niere | Muskel |
| 0 | 1,00 | 1,00 | 1,00 | 1,00 |
| 5 | 1,88 | 1,48 | 2,57 | 1,34 |
| 15 | 1,97 | 1,29 | 2,81 | 1,26 |
| 30 | 1,71 | 1,42 | 2,94 | 1,22 |
| 45 | 1,71 | 1,49 | 2,84 | 1,12 |
| 60 | 1,50 | 1,52 | 2,53 | 1,10 |
| 75 | 1,71 | 1,50 | 2,47 | 1,04 |
| 90 | 1,62 | 1,53 | 2,15 | 1,11 |
| 105 | 1,75 | 1,63 | 2,63 | 1,07 |
| 160 | 1,69 | 1,47 | 2,38 | 1,07 |
| 180 | 1,67 | 1,57 | 2,54 | 1,02 |
| 24h | 1,31 | 0,93 | 1,17 | 1,07 |

Tabelle 5:

| Enhancement nach intravenöser Applikation von 0,1 mmol/kg (Mittelwerte) | | | | |
|---|---|---|---|---|
| Zeit (min) | Tumor | Leber | Niere | Muskel |
| 0 | 1,00 | 1,00 | 1,00 | 1,00 |
| 1 | 2,48 | 1,55 | 1,16 | 1,46 |
| 15 | 2,40 | 1,61 | 2,36 | 1,30 |
| 26 | 2,41 | 1,45 | 2,33 | 1,37 |
| 40 | 2,34 | 1.51 | 2,38 | 1,23 |
| 60 | 2,34 | 1,36 | 1,86 | 1,23 |
| 71 | 2,36 | 1,62 | 1,66 | 1,17 |
| 85 | 2,39 | 1,48 | 1,64 | 1,32 |
| 115 | 2,38 | 1,62 | 2,43 | 1,23 |
| 150 | 2,31 | 1,48 | 2,27 | 1,17 |
| 180 | 2,38 | 1,59 | 2,18 | 1,22 |
| 24h | 2,02 | 1,33 | 0,94 | 1,15 |

Schon 5 min p.i. zeigte sich eine deutliche Signalintensitätssteigerung im Lebergewebe und insbesondere in den untersuchten Tumoren. Im Muskel als Referenzgewebe war dagegen nur eine geringe Änderung festzustellen. Dieser Effekt blieb während des gesamten Beobachtungszeitraums (bis 24 h p.i.) bestehen. Lediglich in den Nieren ist nach Applikation von 0,05 mmol/kg eine stärkere Signalintensitätsänderung festzustellen.

c) An einem Bruker Biospec Kernspintomographen (2.35 Tesla) wurde die Signalintensitätsänderung in verschiedenen Geweben von tumortragenden (Novikoff-Hepatom, i.m.) Nacktratten (LEW/Mol rnu/rnu) nach einmaliger intravenöser Applikation von 0.1 mmol/kg Verbindung Beispiel 1 c (20 mmol/l; pH 7.2-7.4, 37°C in Tris/NaCl-Puffer) untersucht. Die Messungen erfolgten mit einer multi slice single echo Sequenz (MSSE). Aufnahmeparameter: TR: 412 ms, TE: 25 ms, Schichtdicke: 4 mm, Number of averages: 4/slice, Matrix $256^2$. Nach einer Voraufnahme wurden jeweils 0.1 mmol/kg der Substanz in eine Caudalvene appliziert und die Signalintensitätsänderungen (bezogen auf einen mitgemessenen Standard) im Abstand von 15 bzw. 30 min bis 180 min p. i. untersucht. Angegeben sind die relativen Intensitäten bezogen auf die jeweiligen Ausgangsintensität (=1.00 gesetzt).

Tabelle 6:

| gemitteltes Enhancement n = 3: | | | | |
|---|---|---|---|---|
| Zeit (min) | Tumor MW ± S | Leber MW ± S | Niere MW ± S | Muskel MW ± S |
| 0 | 1,00 ± 0,00 | 1,00 ± 0,00 | 1,00 ± 0,00 | 1,00 ± 0,00 |
| 1 | 1,63 ± 0,24 | 1,43 ± 0,24 | 1,67 ± 0,35 | 1,60 ± 0,01 |
| 15 | 1,71 ± 0,19 | 1,34 ± 0,22 | 2,13 ± 0,28 | 1,54 ± 0,06 |
| 30 | 1,72 ± 0,23 | 1,31 ± 0,21 | 2,14 ± 0,38 | 1,47 ± 0,08 |
| 45 | 1,79 ± 0,21 | 1,17 ± 0,16 | 2,10 ± 0,30 | 1,41 ± 0,10 |
| 60 | 1,78 ± 0,24 | 1,31 ± 0,09 | 2,12 ± 0,19 | 1,33 ± 0,05 |
| 75 | 1,64 ± 0,17 | 1,37 ± 0,05 | 1,93 ± 0,19 | 1,37 ± 0,01 |
| 90 | 1,75 ± 0,27 | 1,22 ± 0,24 | 1,96 ± 0,08 | 1,24 ± 0,03 |
| 120 | 1,77 ± 0,23 | 1,23 ± 0,07 | 1,92 ± 0,33 | 1,28 ± 0,08 |
| 150 | 1,73 ± 0,23 | 1,16 ± 0,22 | 1,86 ± 0,18 | 1,20 ± 0,06 |
| 180 | 1,85 ± 0,09 | 1,07 ± 0,20 | 1,84 ± 0,24 | 1,15 ± 0,04 |
| 190 | 1,45 ± 0,00 | 1,27 ± 0,00 | 1,73 ± 0,00 | 1,21 ± 0,00 |

Direkt nach Applikation zeigte sich in den untersuchten Geweben ein deutlicher Anstieg in der Signalintensität (± 60 %). Während aber in Leber und Muskel nach 2-3 h fast wieder die Anfangsintensität erreicht war, blieb die Intensität im Tumor während des Beobachtungszeitraums nahezu unverändert hoch. Die Substanz wird offensichtlich überwiegend renal eliminiert. Die Signalintensität in den Nieren lag deshalb bis 150 min hoch über der, die in den Tumoren bestimmt werden konnte.

d) An einem Bruker Biospec Kernspintomographen (2.35 Tesla) wurde die Signalintensitätänderung in verschiedenen Geweben von tumortragenden (HT29-und WiDr-Coloncarcinom) Nacktmäusen (Balb/c nu/nu) nach i. v. Gabe von 0.5 mmol/kg Verbindung Beispiel 1c (10 mmol/l, pH 7.2; 37°C in Aqua bidest.) untersucht. Die Messung erfolgt mit einer multi slice single echo Sequenz (MSSE). Aufnahmeparameter: TR: 400 ms, TE: 30 ms, Schichtdicke: 4 mm, Number of averages: 4/slice, Matrix $256^2$. Nach einer Voraufnahme wurden 0.5 mmol/kg der Substanz appliziert und die Signalintensitätsänderung bis 180 min p. i. untersucht.

Schon 5 min p. i. zeigte sich eine deutliche Signalintensitätssteigerung im Lebergewebe und in den beiden untersuchten Tumoren. Im Muskel als Referenzgewebe war dagegen ein geringerer Signalanstieg festzustellen. Dieser Effekt blieb während des gesamten Beobachtungszeitraums bestehen. Figur 2 zeigt das $^1$H-NMR-Bild des WiDr-Coloncarcinom nach 5, 82 bzw. 180 Minuten p.i., Figur 3 das des HT29-Carcinoms.

e) 9 Nacktmäusen (Balb/c nu/nu) mit subcutan implantierten Coloncarcinomen (HT 29 und WiDr) wurden je 0.1 mmol/kg des Deuteroporphyrinderivats Verbindung nach Beispiel 1 c (10 mmol gelöst in Aqua bidest., pH 7.2, 37°C) i. v. appliziert. Nach 0.5 h, 3 h und 24 h wurden die Tiere getötet und präpariert. Die Gewebekonzentrationen wurden nach entsprechender Aufarbeitung ($HNO_3$ konz.) mittels ICP-AES bestimmt und auf % der applizierten

Dosis pro Gramm Gewebefrischgewicht berechnet. Außerdem wurden die Geweberelaxationszeiten T1 und T2 bestimmt (Bruker minispec pc 120).

Tabelle 7:

| % der applizierten Dosis pro Gramm Gewebefrischgewicht | | | |
|---|---|---|---|
| | 0.5 Std | 3 Std. | 24 Std. |
| Blut | 4,78 ± 0,77 | 2,60 ± 0.91 | 0,13 ± 0,15 |
| Leber | 1,87 ± 0,71 | 2,03 ± 0.34 | 2,05 ± 0,00 |
| Nieren | 4,06 ± 1,01 | 4.47 ± 1,63 | 4,20 ± 0,27 |
| Milz | 1,31 ± 1,20 | 0,62 ± 0,19 | 0,63 ± 0,06 |
| Muskel | 0.38 ± 0,14 | 0,35 ± 0,02 | 0,10 ± 0,02 |
| Darm | 0.32 ± 0,09 | 0,49 ± 0,07 | 1,56 ± 0,65 |
| Haut | 2,80 ± 0,71 | 1,61 ± 0,14 | 0,65 ± 0,12 |
| Lunge | 2,45 ± 0,14 | 1,71 ± 0,37 | 0,16 ± 0,03 |
| HT29-Tumor | 1,09 ± 0,14 | 1,46 ± 0,22 | 0,86 ± 0,14 |
| WiDr-Tumor | 1,51 ± 0,20 | 1,25 ± 0,16 | 0,58 ± 0,02 |
| Restkörper | 1,13 ± 0.04 | 0,58 ± 0,07 | 0,26 ± 0,04 |

Aufgrund der hohen Relaxivity der Verbindung (17,86 1 $mmol^{-1}s^{-1}$) konnten trotz relativ niedriger Gewebekonzentrationen in beiden Tumoren deutlich verkürzte T1-Geweberelaxionszeiten gemessen werden (Faktor 2-3). Diese Verkürzung der Relaxationszeiten soll zumindest bis 3 h p.i. für ein Enhancement im NMR-Imagingexperiment (T1-gewichtete Spin-Echo-Sequenz) ausreichen.

Tabelle 8:
Geweberelaxationszeit $T_1$

| $T_1$ (ms) | 0,5 Std. | 3 Std. | 24 Std. |
|---|---|---|---|
| Leber | 220 ± 20 | 220 ± 30 | 260 ± 10 |
| Nieren | 160 ± 10 | 230 ± 30 | 240 ± 20 |
| Muskel | 500 ± 40 | 560 ± 40 | 660 ± 10 |
| HT29-Tumor | 410 ± 30 | 440 ± 70 | 540 ± 20 |
| WiDr-Tumor | 320 ± 40 | 430 ± 50 | 640 ± 10 |

| $T_1$ (ms) | Kontrolle |
|---|---|
| Leber | 380 ± 30 |
| Nieren | 500 ± 20 |
| Muskel | 580 ± 10 |
| HT29-Tumor | 840 ± 50 |
| WiDr-Tumor | 760 ± 100 |

Tabelle 9:
Geweberelaxationszeit $T_2$

| $T_2$(ms) | 0,5 Std. | 3 Std. | 24 Std. |
|---|---|---|---|
| Leber | 34 ± 3 | 42 ± 2 | 48 ± 7 |
| Nieren | 42 ± 1 | 47 ± 5 | 54 ± 10 |
| Muskel | 32 ± 1 | 36 ± 3 | 31 ± 1 |
| HT29-Tumor | 81 ± 4 | 72 ± 8 | 83 ± 7 |
| WiDr-Tumor | 67 ± 7 | 72 ± 7 | 85 ± 2 |

| $T_2$ (ms) | Kontrolle |
|---|---|
| Leber | 51 ± 2 |
| Nieren | 69 ± 4 |
| Muskel | 58 ± 5 |
| HT29-Tumor | 101 ± 8 |
| WiDr-Tumor | 118 ± 19 |

f) 9 Nacktmäusen (Balb/c nu/nu) mit subcutan implantierten Carcinomen (HT29, WiDr und MATLu) wurden je 0.1 mmol/kg des Deuteroporphyrinderivats Beispiel 1c (10 mmol/L gelöst in Aqua bidest., pH 7.2, 37°C) i.v. appliziert. Nach 2 h wurden die Tiere getötet und präpariert. Die Gewebekonzentrationen wurden nach entsprechender Aufarbeitung ($HNO_3$ konz.) mittels ICP-AES bestimmt und auf % der applizierten Dosis pro Gramm Gewebefrischgewicht berechnet. Außerdem wurden die Geweberelaxationszeiten T1 und T2 bestimmt (Bruker minispec pc 120). Zur Bestimmung der Leerwerte wurden Kontrolltiere ohne KM-Applikation entsprechend untersucht

Tabelle 10:

| % der applizierten Dosis pro Gramm Gewebefrischgewicht | | | |
|---|---|---|---|
| | HT 29 | WiDr | MATLu |
| Blut | 2,74 ± 0,90 | 2,13 ± 0,01 | 1,14 ± 0,10 |
| Leber | 1,49 ± 0,17 | 1,28 ± 0,07 | 0,97 ± 0,11 |
| Nieren | 6,23 ± 1,80 | 4,23 ± 0,19 | 2,73 ± 0,33 |
| Milz | 0,64 ± 0,21 | 0,44 ± 0,04 | 0,31 ± 0,06 |
| Muskel | 0,24 ± 0,11 | 0,19 ± 0,03 | 0,26 ± 0,10 |
| Darm | 1,18 ± 0,22 | 0,73 ± 0,34 | 0,68 ± 0,08 |
| Haut | 1,34 ± 0,52 | 0,87 ± 0,13 | 0,41 ± 0,38 |
| Lunge | 2,09 ± 0,42 | 1,54 ± 0,09 | 3,69 ± 3,58 |
| HT29 | 1,00 ± 0,27 | | |
| WiDr | | 0,83 ± 0,05 | |
| MATLu | | | 1,92 ± 2,09 |
| Restkörper | 0,54 ± | 0,4 ± 0,04 | 0,27 ± 0,01 |

In allen drei Tumoren konnten, u.a. aufgrund der hohen Relaxivity der Verbindung (17,86 1 $mmol^{-1}s^{-1}$), deutlich verkürzte T1-Geweberelaxationszeiten gemessen werden (Faktor 2-3). Im Prostatacarcinom (MATLu) war die Gewebekonzentration und der Effekt auf die Geweberelaxationszeiten tendenziell größer als in den Coloncarcinomen

(HT29 und WiDr). Die Verkürzung der Relaxationszeiten soll in allen untersuchten Tumoren fiir ein Enhancement im NMR-Imagingexperiment (T1-gewichtete Spin-Echo-Sequenz) ausreichen.

Tabelle 11:
Geweberelaxationszeit $T_1$

| $T_1$ (ms) | HT 29 | WiDr | MATLu |
|---|---|---|---|
| Leber | 174 ± 8 | 180 ± 30 | 191 ± 10 |
| Nieren | 150 ± 12 | 159 ± 30 | 171 ± 20 |
| Muskel | 489 ± 57 | 508 ± 40 | 506 ± 10 |
| HT29-Tumor | 375 ± 32 | | |
| WiDr-Tumor | | 358 ± 70 | |
| MATLu | | | 361 ± 65 |

| $T_1$ (ms) | Kontrolle |
|---|---|
| Leber | 380 ± 30 |
| Nieren | 500 ± 20 |
| Muskel | 580 ± 10 |
| HT29-Tumor | 840 ± 50 |
| WiDr-Tumor | 760 ± 100 |
| MATLu | 1024 ± 22 |

Tabelle 12:
Geweberelaxationszeit $T_2$

| $T_2$(ms) | HT 29 | WiDr | MATLu |
|---|---|---|---|
| Leber | 42 ± 2 | 53 ± 5 | 51 ± 2 |
| Nieren | 59 ± 3 | 64 ± 2 | 60 ± 1 |
| Muskel | 56 ± 2 | 62 ± 3 | 54 ± 2 |
| HT29-Tumor | 94 ± 7 | | |
| WiDr-Tumor | | 110 ± 9 | |
| MATLu | | | 102 ± 10 |

| $T_2$ (ms) | Kontrolle |
|---|---|
| Leber | 51 ± 2 |
| Nieren | 69 ± 4 |
| Muskel | 58 ± 5 |
| HT29-Tumor | 101 ± 8 |
| WiDr-Tumor | 118 ± 19 |
| MATLu | 142 ± 14 |

**Beispiel 24**

An einem Bruker Biospec Kernspintomographen (2.35 Tesla) wurde die Signalintenstätsänderung in verschiedenen Geweben von tumortragenden (HT29- und WiDr-Coloncarcinom) Nacktmäusen (Balb/c nu/nu) nach i. v. Gabe von 0.05 mmol/kg der Verbindung nach Beispiel 2c (10 mmol/l, pH 7.2; 37°C in Aqua bidest.) untersucht. Die Messung erfolgt mit einer multi slice single echo Sequnz (MSSE). Aufnahmeparameter: TR: 400 ms, TE: 30 ms, Schichtdicke: 4 mm, Number of averages: 4/slice, Matrix $256^2$. Nach einer Voraufnahme wurden 0.05 mmol/kg der Substanz appliziert und die Signalintensitätsänderung (bezogen auf einen internen Standard) 5, 30, 60, 120 und 180 min p.i. untersucht.

Tabelle 13:

| Enhancement nach intravenöser Applikation von 0.05 mmol/kg Körpergewicht | | | | | |
|---|---|---|---|---|---|
| Zeit (min) | Leber | Muskel | HT 29 | WiDr | Niere |
| 0 | 100 | 100 | 100 | 100 | 100 |
| 5 | 173 ± 31 | 143 ± 23 | 171 ± 14 | 165 ± 39 | 169 ± 45 |
| 30 | 157 ± 30 | 157 ± 29 | 169 ± 14 | 170 ± 32 | 211 ± 33 |
| 60 | 170 ± 16 | 120 ± 8 | 168 ± 12 | 153 ± 33 | 207 ± 31 |
| 120 | 179 ± 37 | 126 ± 10 | 178 ± 32 | 153 ± 0 | 191 ± 37 |
| 180 | 167 ± 7 | 112 ± 9 | 136 ± 23 | 117 ± 33 | 136 ± 25 |

Schon 5 min p.i. zeigte sich eine deutliche Signalintensitätsteigerung im Lebergewebe und in den beiden untersuchten Tumoren. Im Muskel als Referenzgewebe war dagegen nur eine geringe Änderung festzustellen. Dieser Effekt blieb während des gesamten Beobachtungszeitraums bestehen. Der größte Signalintensitätsunterschied ist 2 h p.i. bzw. 3 h p.i. festzustellen. Lediglich in den Nieren ist bis 2 h p.i. eine noch stärkere Signalintensitätsänderung festzustellen.

**Beispiel 25**

9 Nacktmäusen (Balb/c nu/nu) mit subcutan implantierten Coloncarcinomen (HT 29, WiDr) wurden je 0.1 mmol/kg des Mesoporphyrinderivats Beispiel 8e (10 mmol/l gelöst in Aqua bidest., pH 7.2, 37°C) i.v. appliziert. Nach 0.5 h, 3 h und 24 h wurden die Tiere getötet und präpariert. Die Gewebekonzentrationen wurden nach entsprechender Aufarbeitung ($HNO_3$ konz.) mittels ICP-AES bestimmt und auf % der applizierten Dosis pro g Gewebefrischgewicht berechnet.

Tabelle 14:

| % der applizierten Dosis pro Gramm Gewebefrischgewicht | | | |
|---|---|---|---|
| | über 0.5 Std. MW ± S | über 3 Std. MW ± S | über 24 Std. MW ± S |
| Blut | 23.59 ± 1.54 | 13.84 ± 1.34 | 2.82 |
| Leber | 8.94 ± 0.24 | 15.12 ± 0.23 | 29.73 |
| Nieren | 18.90 ± 1.65 | 22.68 ± 1.39 | 22.69 |
| Milz | 6.67 ± 0.60 | 7.45 ± 0.72 | 11.34 |
| Muskel | 2.39 ± 0.18 | 1.64 ± 0.01 | 1.13 |
| Darm | 3.08 ± 0.36 | 3.23 ± 0.26 | 4.63 |
| Haut | 5.17 ± 0.68 | 6.67 ± 1.04 | 5.26 |
| Lunge | 13.99 ± 0.61 | 8.46 ± 1.35 | 4.69 |
| Ht29-Tumor | 2.57 ± 0.31 | 3.31 ± 0.27 | 2.84 |
| WiDr-Tumor | 2.90 ± 0.21 | 4.12 ± 0.32 | 3.93 |
| Restkörper | 3.65 ± 0.18 | 3.88 ± 0.97 | 2.48 |

Während im Lebergewebe von 10 % der applizierten Dosis pro Gramm Gewebefrischgewicht (0.5 h p.i.) auf rund 30 % der Dosis pro Gramm Gewebefrischgewicht innerhalb von 24 h p.i. ansteigt, sinken die Gewebekonzentrationen in allen anderen Geweben innerhalb von 24 h ab oder bleiben zumindest konstant.Die Leber weist damit 24 h p.i. die absolut höchste Gewebekonzentration aller untersuchten Gewebe auf.

**Beispiel 26**

9 Nacktmäusen (Balb/c nu/nu) mit subcutan implantierten Coloncarcinomen (HT 29, WiDr) wurden je 0.1 mmol/kg des Mesoporphyrinderivats Beispiel 16f (10 mmol/l gelöst in Aqua bidest., pH 7.2, 37°C) i.v. appliziert. Nach 0.5 h und 3 h wurden die Tiere getötet und präpariert. Die Gewebekonzentrationen wurden nach entsprechender Aufarbeitung ($HNO_3$ konz.) mittels ICP-AES bestimmt und auf % der applizierten Dosis pro g Gewebefrischgewicht berechnet.

Tabelle 15:

| % der applizierten Dosis pro Gramm Gewebefrischgewicht (% d. Dosis/g Organ) | | |
|---|---|---|
| | über 0.5 Std. MW ± S | über 3 Std. MW ± S |
| Blut | 30,68 ± 0,85 | 11,63 ± 4,46 |
| Leber | 20,91 ± 9,96 | 27,93 ± *2,57* |
| Nieren | 8,40 ± 1,58 | 9,72 ± 1,80 |
| Milz | 11,31 ± 1,51 | 15,50 ± 2,96 |
| Muskel | 3,53 ± 0,88 | 3,31 ± 2,90 |
| Darm | 2,06 ± 0,67 | 2,75 ± 0,30 |
| Haut | 4,17 ± 1,35 | 7,12 ± 1,45 |
| Lunge | 21,54 ± 0,94 | 15,28 ± 2,68 |
| Ht29-Tumor | 4,47 ± 2,06 | 3,16 ± 0,95 |
| WiDr-Tumor | 3,24 ± 1,23 | 6,30 ± 2,22 |
| Restkörper | 3,69 ± 0,95 | 3,91 ± 0,43 |

Die Leber zeigte rund 21 bzw. 28 % der applizierten Dosis pro Gramm Gewebefrischgewicht 0.5 bzw. 3 h p. i. die stärkste Anreichung aller untersuchten Gewebe.

**Beispiel 27**

9 Nacktmäusen (Balb/c nu/nu) mit subcutan implantierten Coloncarcinomen (HT 29, WiDr) wurden je 0.1 mmol/kg des Mesoporphyrinderivats Beisp. 18d (10 mmol/l gelöst in Aqua bidest., pH 7.2, 37°C) i.v. appliziert. Nach 0.5 h und 3 h wurden die Tiere getötet und präpariert. Die Gewebekonzentrationen wurden nach entsprechender Aufarbeitung ($HNO_3$ konz.) mittels ICP-AES bestimmt und auf % der applizierten Dosis pro Gramm Gewebefrischgewicht berechnet.

Tabelle 16:

| % der appliziertenDosis pro Gramm Gewebefrischgewicht | | |
|---|---|---|
| | über 0.5 Std. MW ± S | über 3 Std. MW ± S |
| Blut | 24,44 ± 2,32 | 16,95 ± 1,53 |
| Leber | 13,28 ± 2,90 | 16,89 ± 1,15 |
| Nieren | 11,74 ± 2,38 | 20,41 ± 1,88 |
| Milz | 7,52 ± 0,40 | 10,82 ± 0,87 |
| Muskel | 0,89 ± 0,17 | 1,04 ± 0,06 |

Tabelle 16: (fortgesetzt)

| % der appliziertenDosis pro Gramm Gewebefrischgewicht | | |
|---|---|---|
| | über 0.5 Std. MW ± S | über 3 Std. MW ± S |
| Darm | 4,93 ± 0,90 | 2,31 ± 0,28 |
| Haut | 3,71 ± 0,52 | 4,46 ± 0,53 |
| Lunge | 17,70 ± 2,50 | 17,56 ± 3,34 |
| Ht29-Tumor | 1,48 ± 0,28 | 3,53 ± 0,52 |
| WiDr-Tumor | 1,94 ± 0,78 | 4,50 ± 0,98 |
| Restkörper | 2,38 ± 0,27 | 2,43 ± 0,13 |

Mit rund 14 bzw. 17 % der applizierten Dosis pro Gramm Gewebefrischgewicht 0.5 bzw. 3 h p.i. zeigt die untersuchte Substanz eine Anreichung im Lebergewebe. In den anderen untersuchten Geweben, mit Ausnahme der Niere, liegen die Gewebekonzentrationen zum Teil erheblich niedriger (Faktor 5 bis 10).

**Beispiel 28**

9 Nacktmäusen (Balb/c nu/nu) mit subcutan implantierten Coloncarcinomen (HT 29, WiDr) wurden je 0.1 mmol/kg des Mesoporphyrinderivats Beisp. 19e (10 mmol/l gelöst in Aqua bidest., pH 7.2, 37°C) i.v. appliziert. Nach 0.5 h, 3 h und 24 h wurden die Tiere getötet und präpariert. Die Gewebekonzentrationen wurden nach entsprechender Aufarbeitung ($HNO_3$ konz.) mittels ICP-AES bestimmt und auf % der applizierten Dosis pro Gramm Gewebefrischgewicht berechnet.

Tabelle 17:

| % der applizierten Dosis pro Gramm Gewebefrischgewicht | | | |
|---|---|---|---|
| | über 0.5 Std. MW ± S | über 3 Std. MW ± S | über 24 Std. MW ± S |
| Blut | 11,68 ± 2,18 | 4,13 ± 3,62 | 0,06 ± 0,01 |
| Leber | 15,07 ± 1,35 | 13,75 ± 1,54 | 10,43 ± 1,56 |
| Nieren | 26.52 ± 9,65 | 9,98 ± 0,50 | 7,87 ± 0,54 |
| Milz | 7,38 ± 2,40 | 4,41 ± 1,41 | 4,00 ± 1,71 |
| Muskel | 1,79 ± 0,34 | 0,35 ± 0,09 | 0,17 ± 0,07 |
| Darm | 2,56 ± 0,50 | 5,43 ± 1,08 | 0,61 ± 0,43 |
| Haut | 7,13 ± 1,95 | 2,68 ± 0,39 | 1,26 ± 0,12 |
| Lunge | 10,14 ± 1,53 | 2,51 ± 0,36 | 0,69 ± 0,13 |
| Ht29-Tumor | 3,86 ± 1,06 | 2,22 ± 0,31 | 0,99 ± 0,33 |
| WiDr-Tumor | 6,15 ± 2,27 | 5,45 ± 6,63 | 1,95 ± 0,54 |
| Restkörper | 3,37 ± 0,51 | 1.18 ± 3,06 | 0,62 ± 0,27 |

Außer in der Leber sinken die Gewebekonzentration innerhalb von 24 h rasch ab (Faktor 5 - 20 gegenüber 0.5 h p.i.). Mit rund 14 bzw 10 % der applizierten Dosis pro Gramm Gewebefrischgewicht weist die Leber 3 h bzw. 24 h p.i. die absolut höchste Gewebekonzentrationen aller untersuchten Geweben auf.

**Beispiel 29**

An einem Siemens Magneton® (1,5 Tesla; 64 MHz) wurde die Signalintensität in verschiedenen Geweben eines tumortragenden Kaninchen [Hasen-Kaninchen, Wulf, weibl. ≈ 4 kg KGW (n=3), Tumor VX-2-Carcinom, ca. 3 Wochen vor Versuchsbeginn, intramuskulär implantiert] nach der Gabe von 0,0025 mmol/kg KGW i.v. (entspricht 0,05

mmol/kg Gadolinium) der erfindungsgemäßen Verbindung hergestellt nach Beispiel 1c untersucht. Die Messung erfolgte mit folgenden Aufnahmeparametern:

$T_1$-betonte Spin-Echo-Technik: $T_R$: 350 ms, $T_E$: 15 ms, Schichtorientierung: coronar, Schichtdicke 3 mm, Number of Averages: 4 pro Schicht, Field of View: 150 mm. Matrix: $256^2$, 4-6 Schichten 1 Echo pro Aufnahmesequenz.

Die Applikation führte zu einem guten langanhaltenden Enhancement und zwar sowohl im implantierten Tumor als auch in der Mehrzahl der Lymphknoten auf der tumortragenden Seite. Aufgrund des ausgeprägten Enhancements in diesen Lymphknoten bzw. Lymphknotenarealen, nach Applikation der erfindungsgemäßen Verbindung konnte daher ein Metastasen-Befall der Lymphknoten diagnostiziert werden. Die histologische Untersuchung der Lymphknoten bestätigte die Ergebnisse der MR-tomographischen Untersuchung.

Die relativen Signalintensitäten in verschiedenen Geweben sind in Figur 4 dargestellt. Die Ausgangssignalintensität (= $SI_{praekontrast}$) in den verschiedenen Geweben wurde jeweils gleich 1 gesetzt.

## Patentansprüche

**1.** Porphyrin-Komplex-Verbindungen bestehend aus einem Liganden der allgemeinen Formel I

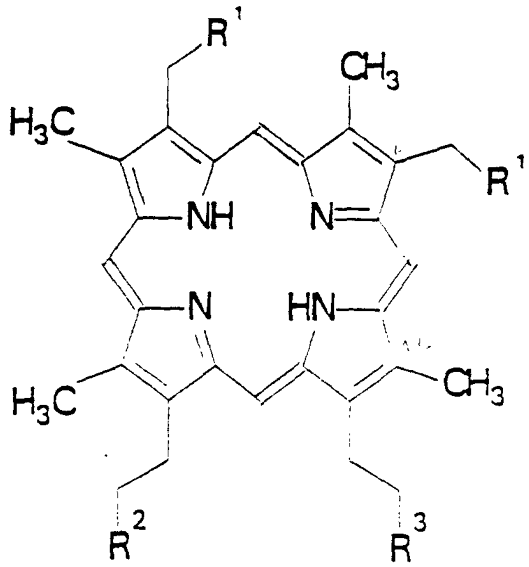

(I)

sowie mindestens einem Ion eines Elementes der Ordnungszahl 21-32, 37-39, 42-51 oder 57-83, worin

$R^1$ für ein Wasserstoffatom, für einen geradkettigen $C_1$-$C_6$-Alkylrest, einen $C_7$-$C_{12}$-Aralkylrest oder für eine Gruppe OR' worin
R' ein Wasserstoffatom oder ein $C_1$-$C_3$-Alkylrest ist, steht,

$R^2$ für $R^3$, eine Gruppe -CO-Z oder eine Gruppe $-(NH)_o-(A)_q-NH-D$ steht, worin

Z eine Gruppe -OL ist, mit L in der Bedeutung eines anorganischen oder organischen Kations oder eines $C_1$-$C_4$-Alkylrestes ist,

A eine $(CH_2)_{\overline{2-4}}$, Phenylenoxy- oder eine im Alkylteil durch ein Sauerstoffatom unterbrochene $C_1$-$C_{12}$-Alkylen- oder $C_7$-$C_{12}$ Aralkylengruppe bedeutet,

o und q unabhängig voneinander die Ziffern 0 oder 1 bedeuten und

D ein Wasserstoffatom oder eine Gruppe $-CO-A-(COOL)_o-(H)_m$ bedeutet, mit m gleich 0 oder 1 und unter der Maßgabe, daß die Summe aus m und o gleich 1 ist,

$R^3$ für eine Gruppe $-(C=M)(NR^4)_o-(A)_q-(NR^5)-K$ steht,
worin M für ein Sauerstoffatom oder für zwei Wasserstoffatome steht,

$R^4$ eine Gruppe $-(A)_q-H$ bedeutet und

K einen Komplexbildner der allgemeinen Formel (IIa) oder (IIb) bedeutet und wobei
$R^5$ für den Fall, daß K ein Komplexbildner der Formel (IIa) ist, die gleiche Bedeutung wie $R^4$ hat und
$R^5$ für den Fall, daß K ein Komplexbildner der Formel (IIb) ist, die gleiche Bedeutung wie D hat, mit der Maßgabe, daß eine direkte Sauerstoff-Stickstoff Bindung nicht zugelassen ist und
K für einen Komplexbildner der allgemeinen Formel (IIa) oder (IIb)

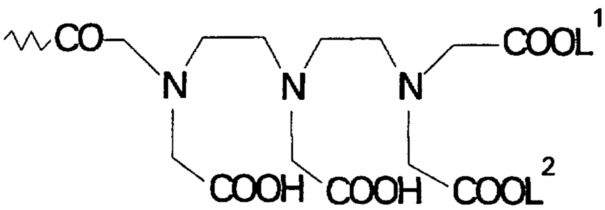

(IIa)

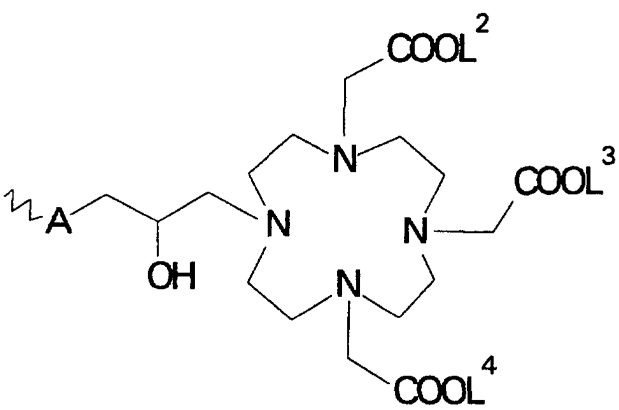

(IIb)

steht, mit $L^1$ in der Bedeutung eines $C_1$-$C_6$-Alkyl-Restes oder eines Lithium-, Kalium- oder Natriumions oder eines Ammoniumsalzes des Ethanolamins, Diethanolamins, Morpholins, Glucamins, N, N-Dimethylglucamins oder Meglumins und worin $L^2$, $L^3$ und $L^4$ unabhängig voneinander die Bedeutung von $L^1$ oder eines Wasserstoffatoms haben, unter der Maßgabe, daß im Komplexbildner mindestens zwei freie Carbonsäuregruppen vorliegen,

sowie zum Ausgleich gegebenenfalls vorhandener Ladungen im Metalloporphyrin weitere Anionen.

2. Komplex-Verbindungen gemäß Anspruch 1, **dadurch gekennzeichnet, daß** das Porphyrin-System ein Metallion enthält.

3. Komplex-Verbindungen gemäß Anspruch 1, **dadurch gekennzeichnet, daß** das Porphyrin-System kein Metallion enthält.

4. Verbindung nach Anspruch 1, nämlich das Dinatriumsalz des Digadoliniumkomplexes des N,N'-Bis[9-carboxylato-2,5,8-tris(carboxylatomethyl)-2,5,8-triazanonyl-carbamoyl]-mesoporphyrin-IX-13,17-diamids.

5. Verbindung nach Anspruch 1, nämlich das Dinatriumsalz des Digadoliniumkomplexes des Mangan(III)-{N,N'-Bis [11-carboxylato-2-oxo-4,7-bis(carboxylatomethyl)-10-(ethoxycarbonylmethyl)-1,4,7,10-tetraazaundecyl]-3,8-bis (1-propyl)-deuteroporphyrin-IX-13,17-diamid}-acetats.

6. Verbindung nach Anspruch 1, nämlich der Digadoliniumkomplex des Mangan(III)-{N,N'-Bis[11-carboxylato-2-oxo-4,7-bis(carboxylatomethyl)-10-(ethoxycarbonylmethyl)-1,4,7,10-tetraazaundecyl] -3,8-bis(1-propyl)-deuteroporphyrin-IX-13,17-diamid} -acetats.

7. Pharmazeutische Mittel enthaltend mindestens eine Komplex-Verbindung nach Anspruch 1, gegebenenfalls mit in der Galenik üblichen Zusätzen.

**8.** Verwendung von mindestens einem Metallkomplex gemäß Anspruch 1 für die Herstellung von Mitteln für die NMR- oder Radiodiagnostik sowie für die Radiotherapie.

**9.** Verfahren zur Herstellung von Porphyrin-Komplex-Verbindungen bestehend aus einem Liganden der allgemeinen Formel I

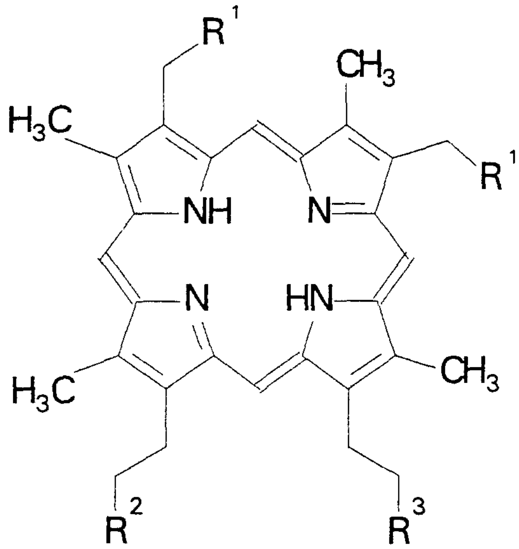

(I)

sowie mindestens einem Ion eines Elementes der Ordnungszahl 21-32, 37-39, 42-51 oder 57-83, worin

$R^1$ für ein Wasserstoffatom, für einen geradkettigen $C_1$-$C_6$-Alkylrest, einen $C_7$-$C_{12}$-Aralkylrest oder für eine Gruppe OR' worin

R' ein Wasserstoffatom oder ein $C_1$-$C_3$-Alkylrest ist, steht.

$R^2$ für $R^3$, eine Gruppe -CO-Z oder eine Gruppe $-(NH)_o-(A)_q-NH-D$ steht, worin

Z eine Gruppe -OL ist, mit L in der Bedeutung eines anorganischen oder organischen Kations oder eines $C_1$-$C_4$-Alkylrestes ist,

A eine $(CH_2)_{\overline{2-4}}$, Phenylenoxy- oder eine im Alkylteil durch ein Sauerstoffatom unterbrochene $C_1$-$C_{12}$-Alkylen- oder $C_7$-$C_{12}$ Aralkylengruppe bedeutet,

o und q unabhängig voneinander die Ziffern 0 oder 1 bedeutet und

D ein Wasserstoffatom oder eine Gruppe $-CO-A-(COOL)_o-(H)_m$ bedeutet, mit m gleich 0 oder 1 und unter der Maßgabe, daß die Summe aus m und o gleich 1 ist,

$R^3$ für eine Gruppe $-(C{=}M)(NR^4)_o-(A)_q-(NR^5)-K$ steht,
worin M für ein Sauerstoffatom oder für zwei Wasserstoffatome steht,
$R^4$ eine Gruppe -(A)q-H bedeutet und
K einen Komplexbildner der allgemeinen Formel (IIa) oder (IIb) bedeutet und wobei $R^5$, für den Fall, daß K ein Komplexbildner der Formel (IIa) ist die gleiche Bedeutung wie $R^4$ hat und $R^5$ für den Fall, daß K ein Komplexbildner der Formel (IIb) ist, die gleiche Bedeutung wie D hat,
mit der Maßgabe, daß eine direkte Sauerstoff-Stickstoff Bindung nicht zugelassen ist,
und K für einen Komplexbildner der allgemeinen Formel (IIa) oder (IIb)

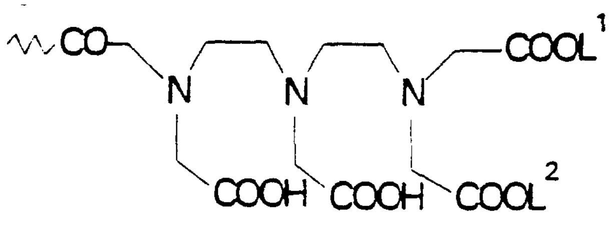

(IIa)

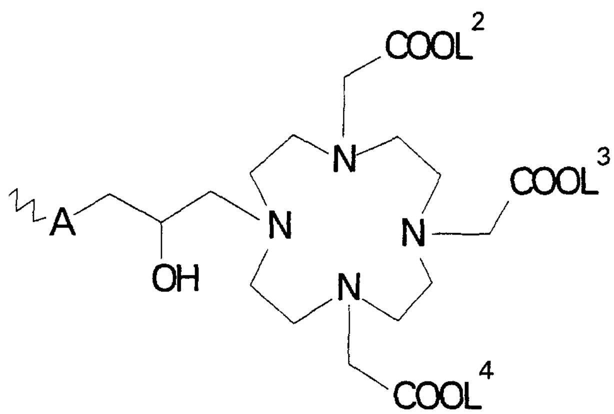

(IIb)

steht, mit $L^1$ in der Bedeutung eines $C_1$-$C_6$-Alkyl-Restes oder eines Lithium-, Kalium- oder Natriumions oder eines Ammoniumsalzes des Ethanolamins, Diethanolamins, Morpholins, Glucamins, N, N-Dimethylglucamins oder Meglumins und worin $L^2$, $L^3$ und $L^4$ unabhängig voneinander die Bedeutung von $L^1$ oder eines Wasserstoffatoms haben, unter der Maßgabe, daß im Komplexbildner mindestens zwei freie Carbonsäuregruppen vorliegen,

sowie gegebenenfalls weitere Anionen zum Ausgleich der Ladungen im Metalloporphyrin, **dadurch gekennzeichnet, daß** man durch

a) Reduktion eines Porphyrins der allgemeinen Formel (IIIa)

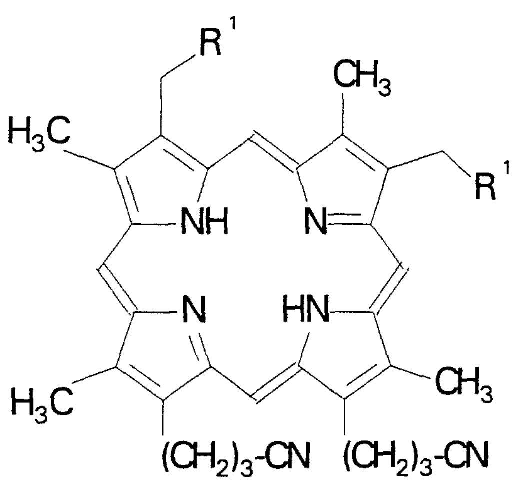

(IIIa)

oder durch
b) Umsetzung eines Porphyrins der allgemeinen Formel (IIIb)

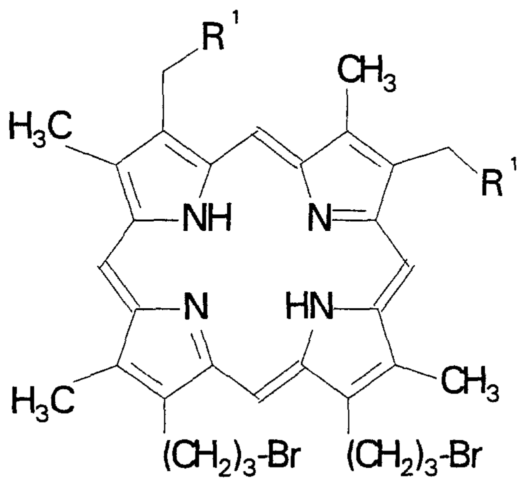

(IIIb)

mit Aminophenol, oder durch

c) Umsetzung eines Porphyrins der allgemeinen Formel (IIIc)

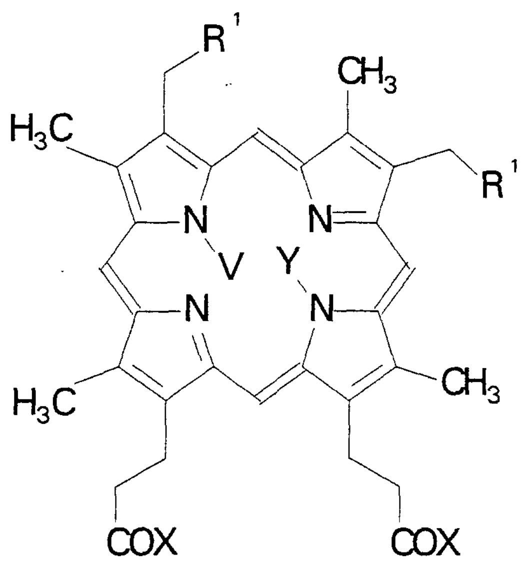

(IIIc)

worin

| | |
|---|---|
| $R^1$ | die angegebene Bedeutung hat, |
| V und Y | jeweils für ein Wasserstoffatom oder gemeinsam für ein mehrwertiges Metall-Ion eines Elementes der Ordnungszahl 21-32, 37-39, 42-51 oder 57-83 , vorzugsweise für ein Zink-(II)- oder ein Mangan-(III)-ion stehen und |
| X | für ein Halogenatom, eine Gruppe -OR' oder für eine Gruppe -O-COOR' mit R' in der genannten Bedeutung steht, |

mit Verbindungen $H\text{-}NR^4\text{-}(A)_q\text{-}NR^4\text{-}H$, worin

A, $R^4$ und q die angegebene Bedeutung haben,

gewünschtenfalls anschließende Reduktion der Carbonylgruppen oder Hofmann schen Abbau des Amids zunächst ein Porphyrin der allgemeinen Formel IV

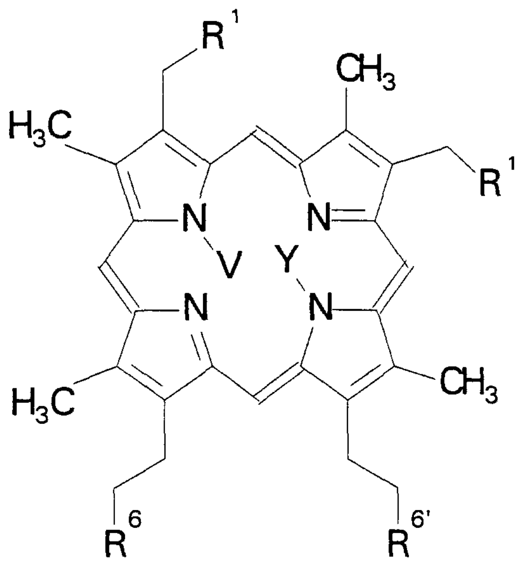

(IV)

erhält, worin

$R^6$ für eine Gruppe $-(C=M)-(NR^4)_o-(A)_q-NR^4-H$ steht, worin

M, $R^4$, A, o und q die angegebene Bedeutung haben und worin

$R^{6'}$ die gleiche Bedeutung wie $R^6$ hat oder für eine Gruppe -OR' steht, das anschließend

a) mit einem Komplexbildner der allgemeinen Formel V,

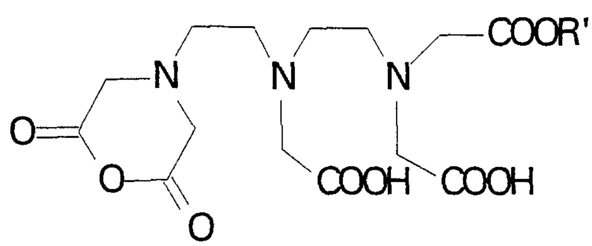

(V)

worin

R' die angegebene Bedeutung hat, umgesetzt wird und gewünschtenfalls die vorhandenen Estergruppen verseift werden oder

b) mit einer Verbindung der Formel VI

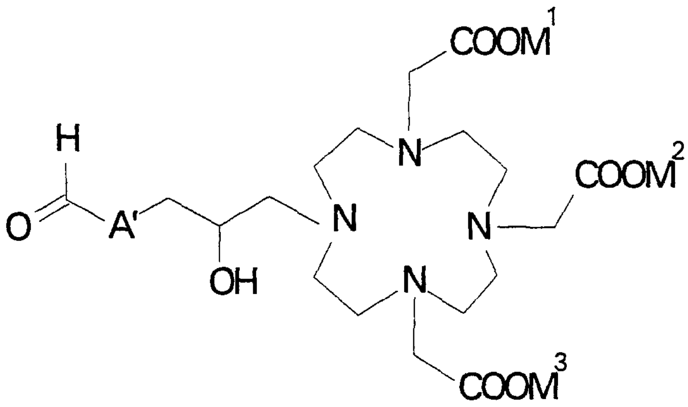

(VI)

worin

A' eine um ein Kohlenstoffatom verkürzte Gruppe A ist und $M^1$, $M^2$ und $M^3$ unabhängig voneinander für R' oder ein Metallionenäquivalent der Elemente der Ordnungszahlen 21-32, 37-39, 42-51 oder 57-83 stehen,

unter den Bedingungen einer reduktiven Aminierung umgesetzt wird, danach das so erhaltene Produkt - gegebenenfalls nach vollständiger oder teilweiser Abspaltung der Estergruppen - mit (einem) Metalloxid(en) oder Metallsalz(en) der Elemente der oben genannten Ordnungszahlen umsetzt, anschließend mit einem Reagenz Nucleofug-D' acyliert, (wobei die letztgenannten Schritte vertauscht werden können) worin D' die unter D angegebene Bedeutung hat, unter der Maßgabe, daß D' nicht für Wasserstoff steht und abschließend gewünschtenfalls eventuell noch vorhandene acide Wasserstoffe durch Kationen anorganischer oder organischer Basen vollständig oder teilweise substituiert.

**10.** Verfahren zur Herstellung der pharmazeutischen Mittel gemäß Anspruch 7, **dadurch gekennzeichnet, daß** man die in Wasser oder physiologischer Salzlösung gelöste oder suspendierte Komplexverbindung, gegebenenfalls mit den in der Galenik üblichen Zusätzen, in eine für die enterale oder parenterale Applikation geeignete Form bringt.

## Claims

**1.** Porphyrin complex compounds consisting of a ligand of the general formula I

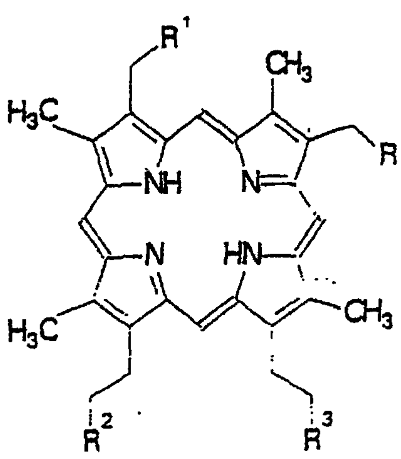

(I)

and at least one ion of an element of atomic number 21-32, 37-39, 42-51 or 57-83, in which

$R^1$ is a hydrogen atom, a straight-chain $C_1$-$C_6$-alkyl radical, a $C_7$-$C_{12}$-aralkyl radical or a group OR' in which R' is a hydrogen atom or a $C_1$-$C_3$-alkyl radical,

$R^2$ is $R^3$, a group -CO-Z or a group $-(NH)_o-(A)_q-NH-D$, in which

- Z is a group -OL with L meaning an inorganic or organic cation or a $C_1$-$C_4$-alkyl radical,
- A is $(CH)_2)_{2-4}$ group, phenyleneoxy group or a $C_1$-$C_{12}$-alkylene or $C_7$-$C_{12}$-aralkylene group which is interrupted by an oxygen atom in the alkyl moiety,
- o and q are, independently of one another, the numbers 0 or 1, and
- D is a hydrogen atom or a group $-CO-A-(COOL)_o-(H)_m$ with m equal to 0 or 1 and with the proviso that the total of m and o equals 1,

$R^3$ is a group $-(C{=}M)(NR^4)_o-(A)_q-(NR^5)-K$, in which M is an oxygen atom or two hydrogen atoms,

$R^4$ is a group $-(A)_q-H$, and

K is a complexing agent of the general formula (IIa) or (IIb), and where

$R^5$ in the case where K is a complexing agent of the formula (IIa) has the same meaning as $R^4$ and

$R^5$ in the case where K is a complexing agent of the formula (IIb) has the same meaning as D,

with the proviso that a direct oxygen-nitrogen bond is not permitted, and

K is a complexing agent of the general formula (IIa) or (IIb)

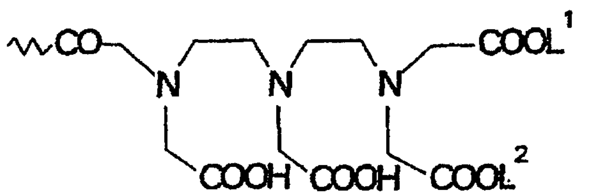

(IIa)

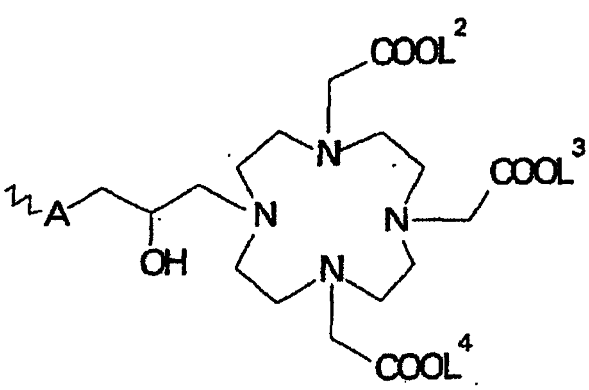

(IIb)

with $L^1$ meaning a $C_1$-$C_6$-alkyl radical or a lithium, potassium or sodium ion or an ammonium salt of ethanolamine, diethanolamine, morpholine, glucamine, N,N-dimethylglucamine or meglumine and in which $L^2$, $L^3$ and $L^4$ have, independently of one another, the meaning of $L^1$ or of a hydrogen atom, with the proviso that at least two free carboxyl groups are present in the complexing agent,

and further anions to balance where appropriate charges present in the metalloporphyrin.

2. Complex compounds according to Claim 1, **characterized in that** the porphyrin system contains a metal ion.

3. Complex compounds according to Claim 1, **characterized in that** the porphyrin system contains no metal ion.

4. Compound according to Claim 1, namely the disodium salt of the digadolinium complex of N,N-bis[9-carboxylato-2,5,8-tris(carboxylatomethyl)-2,5,8-triazanonylcarbamoyl]mesoporphyrin-IX-13,17-diamide.

**5.** Compound according to Claim 1, namely the disodium salt of the digadolinium complex of manganese (III) N,N'-bis[11-carboxylato-2-oxo-4,7-bis(carboxylato-methyl)-10-(ethoxycarbonylmethyl)-1,4,7,10-tetraaza-undecyl]-3,8-bis(1-propyl)deuteroporphyrin-IX-13,17-diamide acetate.

**6.** Compound according to Claim 1, namely the digadolinium complex of manganese(III) N/N'-bis[11-carboxylato-2-oxo-4,7-bis(carboxylatomethyl)-10-(ethoxycarbonylmethyl)-1,4,7,10-tetraazaundecyl]-3,8-bis(1-propyl)deuteroporphyrin-IX-13,17-diamide acetate.

**7.** Pharmaceutical compositions containing at least one complex compound according to Claim 1, where appropriate with additions customary in pharmaceutical technology.

**8.** Use of at least one metal complex according to Claim 1 for the production of compositions for NMR diagnosis or radionuclide diagnosis and for radiotherapy.

**9.** Process for the preparation of porphyrin complex compounds consisting of a ligand of the general formula I

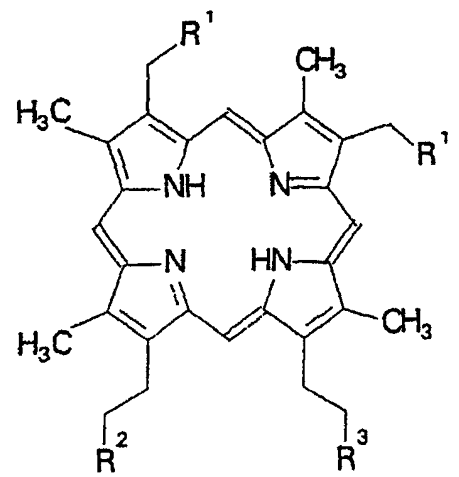

(I)

and at least one ion of an element of atomic number 21-32, 37-39, 42-51 or 57-83, in which

$R^1$ is a hydrogen atom, a straight-chain $C_1$-$C_6$-alkyl radical, a C7-$C_{12}$-aralkyl radical or a group OR' in which R' is a hydrogen atom or a $C_1$-$C_3$-alkyl radical,

$R^2$ is $R^3$, a group -CO-Z or a group $-(NH)_o-(A)_q-NH-D$, in which

- Z is a group -OL with L meaning an inorganic or organic cation or a $C_1$-$C_4$-alkyl radical,
- A is $(CH)_2)_{2-4}$ group, phenyleneoxy group or a $C_1$-$C_{12}$-alkylene or $C_7$-$C_{12}$-aralkylene group which is interrupted by an oxygen atom in the alkyl moiety,
- o and q are, independently of one another, the numbers 0 or 1, and
- D is a hydrogen atom or a group -CO-A- $(COOL)_o-(H)_m$ with m equal to 0 or 1 and with the proviso that the total of m and o equals 1,

$R^3$ is a group - $(C=M)(NR^4)_o-(A)_q-(NR^5)$-K,
in which
M is an oxygen atom or two hydrogen atoms,
$R^4$ is a group $-(A)_q-H$, and
K is a complexing agent of the general formula (IIa) or (IIb), and where $R^5$ in the case where K is a complexing agent of the formula (IIa) has the same meaning as $R^4$ and $R^5$ in the case where K is a complexing agent of the formula (IIb) has the same meaning as D,
with the proviso that a direct oxygen-nitrogen bond is not permitted, and
K is a complexing agent of the general formula (IIa) or (IIb)

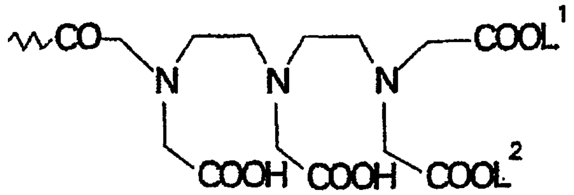

(IIa)

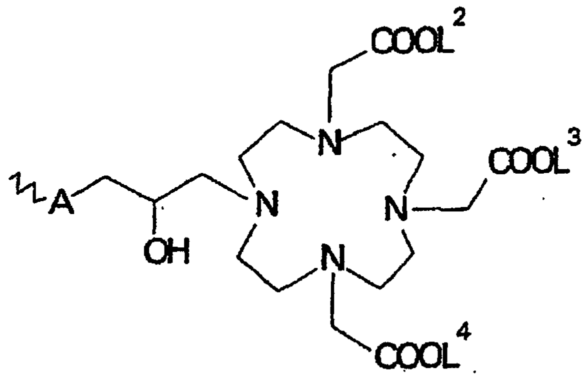

(IIb)

with $L^1$ meaning a $C_1$-$C_6$-alkyl radical or a lithium, potassium or sodium ion or an ammonium salt of ethanolamine, diethanolamine, morpholine, glucamine, N,N-dimethylglucamine or meglumine and in which

$L^2$, $L^3$ and $L^4$ have, independently of one another, the meaning of $L^1$ or of a hydrogen atom, with the proviso that at least two free carboxyl groups are present in the complexing agent,

and further anions to balance where appropriate charges present in the metalloporphyrin, **characterized in that**

a) a porphyrin of the general formula (IIIa)

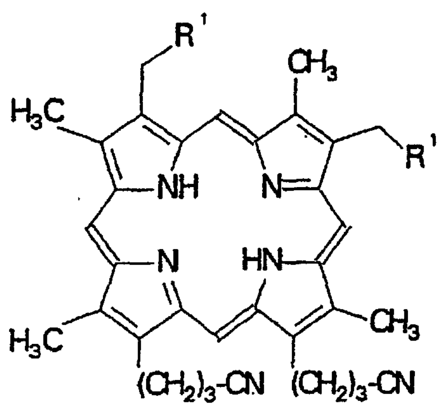

(IIIa)

is reduced or

b) a porphyrin of the general formula (IIIb)

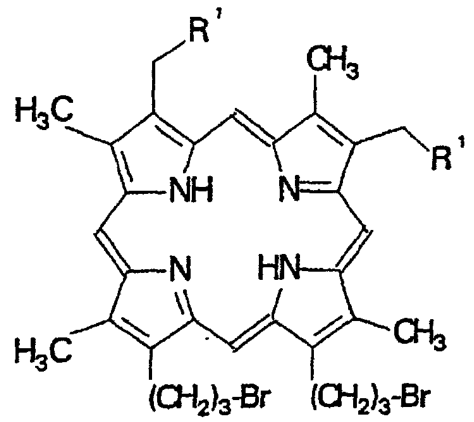

(IIIb)

is reacted with aminophenol or

c) a porphyrin of the general formula (IIIc)

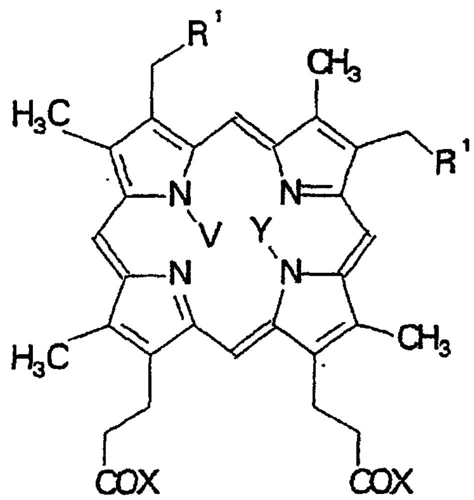

(IIIc)

in which

| | |
|---|---|
| $R^1$ | has the stated meaning, |
| V and Y | are each a hydrogen atom or together are a multiply charged metal ion of an element of atomic number 21-32, 37-39, 41-51 or 57-83, preferably a zinc (II) or a manganese(III) ion, and |
| X | is a halogen atom, a group -OR' or a group -O-COOR' with R' having the stated meaning, |

is reacted with compounds $H-NR^4-(A)_q-NR^4-H$ in which

A, $R^4$ and q have the stated meaning,

if required the carbonyl groups are subsequently reduced or the amide is subjected to Hofmann degradation to result initially in a porphyrin of the general formula IV

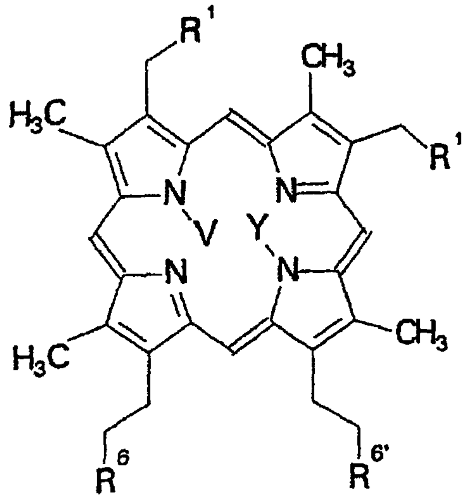

(IV)

in which

$R^6$ is a group - (C=M) - $(NR^4)_o$ - $(A)_q$-$NR^4$-H in which M, $R^4$, A, o and q have the stated meaning, and in which $R^{6'}$ has the same meaning as $R^6$ or is a group -OR', which is subsequently

a) reacted with a complexing agent of the general formula V

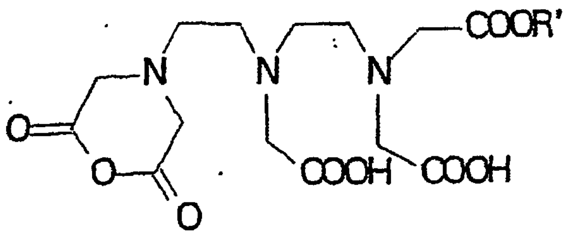

(V)

in which

R' has the stated meaning, and if required the ester groups which are present are hydrolysed, or

b) reacted with a compound of the formula VI

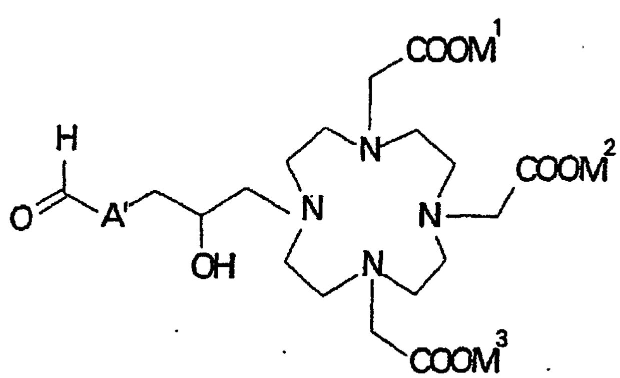

(VI)

in which

A' is a group A which is shortened by one carbon atom, and $M^1$, $M^2$ and $M^3$ are, independently of one

another, R' or a metal ion equivalent of elements of atomic numbers 21-32, 37-39, 42-51 or 57-83,

under the conditions of a reductive amination, then the product obtained in this way - where appropriate after complete or partial elimination of the ester groups-is reacted with (a) metal oxide(s) or metal salt(s) of the elements of the abovementioned atomic numbers, subsequently acylated with a reagent nucleofuge-D' (it being possible to interchange the latter steps), in which D' has the meanings stated for D, with the proviso that D' is not hydrogen, and finally if required possibly also completely or partially replacing acidic hydrogens which are present by cations of inorganic or organic bases.

**10.** Process for the production of the pharmaceutical compositions according to Claim 7, **characterized in that** the complex compound dissolved or suspended in water or physiological saline is converted, where appropriate with the additions customary in pharmaceutical technology, into a form suitable for enteral or parenteral administration.

## Revendications

**1.** Composés complexes de porphyrine constitués d'un ligand de formule générale I

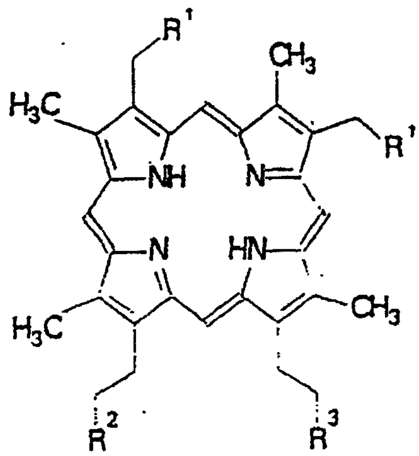

(I)

et d'au moins un ion d'un élément de numéros atomiques 21-32, 37-39, 42-51 ou 57-83, dans laquelle

$R^1$ représente un atome d'hydrogène, un radical alkyle en $C_1$-$C_6$ linéaire, un radical aralkyle en $C_7$-$C_{12}$ ou un groupe OR' dans lequel
R' représente un atome d'hydrogène ou un radical alkyle en $C_1$-$C_3$,

$R^2$ représente $R^3$, un groupe -CO-Z ou un groupe $-(NH)_o-(A)_q-NH-D$, dans lesquels

Z représente un groupe -OL, L représentant un cation inorganique ou organique, ou un radical alkyle en $C_1$-$C_4$,
A représente $(CH_2)_{2-4}$, un groupe phénylène-oxy ou un groupe alkylène en $C_1$-$C_{12}$ ou aralkylène en $C_7$-$C_{12}$ qui est interrompu dans la partie alkyle par un atome d'oxygène,
o et q, indépendamment l'un de l'autre, représentent les nombres 0 ou 1, et
D représente un atome d'hydrogène ou un groupe $-CO-A-(COOL)_o-(H)_m$, m valant 0 ou 1 et à condition que la somme de m et o soit égale à 1,

$R^3$ représente un groupe $-(C=M)\ (NR^4)_o-(A)_q-(NR^5)-K$, dans lequel M représente un atome d'oxygène ou deux atomes d'hydrogène,
$R^4$ représente un groupe $-(A)_q-H$, et
K représente un agent complexant de formules générales (IIa) ou (IIb), et où
$R^5$, si K est un agent complexant de formule (IIa), présente la même signification que $R^4$, et
$R^5$, si K est un agent complexant de formule (IIb), présente la même signification que D,
à condition qu'une liaison directe oxygène-azote ne soit pas permise, et
K représente un agent complexant de formules générales (IIa) ou (IIb)

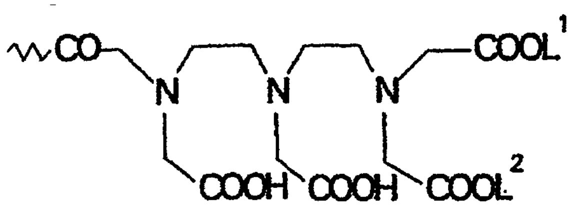

(IIa)

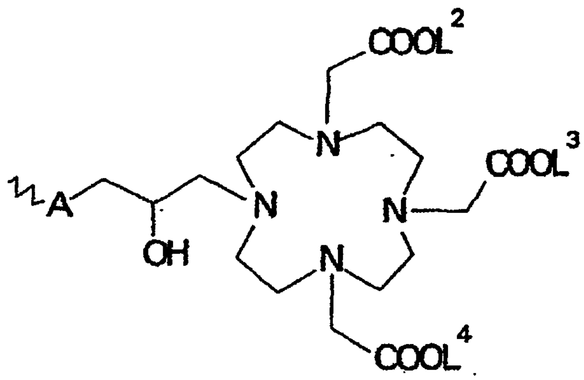

(IIb)

$L^1$ représentant un radical alkyle en $C_1$-$C_6$ ou un ion lithium, potassium ou sodium ou un sel d'ammonium de l'éthanolamine de la diéthanolamine, de la morpholine, de la glucamine, de la N,N-diméthylglucamine ou de la méglumine, et dans lesquelles

$L^2$, $L^3$ et $L^4$ présentent, indépendamment les uns des autres, la signification de $L^1$ ou d'un atome d'hydrogène, à condition qu'au moins deux radicaux carboxy libres soient présents dans l'agent complexant,

et d'autres anions en vue d'équilibrer éventuellement les charges présentes dans la métalloporphyrine.

2. Composés complexes selon la revendication 1, **caractérisés en ce que** le système porphyrinique renferme un ion métallique.

3. Composés complexes selon la revendication 1, **caractérisés en ce que** le système porphyrinique ne renferme pas d'ion métallique.

4. Composé selon la revendication 1, à savoir le sel disodique du complexe de digadolinium du N,N-bis[9-carboxylato-2,5,8-tris(carboxylatométhyl)-2,5,8-triazanonylcarbamoyl]mésoporphyrine-IX-13,17-diamide.

5. Composé selon la revendication 1, à savoir le sel disodique du complexe de digadolinium du manganèse(III)-acétate de {N,N'-bis[11-carboxylato-2-oxo-4,7-bis(carboxylatométhyl)-10-(éthoxycarbonylméthyl)-1,4,7,10-tétra-azaundécyl]-3,8-bis(1-propyl)deutéroporphyrine-IX-13,17-diamide}.

6. Composé selon la revendication 1, à savoir le complexe de digadolinium du manganèse(III)-acétate de {N,N'-bis [11-carboxylato-2-oxo-4,7-bis(carboxylatométhyl)-10-(éthoxycarbonylméthyl)-1,4,7,10-tétra-azaundécyl]-3,8-bis (1-propyl)deutéroporphyrine-IX-13,17-diamide}.

7. Compositions pharmaceutiques comprenant au moins un composé complexe selon la revendication 1, éventuellement avec des additifs habituels en galénique.

8. Utilisation d'au moins un complexe métallique selon la revendication 1 pour la préparation de compositions destinées au diagnostic par RMN et au radiodiagnostic ainsi qu'à la radiothérapie.

9. Procédé de préparation de composés complexes de porphyrine constitués d'un ligand de formule générale I

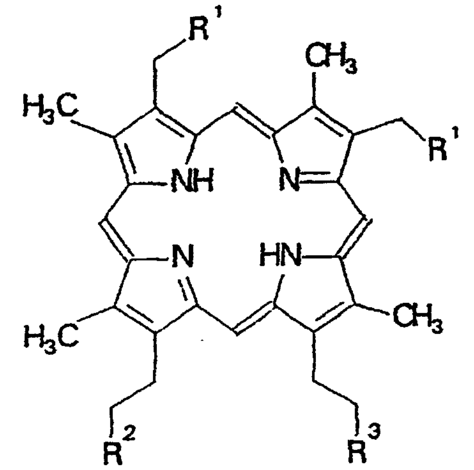

(I)

et d'au moins un ion d'un élément de numéros atomiques 21-32, 37-39, 42-51 ou 57-83, dans laquelle

$R^1$ représente un atome d'hydrogène, un radical alkyle en $C_1$-$C_6$ linéaire, un radical aralkyle en $C_7$-$C_{12}$ ou un groupe OR' dans lequel
R' représente un atome d'hydrogène ou un radical alkyle en $C_1$-$C_3$,

$R^2$ représente $R^3$, un groupe -CO-Z ou un groupe $-(NH)_o-(A)_q-NH-D$, dans lesquels

- Z représente un groupe -OL, L représentant un cation inorganique ou organique, ou un radical alkyle en $C_1$-$C_4$,
- A représente $(CH_2)_{2-4}$, un groupe phénylène-oxy ou un groupe alkylène en $C_1$-$C_{12}$ ou aralkylène en $C_7$-$C_{12}$ qui est interrompu dans la partie alkyle par un atome d'oxygène,
- o et q, indépendamment l'un de l'autre, représentent les nombres 0 ou 1, et
- D représente un atome d'hydrogène ou un groupe $-CO-A-(COOL)_o-(H)_m$, m valant 0 ou 1 et à condition que la somme de m et o soit égale à 1,

$R^3$ représente un groupe $-(C{=}M)\,(NR^4)_o-(A)_q-(NR^5)-K$, dans lequel M représente un atome d'oxygène ou deux atomes d'hydrogène,
$R^4$ représente un groupe $-(A)_q-H$, et
K représente un agent complexant de formules générales (IIa) ou (IIb), et où
$R^5$, si K est un agent complexant de formule (IIa), présente la même signification que $R^4$, et
$R^5$, si K est un agent complexant de formule (IIb), présente la même signification que D,
à condition qu'une liaison directe oxygène-azote ne soit pas permise, et
K représente un agent complexant de formules générales (IIa) ou (IIb)

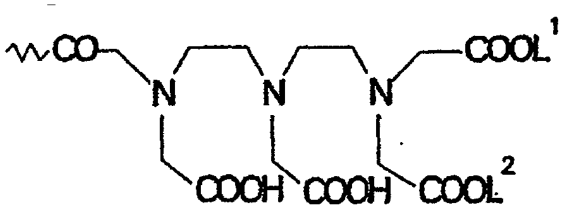

(IIa)

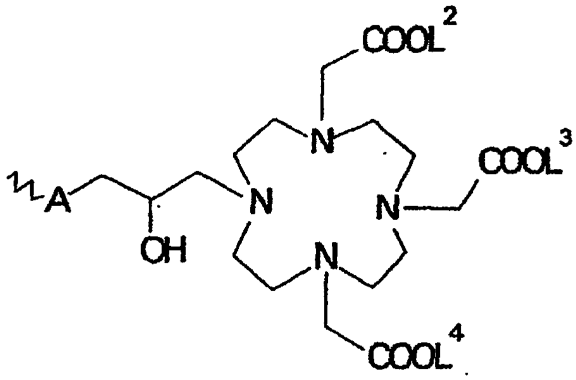

**(IIb)**

$L^1$ représentant un radical alkyle en $C_1$-$C_6$ ou un ion lithium, potassium ou sodium ou un sel d'ammonium de l'éthanolamine, de la diéthanolamine, de la morpholine, de la glucamine, de la N,N-diméthylglucamine ou de la méglumine, et dans lesquelles
$L^2$, $L^3$ et $L^4$ présentent, indépendamment les uns des autres, la signification de $L^1$ ou d'un atome d'hydrogène, à condition qu'au moins deux radicaux carboxy libres soient présents dans l'agent complexant,

et éventuellement d'autres anions en vue d'équilibrer les charges présentes dans la métalloporphyrine, **caractérisé en ce que**

a) par réduction d'une porphyrine de formule générale (IIIa)

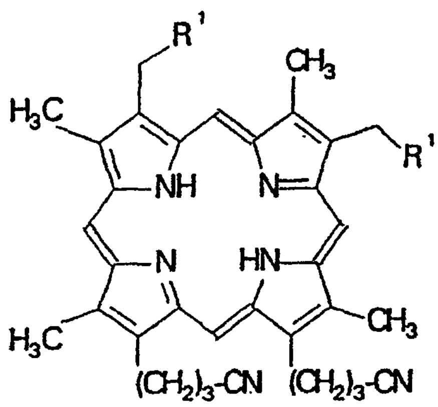

**(IIIa)**

ou par
b) réaction d'une porphyrine de formule générale (IIIb)

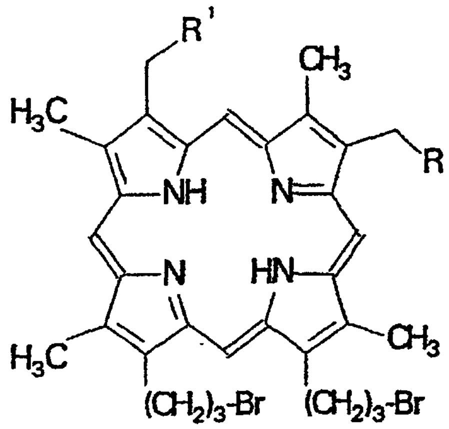

(IIIb)

avec l'aminophénol, ou par

c) réaction d'une porphyrine de formule générale (IIIc)

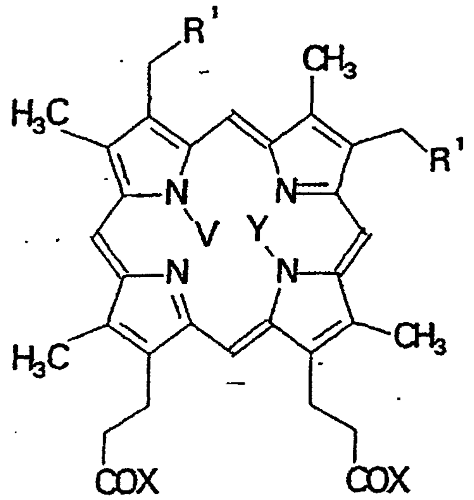

(IIIc)

dans laquelle

$R^1$ présente la signification indiquée,

V et Y représentent chacun un atome d'hydrogène ou représentent, conjointement avec un ion métallique multivalent d'un élément de numéros atomiques 21-32, 37-39, 42-51 ou 57-83, de préférence un ion zinc (II) ou manganèse(III), et

X représente un atome d'halogène, un groupe -OR' ou un groupe -O-COOR', R' présentant la signification indiquée, avec des composés $H-NR^4-(A)_q-NR^4-H$ dans lesquels A, $R^4$ et q présentent la signification indiquée, si on le souhaite, par réduction subséquente des groupes carbonyle ou par dégradation de Hofmann de l'amide, on obtient initialement une porphyrine de formule générale IV

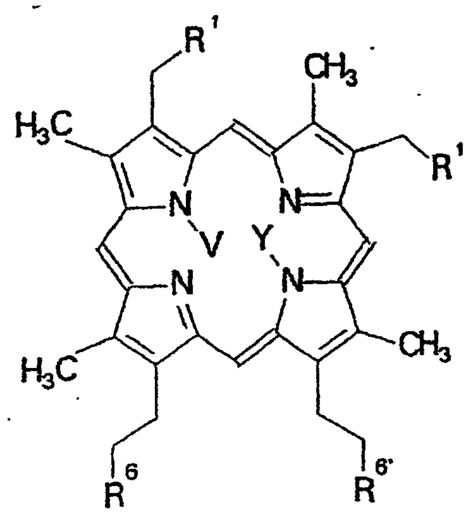

(IV)

dans laquelle
$R^6$ représente un groupe $-(C=M)-(NR^4)_o-(A)_q-NR^4-H$ dans lequel
M, $R^4$, A, o et q présentent la signification indiquée, et dans laquelle
$R^{6'}$ présente la même signification que $R^6$ ou représente un groupe -OR', laquelle est ensuite

a) mise à réagir avec un agent complexant de formule générale V

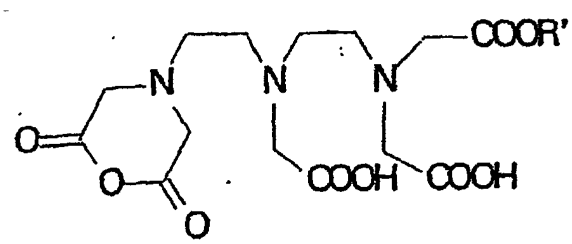

(V)

dans laquelle

R' présente la signification indiquée, et si on le souhaite, les groupes ester présents sont hydrolysés, ou

b) mise à réagir avec un composé de formule VI

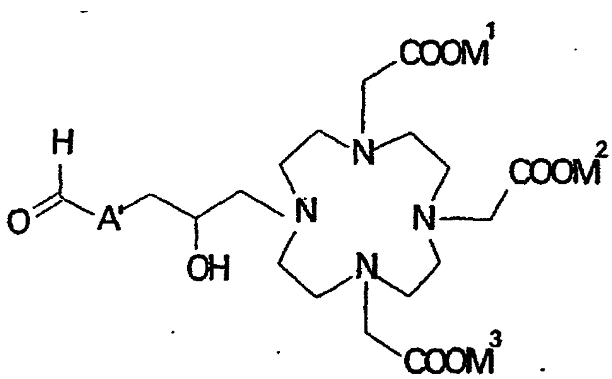

(VI)

dans laquelle

A' représente un groupe A raccourci d'un atome de carbone, et $M^1$, $M^2$ et $M^3$ représentent, indépendamment les uns des autres, R' ou un équivalent d'ion métallique des éléments de numéros atomiques 21-32, 37-39, 42-51 ou 57-83,

dans les conditions d'une amination réductive, puis le produit ainsi obtenu - éventuellement après élimination totale ou partielle des groupes ester - est mis à réagir avec un (des) oxyde(s) métallique(s) ou sel(s) métallique(s) des éléments des numéros atomiques susmentionnés, ensuite acylé avec un réactif nucléofuge-D' (ces dernières étapes pouvant être permutées), dans lequel D' présente la signification indiquée pour D, à condition que D' ne représente pas un hydrogène, et finalement, si on le souhaite, les hydrogènes acides encore présents sont éventuellement remplacés, entièrement ou en partie, pas des cations de bases inorganiques ou organiques.

**10.** Procédé de préparation des compositions pharmaceutiques selon la revendication 7, **caractérisé** en ce le composé complexe dissous ou mis en suspension dans de l'eau ou une solution salée physiologique est transformé, éventuellement avec les additifs habituels en galénique, en une forme appropriée pour l'administration par voie entérale ou parentérale.

Fig. 1

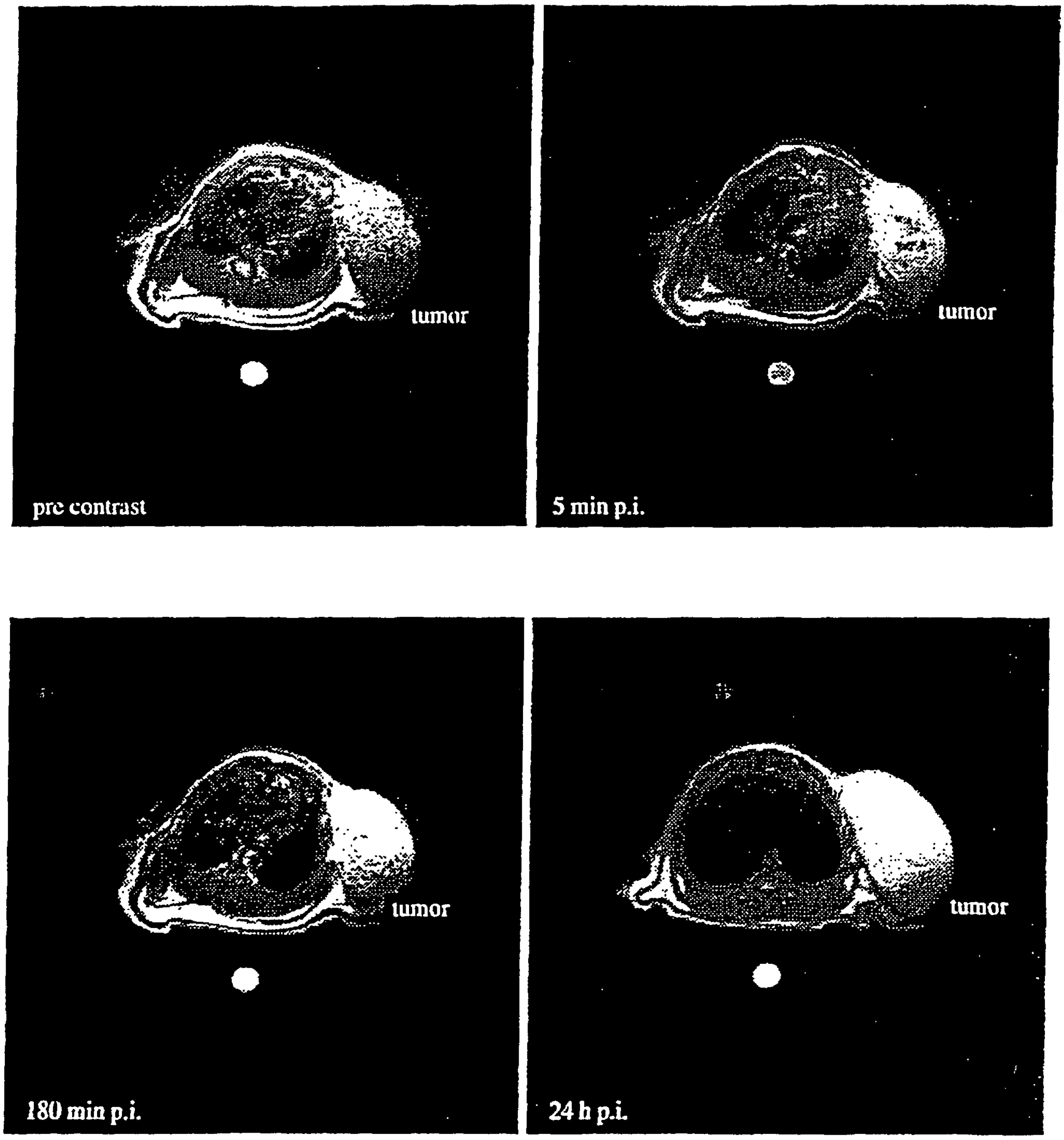
tumor
pre contrast
tumor
5 min p.i.
tumor
180 min p.i.
tumor
24 h p.i.

Fig. 2

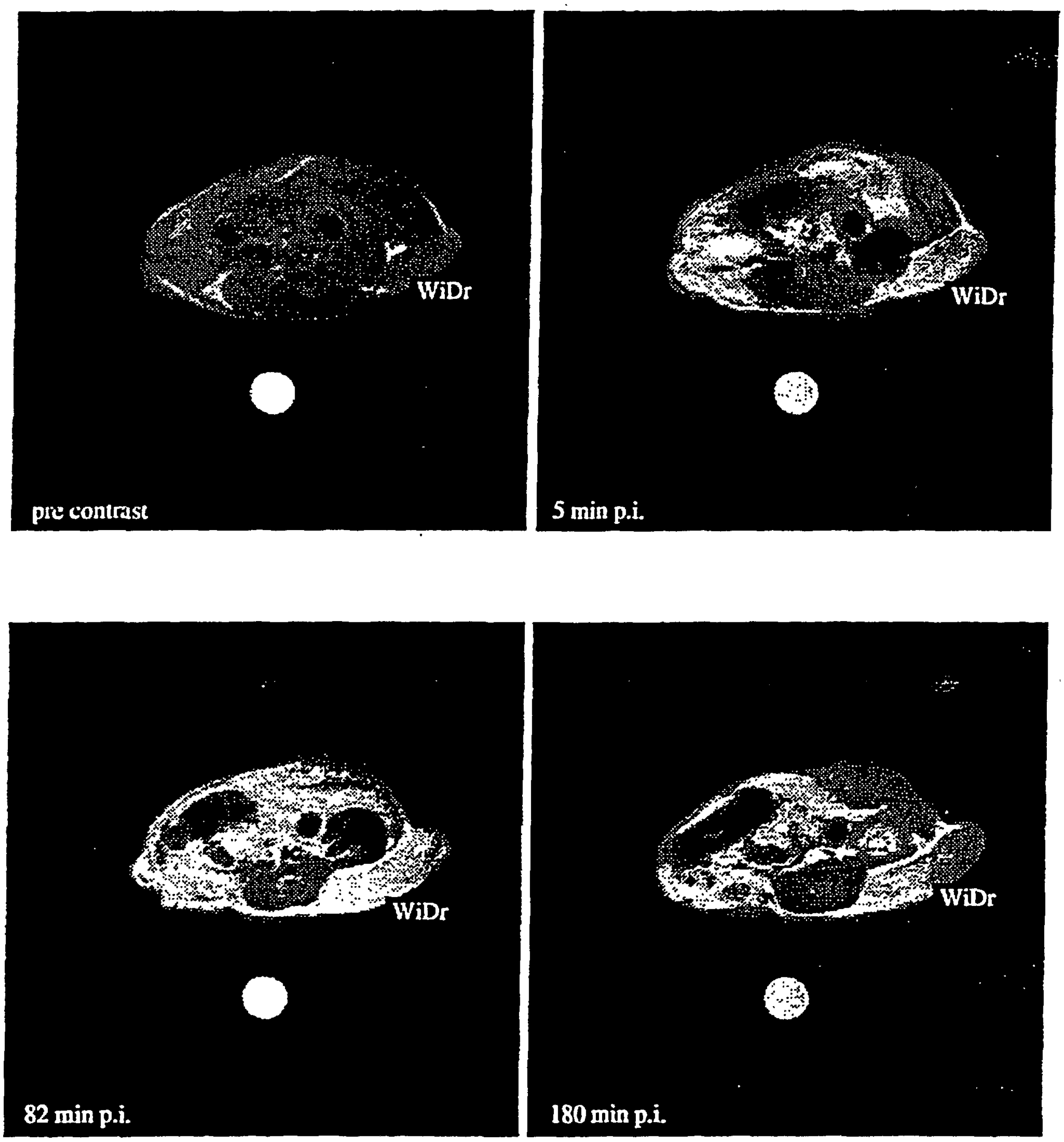

Fig. 3

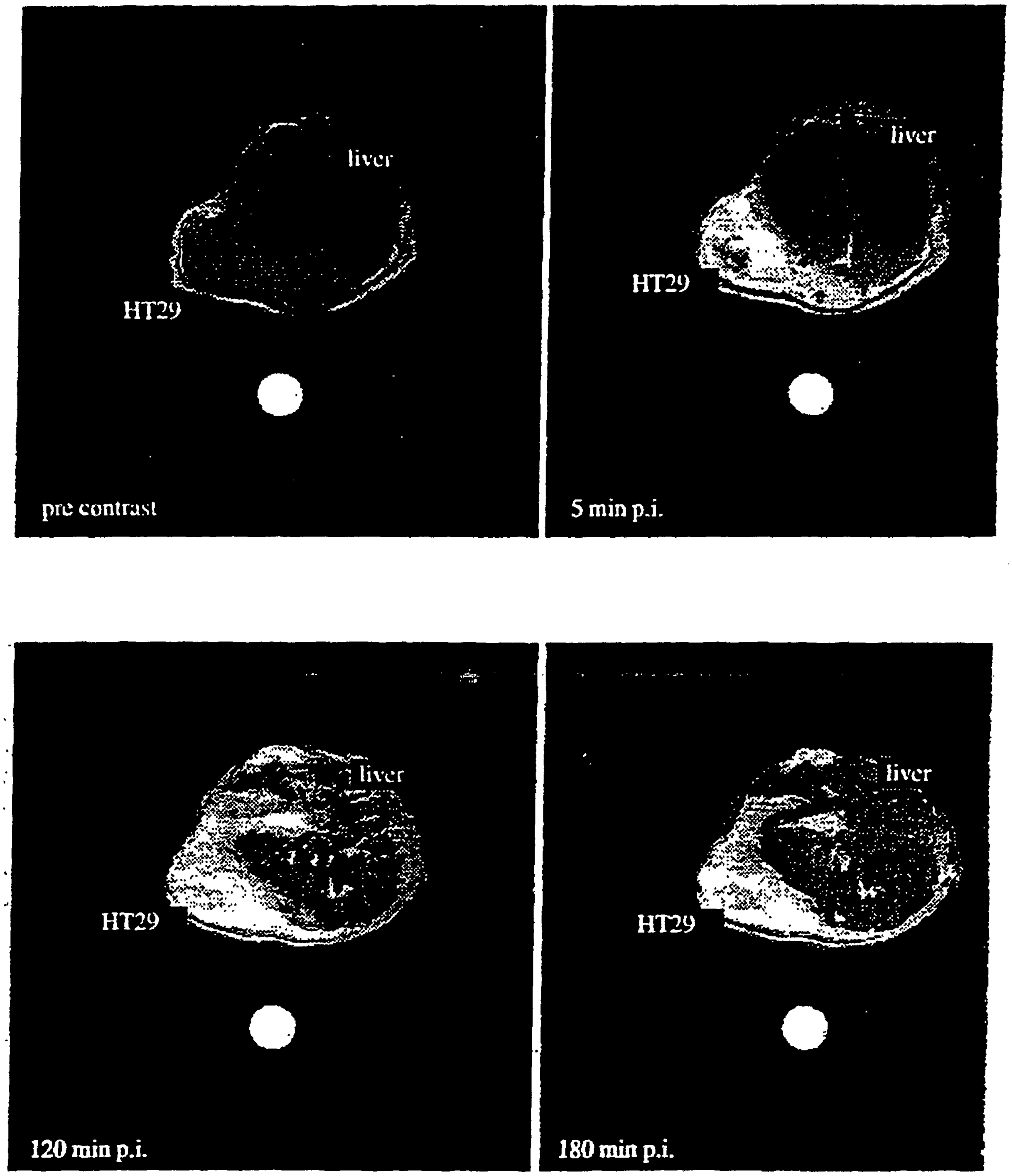
liver
HT29
pre contrast
liver
HT29
5 min p.i.
liver
HT29
120 min p.i.
liver
HT29
180 min p.i.

Fig. 4

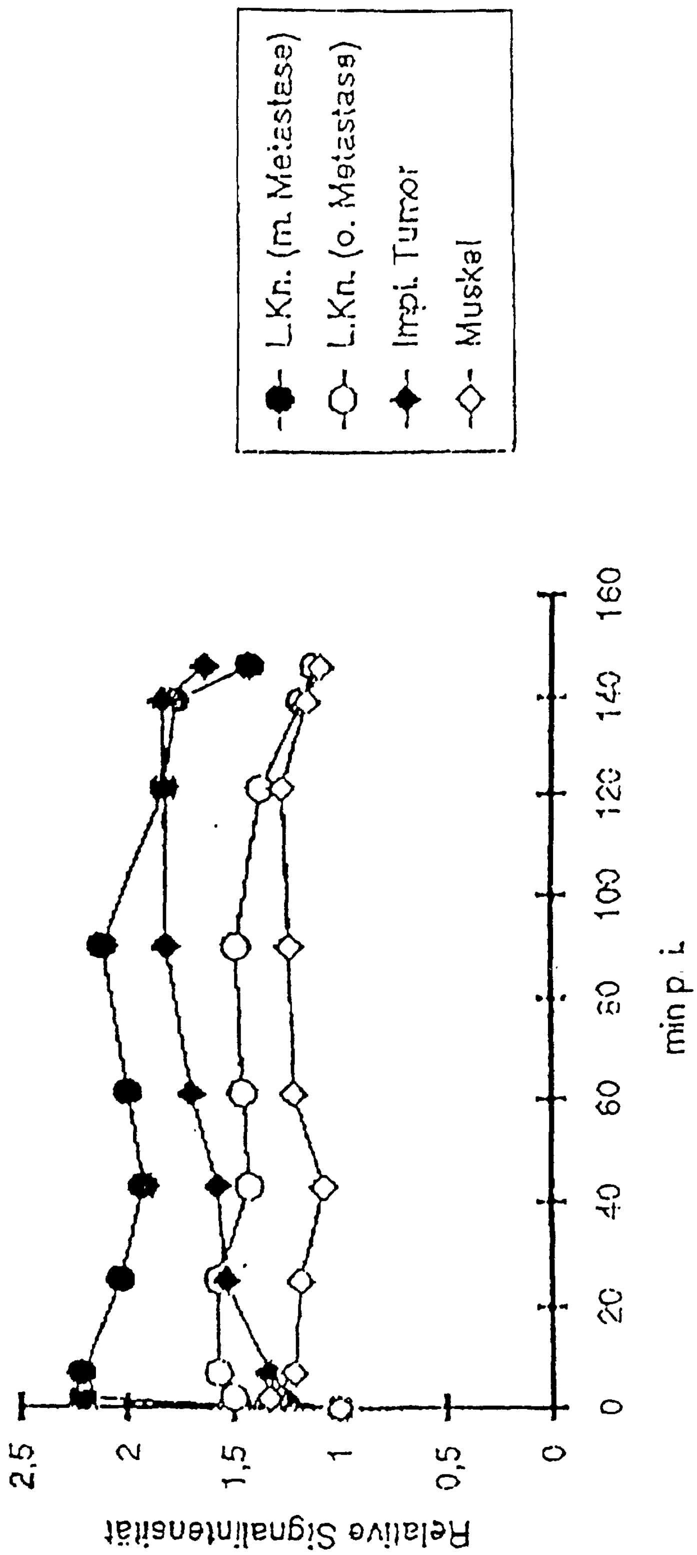


ERSATZBLATT